# EUROPEAN PATENT SPECIFICATION

(11) **EP 3 275 867 B1**
(45) Date of publication and mention of the grant of the patent: **10.06.2020**
(21) Application number: 16767632.9
(22) Date of filing: 01.02.2016
(51) Int. Cl.: C07D 217/14, C07D 217/00, C07D 405/04, C07D 471/04, C07D 231/56, C07D 495/04, C07D 333/60, C07D 237/30, C07D 239/78, A61K 31/472, A61K 31/517, A61P 3/00

(54) **PYRIDO-FUSED BICYCLIC COMPOUNDS AS URAT1 INHIBITORS FOR TREATING HYPERURICEMIA**
PYRIDO-KONDENSIERTE BICYCLISCHE VERBINDUNGEN ALS URAT1 INHIBITOREN ZUR BEHANDLUNG VON HYPERURIKÄMIE
COMPOSÉS PYRIDO-FUSIONNÉS BICYCLIQUES EN TANT QU'INHIBITEURS URAT1 POUR LE TRAITEMENT DE L'HYPERURICÉMIE

(30) Priority: 24.03.2015 CN 201510131828
(43) Date of publication of application: 31.01.2018
(73) Proprietor: Shanghai Yingli Pharmaceutical Co. Ltd., Shanghai 201203 (CN)
(72) Inventor: XU, Zusheng, Shanghai 201203 (CN); ZHANG, Nong, Shanghai 201203 (CN); SUN, Qingrui, Shanghai 201203 (CN); WU, Tianzhi, Shanghai 201203 (CN)
(74) Representative: dompatent von Kreisler Selting Werner - Partnerschaft von Patent- und Rechtsanwälten mbB
(86) International application number: PCT/CN2016/073043
(87) International publication number: WO 2016/150255

(56) References cited:
- WO-A1-2004/103997
- WO-A1-2009/137404
- WO-A1-2014/079787
- WO-A1-2014/177596
- WO-A2-2007/089557
- WO-A2-2011/150156
- CA-A1- 2 880 178
- CN-A- 101 400 660
- CN-A- 102 482 220
- JP-A- H10 120 680
- US-A1- 2013 115 190
- US-A1- 2014 005 221
- NITYA G. KUNDU ET AL: "Cyclopenta[f]isoquinoline derivatives designed to bind specifically to native deoxyribonucleic acid. 1. Synthesis of 3-ethoxy-8-methyl-7(5)H-cyclopenta[f]isoqu inoline", JOURNAL OF MEDICINAL CHEMISTRY, vol. 18, no. 4, 1 April 1975 (1975-04-01), pages 395-399, XP055434979, ISSN: 0022-2623, DOI: 10.1021/jm00238a015
- STRATFORD, E.S. ET AL.: 'Alkylation of Styrylacetic Acid Systems Using Lithium Diisopropylamide-Hexamethylphosphoramide. Effect of Temperature Variation' J. ORG. CHEM. vol. 44, no. 9, 31 December 1979, page 1571, XP055315288

## Description

### Field of invention

The present invention relates to a condensed ring derivative, and preparation method, intermediate, pharmaceutical composition and use thereof.

### Prior arts

Hyperuricemia (HUA) is related to many diseases such as gout, hypertension, diabetes, hypertriglyceridemia, metabolic syndrome, coronary heart disease and renal damage etc. (Puig JG, et al. CurrOpin Rheumatol, 2008, 20, 187-191; Edwards NL, et al. Cleve Clin J Med, 2008, 75(Suppl 5), 13-16), which has been a metabolic disease threatening human's health, and was recognized as one of the twenty stubborn and chronic diseases in the 21^{st} century by the United Nations.

Uric acid is the final product metabolized from the purine *in vivo,* which goes through glomerular filtration mainly in its origin form, and reabsorption, re-secretion by renal tubule, finally excreted with urine, and very few of them can enter enteric cavity through the secreting of the mesenteric cells. (Hediger M A, et al. Physiology 2005, 20(2), 125-133). S1 section of the proximal convoluted tubule is the position where the uric acid is reabsorbed, and 98%-100% filtered uric acid enters the epithelium through urate transporter 1 (URAT1) upon the brush border membrane of the tubular epithelial cells. Inhibiting the activity of the URAT1 can reduce the reabsorption of the uric acid, which allows the uric acid excreted with urine thereby lowering the level of the uric acid in the blood and relieving or treating hyperuricemia and various related diseases.

US20140005221A1 discloses a compound that has URAT1 inhibitory action, and a URAT1 inhibitor, a blood uric acid level-reducing agent and a pharmaceutical composition comprising the compound.

WO2011150156A2 discloses a heteroaryl compound, methods of their synthesis, pharmaceutical compositions comprising the compounds, and methods of their use. The compounds provided herein are useful for the treatment, prevention, and/or management of various disorders, such as CNS disorders and metabolic disorders, including, but not limited to, neurological disorders, psychosis, schizophrenia, obesity, and diabetes.

WO2009137404A1 discloses compounds that are gamma secretase modulators and are therefore useful for the treatment of diseases treatable by modulation of gamma secretase such as Alzheimer's disease.

US20130115190A1 discloses compounds which inhibit the function of the NS3 protease (also referred to herein as "serine protease") encoded by Hepatitis C virus (HCV), compositions comprising such compounds, and methods for inhibiting the function of the NS3 protease.

WO2007089557A2 discloses compounds, pharmaceutical composition comprising such compounds and methods of using such compounds to treat or prevent diseases or disorders associated with the activity of the Peroxisome Proliferator Activated Receptor (PPAR) families.

WO2004103997A1 discloses PPAR alpha activators, pharmaceutical compositions containing such compounds and the use of such compounds to elevate certain plasma lipid levels, including high density lipoprotein-clolesterol and to lower certain other plasma lipid levels, such as LDL-cholesterol and triglycerides and accordingly to treat diseases which are exacerbated by low levels of HDL cholesterol and/or high levels of LDL-cholesterol and triglycerides, such as atherosclerosis and cardiovascular diseases, in mammals, including humans.

CA2880178A1 discloses a compound which has URAT1 inhibitory activity and a URAT1 inhibitor, a blood uric acid level reducing agent and a pharmaceutical composition, each of which contains the compound.

WO2014079787A1 discloses the use of compounds, or a pharmaceutically acceptable acid addition salt, to a racemic mixture or to its corresponding enantiomer and/or optical isomers thereof, for the treatment of schizophrenia, obsessive-compulsive personality disorder, depression, bipolar disorders, anxiety disorders, normal aging, epilepsy.

CN102482220A discloses an excellent preventive or therapeutic agent for dementia, schizophrenia, etc., which acts on the basis of a serotonin 5-HT5A receptor modulation activity. An acylguanidine derivative characterized by having a structure in which guanidine has been bonded to one of the rings of quinoline or isoquinoline through a carbonyl group and a cyclic group has been bonded to the other ring was ascertained to have a potent 5-HT5A receptor modulation activity and have an excellent pharmacological activity based on the modulation activity. The derivative was found to be capable of being an excellent preventive or therapeutic agent for dementia, schizophrenia, bipolar disorder, or attention deficit/hyperactivity disorder.

CN101400660A provides a compound which can be used for the treatment of a disease associated with 11β-hydroxysteroid dehydrogenase type 1 (11β-HSD1), particularly diabetes or insulin resistance. It also discloses a triazole derivative which has a triazole ring substituted by a 3-substituted methyl group at position-3 and further substituted by a lower alkyl, a cycloalkyl or the like at position-5, or a pharmaceutically acceptable salt thereof.

JPH10120680A discloses an indolizine or its salt, which has a cGMP-PDE inhibitory activity, and is useful as a medicine for the treatment of a variety of diseases.

J. ORG. CHEM., vol. 44, No. 9, 31 December 1979 (1979-12-31), page 1571, XP055315288 discloses a reaction concerning the alkylation of styrylacetic scid systems using lithium diisopropylamide-hexamethylphosphoramide and the effect of the temperature variation during the reaction.

### Content of the present invention

The technical problem to be solved in the present invention is to provide a condensed ring derivative totally different from the prior art, and preparation method, intermediate, pharmaceutical composition and use thereof. The condensed ring derivative of the present invention has obvious inhibitory activity against URAT1, which can effectively relieve or treat hyperuricemia and various related diseases.

The embodiments of the present invention are reflected in claims 1, 10, 12 and 14.

The preferred embodiments of the present invention are reflected in claims 2 to 9, 11, 13, 15, and 16.

The present invention provides a condensed ring derivative having a structure of formula II-1 or IV-1, a tautomer, a mesomer, a racemate, an enantiomer, a diastereoisomer, or a pharmaceutically acceptable salt thereof,

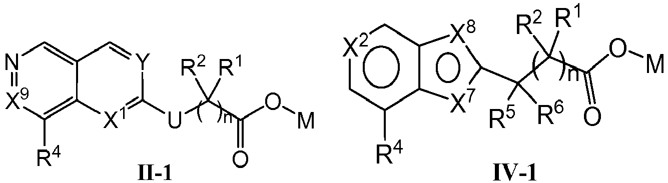

Wherein M is a hydrogen, a deuterium or a pharmaceutically acceptable cation (preferably a sodium ion, a potassium ion or a calcium ion);
U is 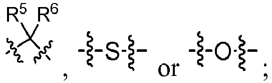 X¹ is CH or N; X² is N;
X⁹ is C or N;
Y is CH or N;
each of X⁷ and X⁸ is independently CH or S;
R¹ and R² are independently H, D, a halogen (preferably F, Cl, Br or I, more preferably F), an alkyl (preferably a C₁₋₄ alkyl), CN, an alkoxy (preferably a C₁₋₄ alkoxy), a cycloalkyl (preferably a C₃₋₆ cycloalkyl), an alkenyl (preferably a C₂₋₄ alkenyl), a alkynyl (preferably a C₂₋₄ alkynyl) or a heterocycloalkyl (preferably a C₂₋₁₀ heterocycloalkyl having 1-2 heteroatoms selected from O, S or N; the C₂₋₁₀ heterocycloalkyl is preferably a C₂₋₅ heterocycloalkyl); or, R¹, R² together with the carbon atom attached form a cycloalkyl (preferably a C₃₋₆ cycloalkyl, more preferably cyclobutyl) or a heterocyclic group (preferably a C₂₋₅ heterocyclic group having 1-2 heteroatoms selected from O or S); the alkyl, the alkoxy, the cycloalkyl, the alkenyl, the alkynyl, the heterocycloalkyl, the cycloalkyl formed by R¹, R² and the carbon atom attached, or the heteroalkyl formed by R¹, R² and the carbon atom attached can further be substituted by a substituent selected from the group consisting of D (e.g. 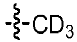 ), a halogen (e.g. F, Cl, Br or I, preferably Cl), CN, an alkyl (preferably a C₁₋₄ alkyl), an alkoxy (preferably a C₁₋₄ alkoxy), a cycloalkyl (preferably a C₃₋₆ cycloalkyl), an alkenyl (preferably a C₂₋₄ alkenyl), an alkynyl (preferably a C₂₋₄ alkynyl), a heterocycloalkyl (preferably a C₂₋₁₀ heterocycloalkyl having 1-2 heteroatoms selected from O, S or N; the C₂₋₁₀ heterocycloalkyl is preferably a C₂₋₅ heterocycloalkyl) or an aryl (preferably a C₆₋₁₀ aryl);
R⁴ is an alkyl (preferably a C₁₋₄ alkyl), an alkoxy (preferably a C₁₋₄ alkoxy), a cycloalkyl (preferably a C₃₋₆ cycloalkyl), an alkenyl (preferably a C₂₋₄ alkenyl), an alkynyl (preferably a C₂₋₄ alkynyl), a heterocycloalkyl (preferably a C₂₋₁₀ heterocycloalkyl having 1-3 heteroatoms selected from N, S or O; the C₂₋₁₀ heterocycloalkyl is preferably a C₂₋₅ heterocycloalkyl), each of which are substituted by a substituent selected from the group consisting of an aryl, an aryl substituted by a halogen and/or CN, a heteroaryl or a heteroaryl substituted by CN; or, an aryl (preferably a C₆₋₁₀ aryl, more preferably a phenyl or a naphthyl) or a heteroaryl (preferably a C₂₋₁₀ heteroaryl having 1-2 heteroatoms selected from O, more preferably 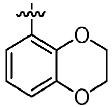 ); wherein the aryl or the heteroaryl can further be substituted by a substituent selected from the group consisting of D, a halogen (e.g. F, Cl, Br or I, preferably F or Cl), CN, an alkyl (preferably a C₁₋₄ alkyl), an alkoxy (preferably a C₁₋₄ alkoxy), a cycloalkyl (preferably a C₃₋₆ cycloalkyl), an alkenyl (preferably a C₂₋₄ alkenyl), an alkynyl (preferably a C₂₋₄ alkynyl), a heterocycloalkyl (preferably a C₂₋₁₀ heterocycloalkyl having 1-2 heteroatoms selected from O, S or N; the C₂₋₁₀ heterocycloalkyl is preferably a C₂₋₅ heterocycloalkyl), an aryl (preferably a C₆₋₁₀ aryl), an aryl substituted by a halogen and/or CN (e.g. 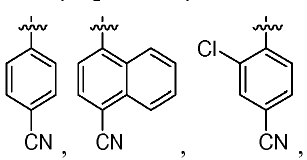 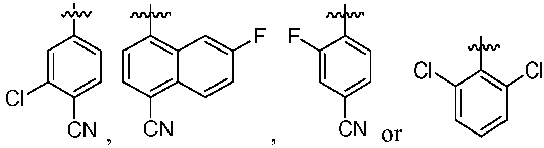 ), a heteroaryl (preferably a C₂₋₁₀ heteroaryl having 1-2 heteroatoms selected from O) or a heteroaryl substituted by CN (e.g. 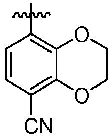 ); each of R⁵ and R⁶ is independently H, D, OH, a halogen (e.g. F, Cl, Br or I, preferably F), an alkyl (preferably a C₁₋₄ alkyl), CN, an alkoxy (preferably a C₁₋₄ alkoxy), a cycloalkyl (preferably a C₃₋₆ cycloalkyl), an alkenyl (preferably a C₂₋₄ alkenyl), an alkynyl (preferably a C₂₋₄ alkynyl) or a heterocycloalkyl (preferably a C₂₋₁₀ heterocycloalkyl having 1-2 heteroatoms selected from O, S or N; the C₂₋₁₀ heterocycloalkyl is preferably a C₂₋₅ heterocycloalkyl); or R⁵, R⁶ together with the carbon atom attached form a cycloalkyl (preferably a C₃₋₆ cycloalkyl) or a heterocyclic group (preferably a C₂₋₅ heterocyclic group having 1-2 heteroatoms selected from O or S); the alkyl, the alkoxy, the cycloalkyl, the alkenyl, the alkynyl, the heterocycloalkyl, the cycloalkyl formed by R⁵, R⁶ together with the carbon atom attached or the heteroalkyl formed by R⁵, R⁶ together with the carbon atom attached can further be substituted by a substituent selected from the group consisting of D, a halogen (e.g. F, Cl, Br or I, preferably Cl), CN, an alkyl (preferably a C₁₋₄ alkyl), an alkoxy (preferably a C₁₋₄ alkoxy), a cycloalkyl (preferably a C₃₋₆ cycloalkyl), an alkenyl (preferably a C₂₋₄ alkenyl), an alkynyl (preferably a C₂₋₄ alkynyl), a heterocycloalkyl (preferably a C₂₋₁₀ heterocycloalkyl having 1-2 heteroatoms selected from O, S or N; the C₂₋₁₀ heterocycloalkyl is preferably a C₂₋₅ heterocycloalkyl) or an aryl (preferably a C₆₋₁₀ aryl);
n is 0, 1 or 2.Each of the letter and the substituent in the condensed ring derivative having a structure of formula II-1 and IV-1 is preferably as follows:
M is H or a pharmaceutically acceptable cation;
each of R¹ and R² is H, a halogen (e.g. F, Cl, Br or I, preferably F), D or an alkyl (preferably a C₁₋₄ alkyl); or R¹, R² together with the carbon atom attached form a cycloalkyl (preferably a C₃₋₆ cycloalkyl, more preferably a cyclobutyl);
R³ is H, a halogen (preferably F, Cl, Br or I, more preferably F), an alkyl (preferably a C₁₋₄ alkyl) or an aryl (preferably a C₆₋₁₀ aryl, more preferably a phenyl, wherein the C₆₋₁₀ aryl can be further substituted by one or more than one CN(s), e.g 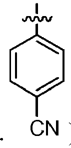 );
R⁴ is an alkyl (preferably a C₁₋₄ alkyl, wherein the C₁₋₄ alkyl can be further substituted by an aryl substituted by halogen(s), (e.g. 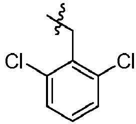 ), an aryl (preferably a C₆₋₁₀ aryl, more preferably a phenyl or a naphthyl, wherein the C₆₋₁₀ aryl can be further substituted by one or more than one CN(s) and/or halogen(s) (preferably F, Cl, Br or I, more preferably F or Cl), e.g. 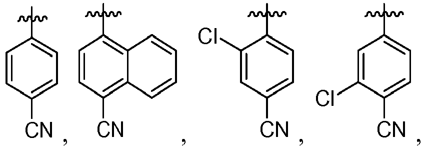 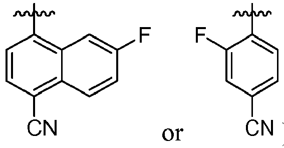 ) or a heteroaryl (preferably a C₂₋₁₀ heteroaryl having 1-2 heteroatoms selected from O, more preferably 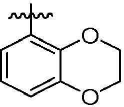 ; wherein the C₂₋₁₀ heteroaryl can be further substituted by one or more than one CN(s), e.g. 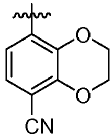 );
each of R⁵ and R⁶ is independently H, a halogen (e.g. F, Cl, Br or I, preferably F), an alkyl (preferably a C₁₋₄ alkyl) or OH.

In a preferred embodiment of the present invention, in the compound having a structure of formula II-1,
each of R¹ and R² is independently H or an alkyl (preferably a C₁₋₄ alkyl); or R¹, R² together with the carbon atom attached form a cycloalkyl (preferably a C₃₋₆ cycloalkyl, more preferably a cyclobutyl);
M is H;
R⁴ is an aryl (preferably a C₆₋₁₀ aryl, more preferably a phenyl or a naphthyl, wherein the C₆₋₁₀ aryl can be further substituted by one or more than one CN(s) and/or halogen(s) (preferably F, Cl, Br or I, more preferably F or Cl), e.g. 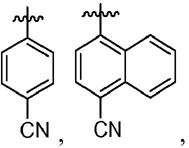 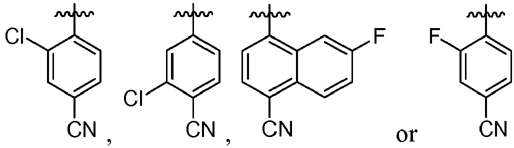 ) or a heteroaryl (preferably a C₂₋₁₀ heteroaryl having 1-2 heteroatoms selected from O, wherein the C₂₋₁₀ heteroatom can be further substituted by one or more than one CN(s), e.g. 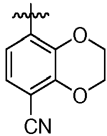 ); U is 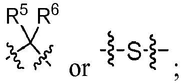 each of R⁵ and R⁶ is independently H or an alkyl (preferably a C₁₋₄ alkyl);
n is 1.

Preferably, in the compound having a structure of formula II-1,
where U is 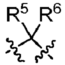 and both of R⁵ and R⁶ are hydrogen, R¹ and R² are not H at the same time.

In a preferred embodiment of the present invention, in the compound having a structure of formula IV-1,
X² is N;
each of X⁷ and X⁸ is independently CH or S;
each of R¹ and R² is independently H or an alkyl (preferably a C₁₋₄ alkyl);
R4 is an aryl (preferably a C₆₋₁₀ aryl, more preferably a phenyl or a naphthyl, wherein the C₆₋₁₀ aryl can be further substituted by one or more than one CN(s) and/or halogen(s) (preferably F, Cl, Br or I, more preferably F or Cl), e.g. 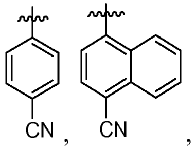 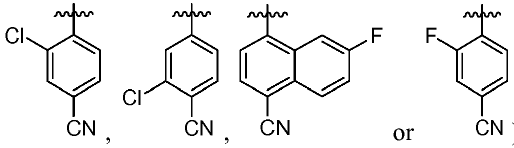 ); each of R⁵ and R⁶ is independently H or an alkyl (preferably a C₁₋₄ alkyl);
n is 0 or 1.

Preferably, X² is N; X⁷ is CH; X⁸ is S.

Preferably, in the compound having a structure of formula IV-1, M is H.

Preferably, in the compound having a structure of formula IV-1, where R¹ and/or R² is an alkyl, R⁵ and R⁶ are H; where R⁵ and/or R⁶ is an alkyl, R¹ and R² are H.

Preferably, in the compound having a structure of formula IV-1, where X⁷ is S, X⁸ is CH, R¹ and R² are alkyl, and R⁵ and R⁶ are H, R⁴ is a phenyl.

The condensed ring derivative in the present invention is preferably selected from the compound consisting of

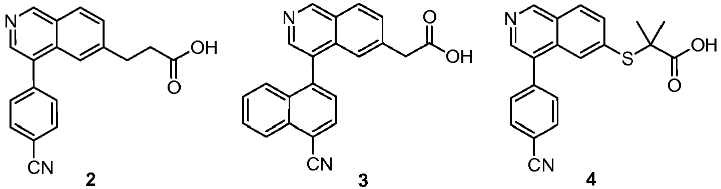

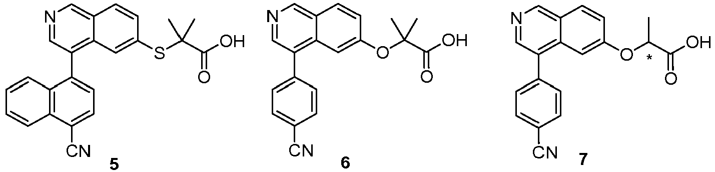

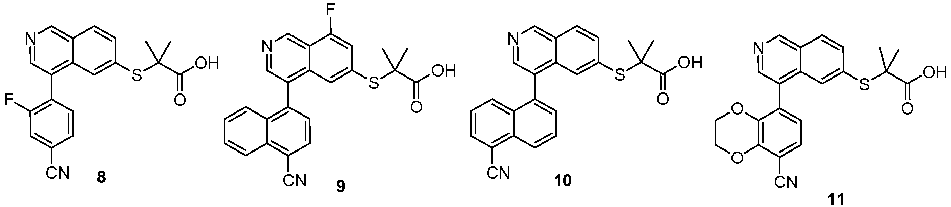

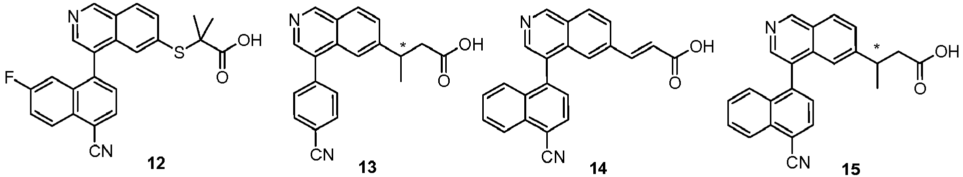

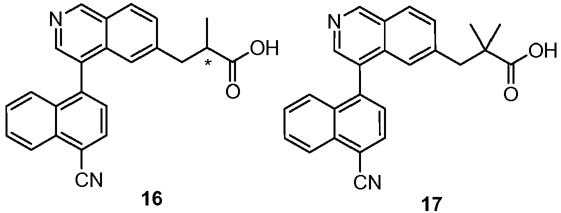

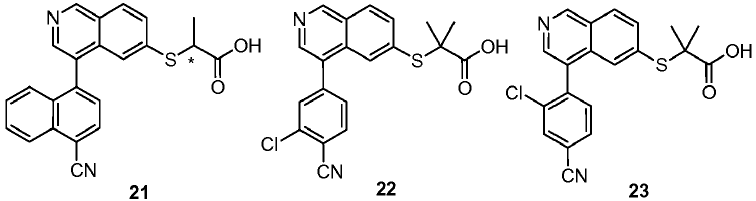

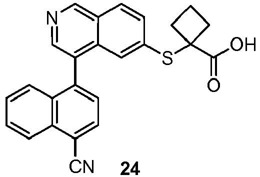

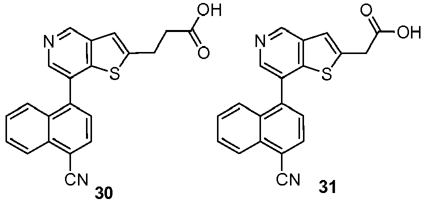

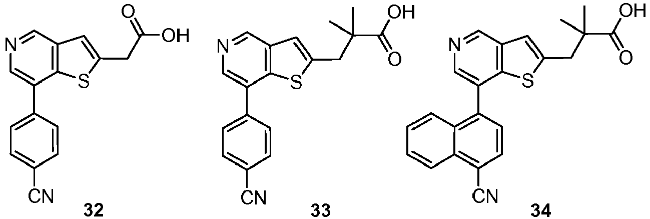

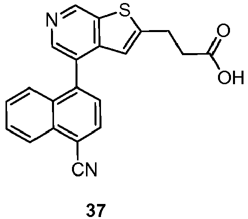

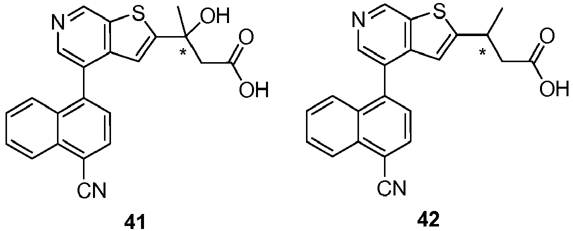

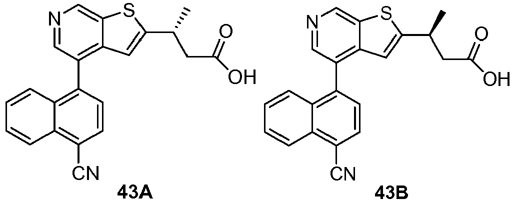

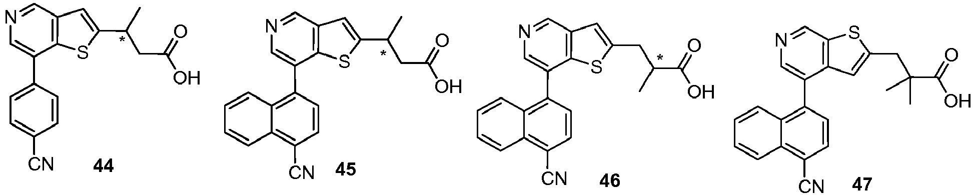

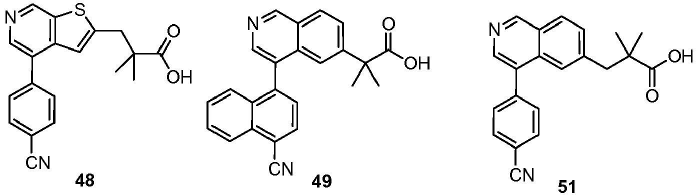

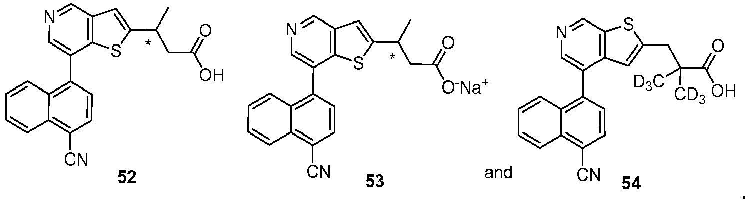

In the above compounds, the carbon marked with * is a chiral carbon or a non-chiral carbon; where it is a chiral carbon, it has S configuration or R configuration, where it is a non-chiral carbon, it is racemic.

The present invention also provides a process for preparing the condensed ring derivative having a structure of formula I, the tautomer, the mesomer, the racemate, the enantiomer, the diastereoisomer, or the pharmaceutically acceptable salt thereof, which can be synthesized according to the method known in the art with commercially available raw materials. In the present invention, the process for preparing the condensed ring derivative having a structure of formula I preferably comprises that in a solvent, in the presence of a base, carrying out a hydrolysis reaction on the compound having a structure of formula I-a to give the compound having a structure of formula I; 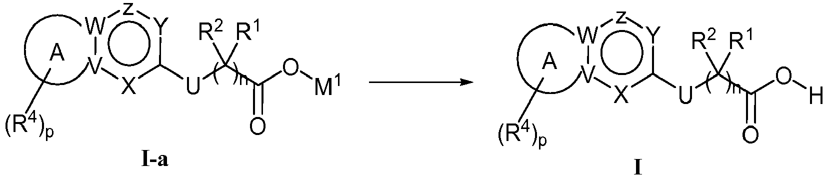 wherein the structure of formula I-a is formula II-a-1 or IV-a-1, 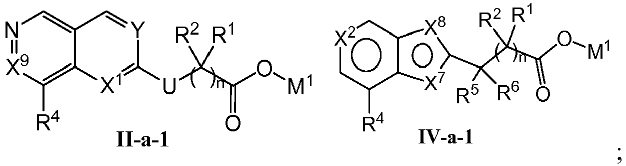 the structure of formula I is formula II-1 or IV-1, 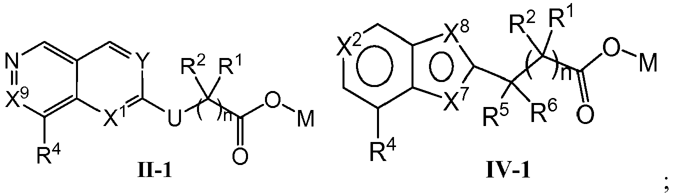 M¹ is an alkyl;
in the compound of formula II-a-1, X¹ is CH or N; Y is CH or N; the definitions of X⁹, R¹, R², R³, R⁴, U and n are defined as above;
in the compound of formula IV-a-1, each of X⁷ and X⁸ is independently CH or S; the definitions of R¹, R², R⁴, R⁵, R⁶, X² and n are defined as above;
in the compound of formula II-1, M is H, the definitions of X⁹, R¹, R², R³, R⁴, U and n are defined as above;
in the compound of formula IV-1, M is H, the definitions of R¹, R², R⁴, R⁵, R⁶, X² and n are defined as above.

The method and condition of the hydrolysis reaction are common method and condition in the art, therefore, in the hydrolysis reaction, the solvent used and the amount thereof, the base used and the amount thereof, the temperature and time of the hydrolysis reaction, and the post treatment after the hydrolysis reaction can all be selected according to the common processes and conditions in the art. For example, the solvent can be a mixed solution of alcohols (e.g. methanol), ethers (e.g. THF) and water, or a mixed solution of alcohols (e.g. methanol) and water. The base can be alkalis hydroxide (e.g. LiOH and/or NaOH). Where a base is used, the base can be in the form of its aqueous solution (the molar concentration of the base aqueous solution can be 1mol/L). The hydrolysis reaction can be carried out at room temperature. The process of the hydrolysis reaction can be monitored by the common method in the art (e.g. TLC, GC, HPLC or NMR etc.). The post treatment can comprise that mixing the reaction solution obtained after the hydrolysis reaction with the hydrochloric acid aqueous solution (e.g. 2mol/L hydrochloric acid aqueous solution) and water (solid precipitated), filtering, washing the filtrate cake with water, drying under reduced pressure.

When M is a pharmaceutically acceptable cation, the compound having a structure of formula (I) wherein M is H can be neutralized by a hydroxide containing a pharmaceutically acceptable cation (e.g. sodium hydroxide). The conditions used in the process of a neutralizing reaction are common conditions used in the neutralizing reaction in the organic synthesis field.

The compound having a structure of formula I can be prepared according to the following process, comprising where U is 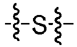 , the compound having a structure of formula I can be prepared according to route I, which comprises 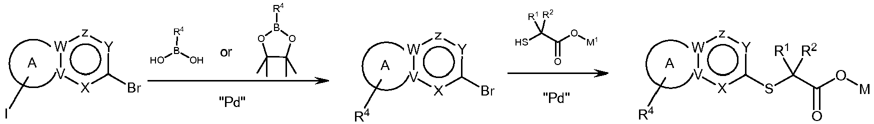 where U is 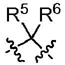 , R⁶ is H and n is 1, the compound having a structure of formula I can be prepared according to route II, which comprises 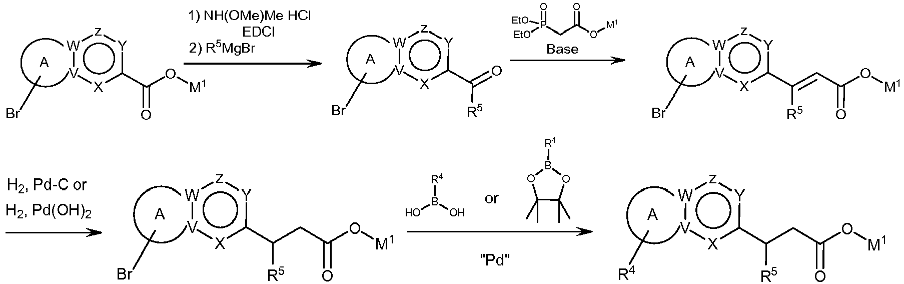 where U is 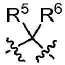 , both R⁵ and R⁶ are H and n is 1, the compound having a structure of formula I can be prepared according to route III, which comprises

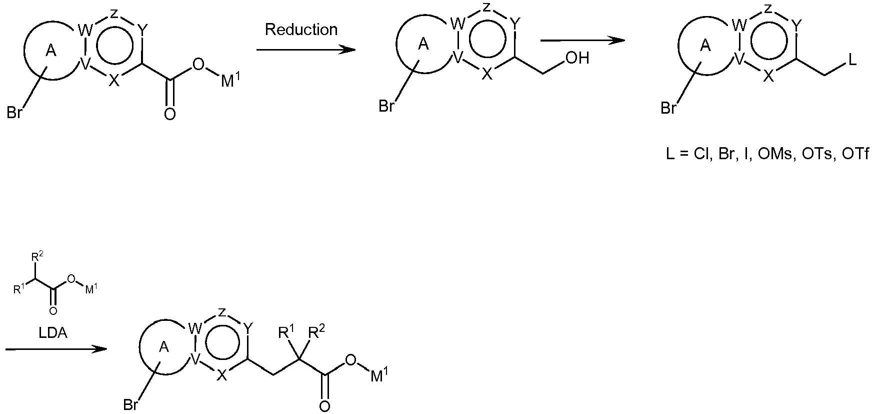

In the above three routes, each letter or group involved is defined as above. Meanwhile, the conditions and steps used in the chemical reactions involved in the above three routes can refer to the common conditions and steps used in the art, and the compound obtained according to the above process can further be modified in the peripheral region, thereby obtaining other target compound of the present invention.

The present invention also provides a compound of formula II-a-1 or IV-a-1: 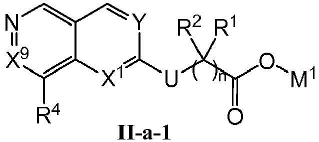 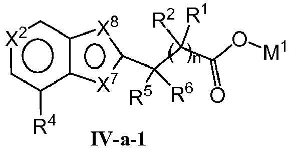 M¹ is an alkyl;
in the compound of formula II-a-1, X¹ is CH or N; Y is CH or N; the definitions of X⁹, R¹, R², R³, R⁴, U and n are defined as above;
in the compound of formula IV-a-1, each of X⁷ and X⁸ is independently CH or S; the definitions of R¹, R², R⁴, R⁵, R⁶, X² and n are defined as above.

The intermediate having a structure of the present invention, preferably is selected from the compound consisting of

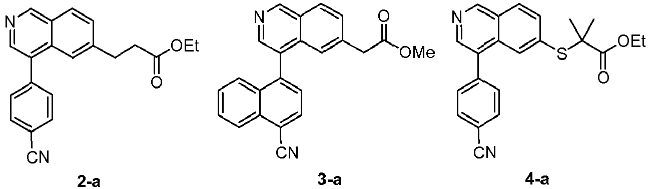

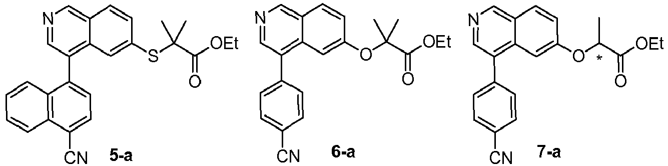

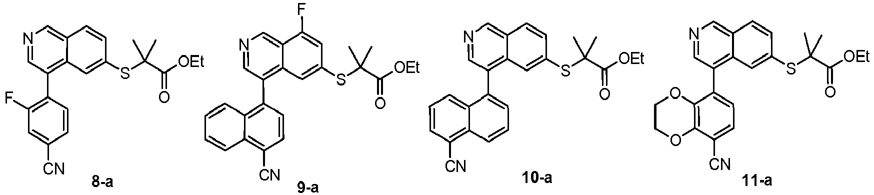

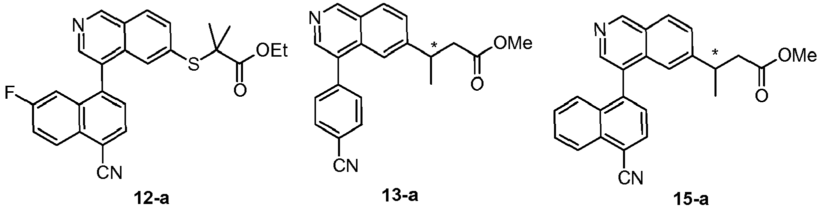

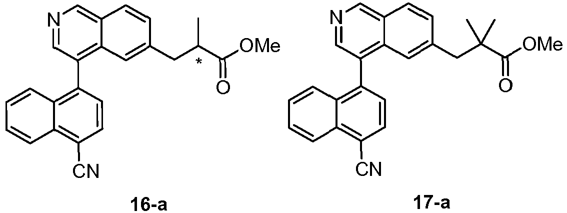

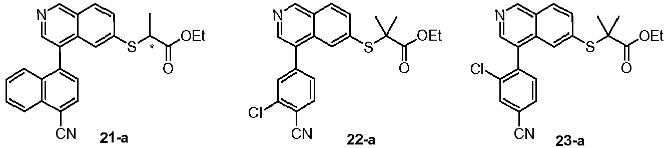

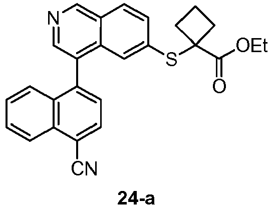

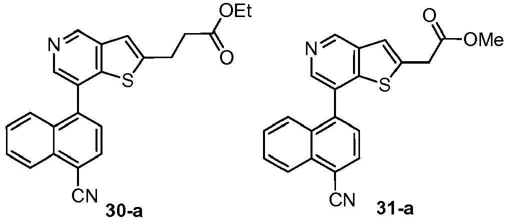

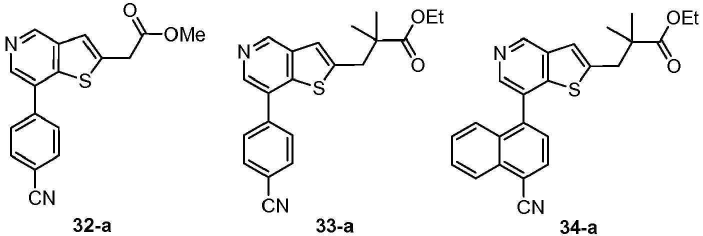

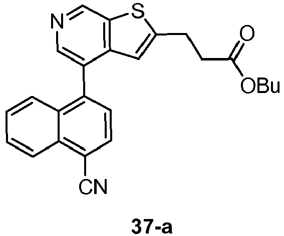

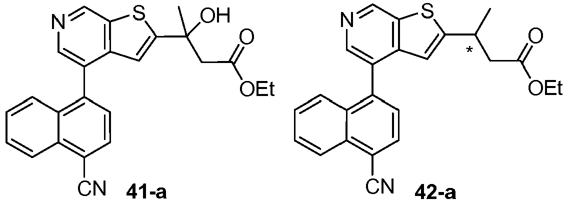

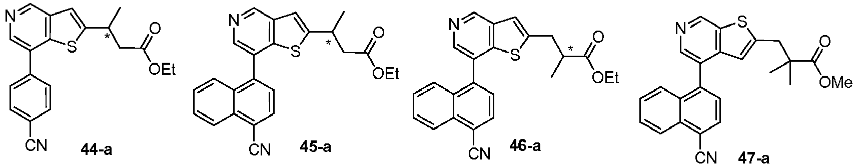

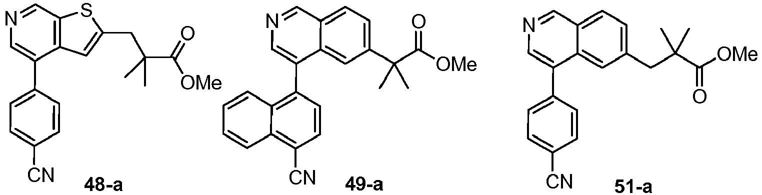

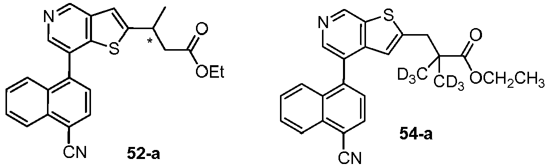

In above compounds, the carbon marked with * is a chiral carbon or a non-chiral carbon; where it is a chiral carbon, it has S configuration or R configuration, where it is a non-chiral carbon, it is racemic.

The present invention also provides one of enantiomers contained in the racemic compound 7-a, which is given by separating the racemic compound 7-a by an enantiomeric chromatographic column: 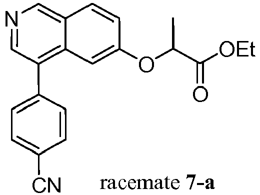 wherein the chromatograph is preferably Gilson 281, the preparative column is preferably r, r-Whelk-O1 (20×250 mm, 5 µm), the mobile phase is preferably Hexane : EtOH : DEA = 70 : 30 : 0.1 (v/v/v); when the retaining time is 9.0 min, one enantiomer 7A-a is given; when the retaining time is 11.0 min, the other enantiomer 7B-a is given.

The present invention also provides one of enantiomers contained in the racemate 7: 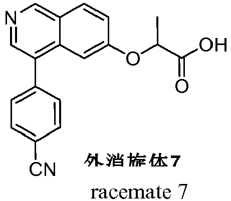 wherein the process for preparing one enantiomer preferably comprises that in an organic solvent, in the presence of a base, carrying out a hydrolysis reaction on the enantiomer 7A-a; the process for preparing the other enantiomer preferably comprises that in an organic solvent, in the presence of a base, carrying out a hydrolysis reaction on the enantiomer 7B-a.

The present invention also provides one of the enantiomers contained in the racemate 42-a, which is given by separating the racemate 42-a by an enantiomeric chromatographic column: 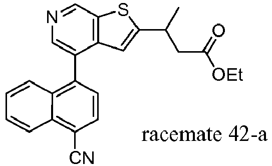 wherein the chromatograph is preferably SFC-80 (Thar, Waters), the preparative column is preferably AD 30×250mm, 5µm (Decial), the mobile phase is preferably n-Hexane-0.1% DEA : EtOH-0.1%DEA = 80 : 20 (v/v); when the retaining time is 18.0 min, one enantiomer 43A-a is given; when the retaining time is 20.0 min, the other enantiomer 43B-a is given.

The present invention also provides one of the enantiomers contained in the racemate 42: 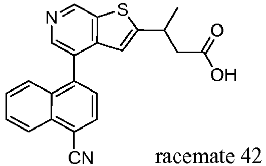 wherein the process for preparing one enantiomer preferably comprises that in an organic solvent, in the presence of a base, carrying out a hydrolysis reaction on the enantiomer 43A-a; the process for preparing the other enantiomer preferably comprises that in an organic solvent, in the presence of a base, carrying out a hydrolysis reaction on the antimer 43B-a.

The present invention also provides one of the enantiomers contained in the racemate 44-a, which is given by separating the racemate 44-a by an enantiomeric chromatographic column: 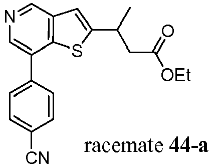 wherein the chromatograph is preferably Gilson 281, the preparative column is preferably r, r-Whelk-O1 (20×250 mm, 5 µm), the mobile phase is preferably n-Hexane : EtOH : DEA = 70 : 30 : 0.1 (v/v/v); when the retaining time is 6.0 min, one enantiomer 44A-a is given; when the retaining time is 7.0 min, the other enantiomer 44B-a is given.

The present invention provides one of the enantiomers contained in the racemate 44: 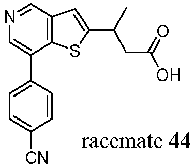 wherein the process for preparing one enantiomer preferably comprises that in an organic solvent, in the presence of a base, carrying out a hydrolysis reaction on the antimer 44A-a; the process for preparing the other enantiomer preferably comprises that in an organic solvent, in the presence of a base, carrying out a hydrolysis reaction on the antimer 44B-a.

The present invention provides one of the enantiomers contained in the racemate 45-a, which is given by separating the racemate 45-a by an enantiomeric chromatographic column: 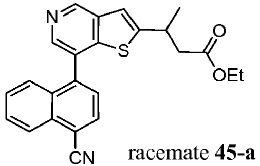 wherein the chromatograph is preferably SFC-80 (Thar, Waters), the preparative column is preferably AD 30×250mm, 5µm (Decial), the mobile phase is preferably CO₂ : (Methanol-0.1%NH₄OH) = 65 : 35 (v/v); when the retaining time is 8.5 min, one enantiomer 45A-a is given; when the retaining time is 10.5 min, the other enantiomer 45B-a is given.

The present invention provides one of the enantiomers contained in the racemate 45: 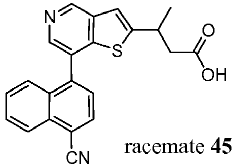 wherein the process for preparing one enantiomer preferably comprises that in an organic solvent, in the presence of a base, carrying out a hydrolysis reaction on the antimer 45A-a; the process for preparing the other enantiomer preferably comprises that in an organic solvent, in the presence of a base, carrying out a hydrolysis reaction on the enantiomer 45B-a.

The present invention provides one of the enantiomers contained in the racemate 46-a, which is given by separating the racemate 46-a by an enantiomeric chromatographic column: 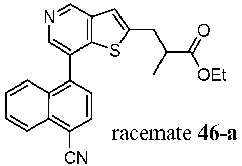 wherein the chromatograph is preferably Gilson 281, the preparative column is preferably r, r-Whelk-O1 (20×250 mm, 5 µm), the mobile phase is preferably Hexane : EtOH : DEA = 80 : 20 : 0.1 (v/v/v); when the retaining time is 14.0 min, one enantiomer 46A-a is given; when the retaining time is 18.0 min, the other antimer 46B-a is given.

The present invention provides one of the enantiomers contained in the racemate 46: 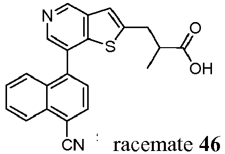 wherein the process for preparing one enantiomer preferably comprises that in an organic solvent, in the presence of a base, carrying out a hydrolysis reaction on the enantiomer 46A-a; the process for preparing the other enantiomer preferably comprises that in an organic solvent, in the presence of a base, carrying out a hydrolysis reaction on the enantiomer 46B-a.

The present invention also provides one of the enantiomers included in the racemate 53, the process for preparing one enantiomer preferably comprises, in water, neutralizing the enantiomer 45A by NaOH.

The present invention also provides a use of the condensed ring derivative having a structure of formula II-1 or IV-1, the tautomer, the mesomer, the racemate, the enantiomer, the diastereoisomer, or the pharmaceutically acceptable salt thereof for use in preventing and/or treating hyperuricemia or its related diseases. The hyperuricemia related diseases generally include gout, hypertension, diabetes, hypertriglyceridemia, metabolic syndrome, coronary heart disease and renal damage and so on.

The present invention also provides a pharmaceutical composition, which comprises therapeutically effective amount of the condensed ring derivative having a structure of formula II-1 or IV-1, the tautomer, the mesomer, the racemate, the enantiomer, the diastereoisomer, or the pharmaceutically acceptable salt thereof, and one or more than one pharmaceutically acceptable carrier and/or diluter.

In the present invention, the pharmaceutical composition can be in the form of an oral administration, as well as a sterile injectable aqueous solution, which can be prepared according to any known process for preparing pharmaceutical composition in the art.

The pharmaceutical composition can be used alone, as well as in combination with other medicament having activity on lowering uric acid. The medicament having activity on lowering uric acid is selected from the group consisting of uric acid transporter 1 inhibitor, Xanthine oxidase inhibitor, Xanthine oxidoreductase and Xanthine dehydrogenase inhibitor, preferably Allopurinol and/or Febuxostat.

Unless otherwise specified, the terms involved in the description and the claims of the present invention have following definitions:
"Alkyl" used herein (including used alone and contained in other groups) refers to a saturated linear and branched aliphatic hydrocarbyl containing 1-20 carbon atoms, preferably containing 1-10 carbon atoms, more preferably containing 1-8 carbon atoms, such as methyl, ethyl, *n*-propyl, *iso*-propyl, *n*-butyl, *tert*-butyl, *iso*-butyl, pentyl, hexyl, heptyl, octyl, nonyl, decyl, 4,4-dimethylpentyl, 2,2,4-trimethylpentyl, undecyl, dodecyl, and isomers thereof; as well as the alkyl containing 1-4 substituents (with no further substitutions) selected from the group consisting of D, a halogen (preferably F, Br, Cl or I), an alkyl, an alkoxy, an aryl, an aryloxy, an aryl or diaryl substituted by an aryl, an aralkyl, an aralkoxy, an alkenyl, an alkynyl, a cycloalkyl, a cycloalkenyl, a cycloalkyl alkyl, a cycloalkyl alkoxy, an optionally substituted amino, a hydroxyl, a hydroxyl alkyl, an acyl, an aldehyde group, a heteroaryl, a heteroaryloxy, a heterocycloalkyl, a heterocycloalkoxy, an aryl heteroaryl, an arylalkoxycarbonyl, a heteroarylalkyl, a heteroarylalkoxy, an aryloxyalkyl, an aryloxyaryl, an alkylamino, an amido, an arylcarbonylamino, a nitro, a nitrile group, a sulphydryl, a haloalkyl, a trihaloalkyl and/or an alkylthio. In the present invention, "Cₓ₁-C_{y1}" alkyl (x1 and y1 are integers) having indicated number of carbon atoms, such as "C₁₋₄ alkyl", has the same definition of the "alkyl" described in this paragraph, except for the number of carbon atoms.

"Alkylidene" used herein (including used alone and contained in other groups) refers to a sub-saturated linear and branched aliphatic hydrocarbyl containing 1-20 carbon atoms, preferably containing 1-10 carbon atoms, more preferably containing 1-8 carbon atoms, such as methylene, ethylidene, propylidene, *iso*-propylidene, n-butylidene, *tert*-butylidene, *iso*-butylidene, pentylidene, hexylidene, heptylidene, octylidene, nonylidene, decylidene, bis(4,4-dimethylpentyl), bis(2,2,4-trimethylpentyl), undecylidene, dodecylidene, and the isomers thereof; including the alkylidene containing 1-4 substituents (without further substituents) selected from the group consisting of D, a halogen (preferably F, Br, Cl or I), an alkyl, an alkoxy, an aryl, an aryloxy, an aryl or diaryl substituted by an aryl, an aralkyl, an aralkoxy, an alkenyl, an alkynyl, an cycloalkyl, an cycloalkenyl, an cycloalkylalkyl, an cycloalkylalkoxy, an optionally substituted amino, an hydroxyl, an hydroxyalkyl, an acyl, an aldehyde group, an heteroaryl, an heteroaryloxy, an heterocycloalkyl, an heterocycloalkoxy, an arylheteroaryl, an arylalkoxycarbonyl, an heteroarylalkyl, an heteroarylalkoxy, an aryloxyalkyl, an aryloxyaryl, an alkylamino, an amido, an arylcarbonylamino, a nitro, a nitrile group, a sulphydryl, a haloalkyl, a trihaloalkyl and/or an alkylthio; one or more than one substituents together with the alkylidene can form a ring, thereby forming a fused ring or a spiro ring.

The term "aliphatic ring" or "cycloalkyl" (including used alone and contained in other groups) includes saturated or partially unsaturated (containing 1 or 2 double bonds) cyclic hydrocarbon group containing 1-3 rings, including monocycloalkyl, bicycloalkyl and tricycloalkyl which contains 3-20 carbon atoms which can form a ring, preferably contains 3-10 carbon atoms, e.g. cyclopropyl, cyclobutyl, cyclopentyl, cyclohexyl, cycloheptyl, cyclooctyl, cyclodecyl and cyclododecyl, cyclohexenyl; the cycloalkyl can be substituted by any of 1-4 substituents (without further substitutions) selected from the group consisting of D, a halogen, an alkyl, an alkoxy, a hydroxyl, an aryl, an aryloxy, an aralkyl, a cycloalkyl, an alkylamino, an amido, an oxo, an acyl, an arylcarbonylamino, an amino, a nitro, a nitrile group, a sulphydryl and/or an alkylthio and/or any alkyl group.

The term "alkoxy" refers to a cyclic or non-cyclic alkyl having indicated number of carbon atoms linked by an oxygen bridge. Therefore, "alkoxy" includes the definitions of the alkyl and the cycloalkyl.

The term "alkenyl" refers to a linear, branched or cyclic non-aryl hydrocarbyl having indicated carbon atoms and at least one carbon-carbon double bond. Preferably, it has one carbon-carbon double bond and can exist up to four non-aryl carbon-carbon double bonds. Therefore, "C₂₋₁₂ alkenyl" refers to an alkenyl having 2-12 carbon atoms. "C₂₋₆ alkenyl" refers to an alkenyl having 2-6 carbon atoms, including vinyl, propenyl, butenyl, 2-methyl butenyl and cyclohexenyl. The linear, branched and cyclic part of the alkenyl can contain a double bond, and if it is a substituted alkenyl, the alkenyl can be substituted (but the substituent with no further substitution).

The term "alkynyl" refers to a linear, branched or cyclic hydrocarbyl having indicated carbon atoms and at least one carbon-carbon triple bond. It can have up to three carbon-carbon triple bonds. Therefore, "C₂₋₁₂ alkynyl" refers to an alkynyl having 2-12 carbon atoms. "C₂₋₆ alkynyl" refers to an alkynyl having 2-6 carbon atoms, including ethynyl, propynyl, butynyl and 3-methyl butynyl etc.

The term "aryl" used herein refers to any stable monocyclic or bicyclic carbon rings which can have up to 7 atoms in each ring, and at least one of the rings is an aromatic ring. The typical aryl unit includes phenyl, naphthyl, tetrahydronaphthyl, 2, 3-dihydroindenyl, biphenyl, phenanthryl, anthryl or acenaphthyl. It can be understood that where the aryl is a bicyclic group and one of the ring is a non-aromatic ring, the linkage is through the aromatic ring. The aryl also includes any of 1-4 substituents (without further substitutions) selected from the group consisting of D, a halogen (F, Br Cl or I), an alkyl, an alkoxy, an aryl, an aryloxy, an aryl or diaryl substituted by an aryl, an aralkyl, an aralkoxy, an alkenyl, an alkynyl, a cycloalkyl, a cycloalkenyl, a cycloalkylalkyl, a cycloalkylalkoxy, an optionally substituted amino, a hydroxyl, a hydroxyalkyl, an acyl, an aldehyde group, a heteroaryl, a heteroaryloxy, a heterocycloalkyl, a heterocycloalkoxy, an arylheteroaryl, an arylalkoxycarbonyl, a heteroarylalkyl, a heteroarylalkoxy, an aryloxyalkyl, an aryloxyaryl, an alkylamino, an acylamino, an arylcarbonylamino, a nitro, a nitrile group, a sulphydryl, a haloalkyl, a trihaloalkyl and/or an alkylthio.

The term "halogen" refers to F, Cl, Br, I or At.

The term "hydroxyl" refers to

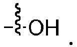

The term "amino" refers to

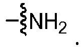

The term "cyano" refers to

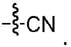

The term "carboxyl" refers to

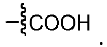

The term "sulfonyl" refers to

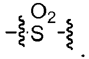

The term "acyl" refers to 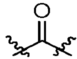 , a univalence group derived from an organic or inorganic oxygenic acid cleaving a hydroxyl off.

The term "haloalkyl" refers to an alkyl substituted by halogen at any position. Therefore, "haloalkyl" includes the definitions of the halogen and the alkyl.

The term "haloalkoxy" refers to an alkoxy substituted by halogen at any position. Therefore, "haloalkoxy" includes the definitions of the halogen and the alkoxy.

The term "aryloxy" refers to an aryl having indicated number of carbon atoms linked by an oxygen bridge. Therefore, "aryloxy" includes the definition of the aryl.

The term "aromatic hetero group" or "heteroaryl" used herein refers to a stable monocycle or bicycle which can have up to 7 atoms in each ring, and at least one of the rings is an aromatic ring containing 1-4 heteroatoms selected from O, N and S. The heteroaryl defined herein includes but not limited to acridinyl, carbazolyl, cinnolinyl, quinoxalinyl, pyrazolyl, indolyl, benzotriazolyl, furanyl, thienyl, benzothienyl, benzofuranyl, quinolinyl, isoquinolyl, oxazolyl, isoxazolyl, indolyl, pyrazinyl, pyridazinyl, pyridyl, pyrimidyl, pyrryl, tetrahydroquinoline. As defined in the heterocyclo, "heteroaryl" can also be understood including the N-Oxide derivative of any N-containing heteroaryl. Where the heteroaryl is a bicyclic group and one of the rings is a non-aromatic ring or without any heteroatom, it can be understood, the linkage is through the aryl or the ring containing the heteroatom. The heteroaryl can be substituted by any of 1-4 substituents selected from the group consisting of D, a halogen, an alkyl, an alkoxy, a hydroxyl, an aryl, an aryloxy, an aralkyl, a cycloalkyl, an alkylamino, an acylamino, an acyl, an arylcarbonylamino, an amino, a nitro, a nitrile group, a sulphydryl and/or an alkylthio and/or any of alkyl group.

The term "heterocyclo" or "heterocyclic group" used herein refers to a 5-10 membered aromatic or non-aromatic heterocycle having 1-4 heteroatoms selected from O, N and S, including bicyclic group. Therefore, "heterocyclic group" includes the aryl and the dihydro- or tetrahydro- analogues thereof. The embodiments of the "heterocyclic group" include but not limited to benzimidazolyl, benzofuranyl, benzofurazinyl, benzopyrazolyl, benzotriazolyl, benzothienyl, benzoxazolyl, carbazyl, carbazolyl, cinnolinyl, furanyl, imidazolyl, indolinyl, indolyl, indazolyl, isobenzofuranyl, pseudoindolyl, isoquinolyl, isothiazolyl, isoxazolyl, naphthalene pyrimidinyl, oxadiazolyl, oxazolyl, oxazoline, isoxazoline, oxycyclobutyl, pyranyl, pyrazinyl, pyrazolyl, pyridazinyl, pyridopyridyl, pyridazinyl, pyridyl, pyrimidyl, pyrryl, quinazolyl, quinolyl, quinoxalinyl, tetrahydropyranyl, tetrazolyl, tetrazolopyridyl, thiadiazolyl, thiazolyl, thienyl, triazolyl, azetidinyl, 1,4-dioxanyl, hexahydroazepinyl, piperazinyl, piperidinyl, pyrrolidinyl, morpholinyl, thiomorpholinyl, dihydrobenzimidazolyl, dihydrobenzofuranyl, dihydrobenzothienyl, dihydrobenzoxazolyl, dihydrofuranyl, dihydroimidazolyl, dihydroindolyl, dihydroisoxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyryl, dihydroquinolyl, dihydrotetrazolyl, dihydrothiadiazolyl, dihydrothiazolyl, dihydrothienyl, dihydrotriazolyl, dihydro-azetidinyl, methylenedioxybenzoyl, tetrahydrofuranyl, tetrahydrothienyl and N-Oxide thereof. The heterocyclo (without further substitution) can link with other groups by its carbon atom or heteroatom.

The term "cycloheteroaliphatic" or "heterocycloalkyl" used herein alone or contained in other groups refers to a saturated or partially unsaturated 4-12 membered ring having 1-4 heteroatoms (e.g. N, O and/or S). The heterocycloalkyl can contain 1-4 substituents (without further substitution), such as an alkyl, a halogen, an oxo and/or any of alkyl list above. Besides, any heterocycloalkyl can fuse to a cycloalkyl, an aryl, a heteroaryl or a heterocycloalkyl. The heterocycloalkyl can link to other groups through its carbon atom or heteroatom.

The term "aromatic ring" used herein refers to any stable monocyclic or bicyclic carbon rings which can have up to 7 atoms in each ring and at least one of the ring is an aromatic ring. The embodiments of the aromatic unit include phenyl, naphthyl, tetrahydronaphthyl, 2, 3-dihydroindenyl, biphenyl, phenanthryl, anthryl or acenaphthyl. It can be understood that when the aryl is a bicyclic group and one of the ring is a non-aromatic ring, the linkage is through "aromatic ring". The aromatic ring includes any of 1-4 substituents (without further substitution) selected from the group consisting of D, a halogen (F, Br, Cl or I), an alkyl, an alkoxy, an aryl, an aryloxy, an aryl or biaryl substituted with an aryl, an aralkyl, an aralkoxy, an alkenyl, an alkynyl, a cycloalkyl, a cycloalkenyl, a cycloalkylalkyl, a cycloalkylalkoxy, an amino, a hydroxyl, a hydroxyalkyl, an acyl, an aldehyde group, a heteroaryl, a heteroaryloxy, a heterocycloalkyl, a heterocycloalkoxy, an arylheteroaryl, an arylalkoxycarbonyl, a heteroarylalkyl, a heteroarylalkoxy, an aryloxyalkyl, an aryloxyaryl, an alkylamino, an acylamino, an arylcarbonylamino, a nitro, a nitrile group, a sulphydryl, a haloalkyl, a trihaloalkyl and/or an alkylthio.

The term "heteroaryl" or "aromatic heterocyclo" used herein refers to a stable monocyclic or bicyclic group which can have up to 7 atoms in each ring and at least one of the ring is an aromatic ring having 1-4 heteroatoms selected from O, N and S. In this definition, the heteroaryl includes but not limited to acridine, carbazole, cinnoline, carboline, quinoxaline, imidazole, pyrazole, pyrrole, indole, indoline, benzotriazole, benzimidazole, furan, thiophen, isothiazole, benzothiophene, dihydrobenzothiophene, benzofuran, isobenzofuran, benzoxazole, benzofuraxan, benzopyrazole, quinoline, isoindoline, isoquinoline, oxazole, oxadiazole, isoxazole, indole, pyrazine, pyridopyridine, tetrazolopyridine, pyridazine, pyridine, naphthalene pyrimidine, pyrimidine, pyrrole, tetrazole, thiadiazole, thiazole, thiophene, triazole, quinazoline, tetrahydroquinoline, dihydrobenzimidazole, dihydrobenzofuran, dihydrobenzoxazole, dihydroquinoline. As defined in the definition of heterocycle, "heteroaryl" is also understood to include N-Oxide derivatives of any N-containing heteroaryl. Where the heteroaryl is a bicyclic group and one of the ring is a non-aromatic ring or without any heteroatom, it can be understood that the linkage is through the aryl or the heteroatom contained in the ring. The heteroaryl can be substituted by any of 1-4 substituents (without further substitutions) selected from the group consisting of D, a halogen, an alkyl, an alkoxy, a hydroxyl, an aryl, an aryloxy, an aralkyl, a cycloalkyl, an alkylamino, an acylamino, an acyl, an arylcarbonylamino, an amino, a nitro, a nitrile group, a sulphydryl and/or an alkylthio and/or any alkyl defined in the present invention.

"Proactively effective amount and/or therapeutically effective amount" refers to an amount of the compound administered to a subject sufficient to prevent and/or treat the diseases involved in the present invention. Though the proactively effective amount and/or therapeutically effective amount of the compound depends on the compound, the condition and its severity, and the age of the subject to be treated, it can be determined by the person skilled in the art according to the common method.

As used in the present invention, when the specific salt, pharmaceutical composition, composition, excipient are mentioned to be "pharmaceutically acceptable", it means that the salt, pharmaceutical composition, composition, excipient are generally nontoxic, safe and suitable to be administered to the subject; the subject is preferably a mammal, more preferably human.

The term "pharmaceutically acceptable salt" as used herein refers to a pharmaceutically acceptable organic or inorganic salt of the compound of the present invention. Typical embodiments are include but not limited to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, gluconate, glucuronate, saccharate, formate, benzoate, glutamate, methylsulfonate, ethylsulfonate, benzene sulfonate, tosilate, embonate (i.e. 1-1-methylene-bis(2-hydroxyl-3-naphthoate)).

The compound of the present invention can contain one or more than one asymmetric centers ("stereoisomers"). As used herein, the term "stereoisomers" refers to *Cis-* and *Trans-* isomer, R- and S- antimer and diastereomer. These stereoisomers can be prepared by asymmetric synthesis or chiral separation (e.g. isolating, crystallizing, TLC, column chromatography, gas chromatography, HPLC). These stereoisomers can also derive from the diastereomer obtained from reacting a mixture of the enantiomers or racemate with a proper chiral compound, followed by crystallizing or conducting any other proper common method.

As used herein, the term "subject" refers to any animal to be administered or have been administered with the compound or the pharmaceutical composition according to the embodiment of the present invention, preferably a mammal, most preferably human. As used herein, the term "mammal" includes any mammal. Typical mammal includes but not limited to cattle, horse, sheep, pig, cat, dog, mouse, rat, rabbit, Guinea pig, monkey, human and so on, human is the most preferable.

In one embodiment, "treat" or "treating" refers to an improvement, prevention or reversion of a disease or a condition or at least one distinguished symptom thereof. In another embodiment, "treat" or "treating" refers to an improvement, prevention or reversion of at least one of measurable body parameters of a disease or a condition which is being treated, which may not been distinguished in a mammal. However, in another embodiment, "treat" or "treating" refers to slowing the development of a disease or a condition, or refers to stabilizing in body, such as a recognizable symptom, or refers to stabilizing in physiology, such as body parameters, or refers to both. In another embodiment, treat" or "treating" refers to slowing the initiation of a disease or a condition.

In certain embodiments, the claimed compound is administered for prevention. As used herein, "prevent" or "preventing" refers to lowering a risk of having a disease or a condition. In a preferred embodiment, administering an indicated compound to a subject for a preventive purpose, such as the subject having a tendency to catch or having a family history of cancer or autoimmune diseases.

In the present invention, abbr. "Abs" refers to the absolute configuration of the chiral carbon atom contained in the compound is unknown, indicating S-configuration or R-configuration.

In the present invention, 0.1% DEA refers to that DEA volume accounts for 0.1% volume of the mixture solution containing DEA, for example, in Hexane-0.1% DEA, 0.1% DEA refers to that DEA volume accounts for 0.1% total volume of Hexane and DEA. Additionally, the definition of 0.1% NH₄OH is the same as that of 0.1% DEA.

In the present invention, room temperature refers to ambient temperature, generally refers to 10-30°C.

Without departing from the common knowledge in the art, the optimized embodiments can be obtained by optionally combining the preferred conditions above.

The reagents and raw materials are commercially available.

The positive effects achieved by the present invention lie in that:
the present invention provides a condensed ring derivative which is totally distinguished from the prior art, the preparation method, the intermediate, the pharmaceutical composition and the use thereof. The condensed ring derivative of the present invention has distinct inhibitory effects against URAT1, which can relieve or treat hyperuricemia etc. and related diseases.

### Detailed description of the preferred embodiment

Embodiments 1, 18, 19, 20, 25, 26, 27, 28, 29, 35, 36, 38, 39, 40 and 50 are reference embodiments and are not according to the invention.

The structure of the compound is determined by NMR or MS, NMR is obtained by Bruker Avance-500 apparatus, d₆-DMSO, CDCl₃ and CD₃OD etc. as a solvent, TMS as an interior label. MS is obtained by LC-MS Agilent Technologies 6110, ESI as an ion source.

Microwave reaction is conducted in Explorer full automatic microwave irradiation equipment supplied by CEM, US Corporation, magnetron frequency is 2450MHz, continuous microwave output power is 300W.

HPLC is Gilson 281, the preparative column is Xbridge, 21.2 × 250mm C18, 10µm.

Process I for separating enantiomers: the apparatus is Gilson 281, the preparative column is r,r-Whelk-O1 (20 × 250mm, 5µm); process II: the apparatus is SFC-80 (Thar, Waters), the preparative column is AD 30 × 250mm, 5µm (Decial).

### Embodiment 1

### 4-(4-cyanophenyl)isoquinoline-6-carboxylic acid (Compound 1)

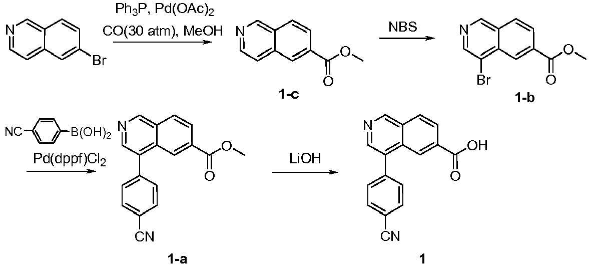

### Synthesis of compound 1-c

Under CO atmosphere (30 atm), 6-bromoisoquinoline (10.0g, 48mmol), sodium acetate (5.0g, 61mmol), triphenylphosphine (3.8g, 14mmol) and palladium acetate (2.8g, 12mmol) were dissolved in DMF (40mL) and methanol (40mL), the mixture was reacted at 100°C for 24hrs. The mixture was then cooled to room temperature, evaporated to remove methanol, the residue was filtered through celite, the filtrate cake was washed with EA (200mL). The filtrate was washed in turn with water (100 mL×3) and saturated brine (100 mL), dried over anhydrous sodium sulfate, filtered, concentrated under reduced pressure. The residue was purified with silica column chromatography (PE: EA = 10:1) to give white solid **1-c** (7g, yield: 78%). LC-MS (ESI): m/z = 188 [M+H]⁺

### Synthesis of compound 1-b

Compound **1-c** (1.88g, 10mmol) and N-bromosuccinimide (2.7g, 15mmol) were dissolved in acetic acid (40mL), the mixture was cooled to room temperature after reacting at 80°C for 24hrs. Part of acetic acid was removed under reduced pressure, the residue was filtered through celite, the filtrate cake was washed with DCM (200mL). The filtrate was in turn washed with saturated sodium sulfite solution (200mL), dried over anhydrous sodium sulfate, filtered, evaporated under reduced pressure. The residue was purified with silica column chromatography (PE:EA = 50:1) to give colorless solid **1-b** (2.5g, yield 94%). LC-MS (ESI): m/z = 266 [M+H]⁺.

### Synthesis of compound 1-a

Under N₂ atmosphere, compound **1-b** (133mg, 0.5mmol), 4-cyanophenylboronic acid (75mg, 0.5mmol) and sodium carbonate (60mg, 0.6mmol) were suspended in a mixed solution of dioxane (4mL) and water (1mL), [1,1'-bis(diphenylphosphine)ferrocene] palladium dichloride (25mg, 0.03mmol) was added. The mixture was stirred at 80°C for 3hrs, then cooled to room temperature. The mixture was filtered through celite, the filtrate cake was washed with EA (50mL). The filtrate was in turn washed with water (20mL×3) and saturated brine (10mL), dried over anhydrous sodium sulfate, filtered, evaporated under reduced pressure. The residue was purified by silica column chromatography (PE:EA = 1:1) to give compound **1-a** (126mg, yield 83%). LC-MS (ESI): m/z = 289 [M+H]⁺.

### Synthesis of compound 1

At room temperature, LiOH (42mg, 1.0mmol) was added to a solution of compound **1-a** (120mg, 0.42mmol) in a mixed solution of methanol (1mL), THF (4mL) and water (1mL). The mixture was stirred at room temperature for 1h, followed by adding 2M HCl aqueous solution (1mL) and water (20mL), solid was precipitated and filtered out. The solid was washed with water (10mL), dried under vacuum to give white solid **1** (91mg, yield 79%). LC-MS (ESI): m/z = 295 [M+H]⁺.
¹H-NMR (400MHz, DMSO-d6) δ: 13.55 (s, 1H), 9.53 (s, 1H), 8.60 (s, 1H), 8.34 (m, 2H), 8.21 (m, 1H), 8.10 (d, J=8.0 Hz, 1H), 7.82 (d, J=8.0 Hz, 1H) ppm.

### Embodiment 2

### 3-[4-(4-Cyanophenyl)isoquinolin-6-yl]propionic acid (Compound 2)

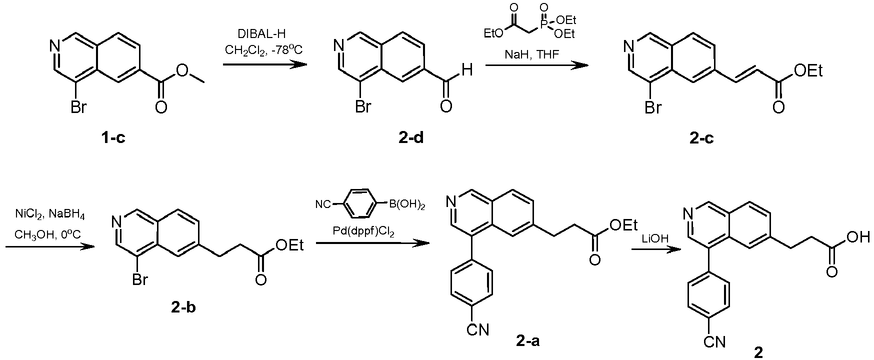

### Synthesis of compound 2-d

A solution of compound **1-c** (1.33g, 5mmol) in DCM (50mL) was cooled to -78°C, 1.0M diisobutylaluminum hydride in DCM (20mL, 20mmol) was slowly added dropwise, the mixture was further stirred for 1h. The mixture was warmed to room temperature, saturated aqueous solution of NH₄Cl (300mL) was added, organic phase was seperated, aqueous phase was extracted with DCM (50mL×3). The organic phases were combined, dried over anhydrous sodium sulfate, filtered, evaporated under reduced pressure. The residue was purified with silica column chromatography (PE:EA = 3:1) to give light yellow solid **2-d** (900 mg, yield 76%). LC-MS (ESI): m/z =236 [M+H]⁺.

### Synthesis of compound 2-c

At 0°C, triethyl phosphonoacetate (1.4mL, 5mmol) and sodium hydride (240mg, 6mmol) were added into a solution of compound **2-d** (470mg, 2mmol) in THF (10mL), the mixture was further stirred for 1h. The mixture was warmed to room temperature, followed by adding saturated aqueous solution of NH₄Cl (300mL), extracted with EA (50mL×3). The organic phases were combined, washed in turn with water (10mL×3) and saturated brine (10mL), dried over anhydrous sodium sulfate, filtered, evaporated under reduced pressure. The residue was purified with silica column chromatography (PE:EA = 5:1) to give light yellow solid **2-c** (380mg, yield 62%). LC-MS (ESI): m/z =306 [M+H]⁺.

### Synthesis of compound 2-b

At 0°C, NaBH₄ (40mg, 1mmol) was added slowly into a solution of compound **2-c** (310mg, 1mmol) and NiCl₂ (13mg, 0.1mmol) in methanol (5mL), the mixture was further stirred for 3hrs. The mixture was warmed to room temperature, followed by adding saturated aqueous solution of NH₄Cl (30mL), being extracted with EA (10mL×3). The organic phases were combined, washed in turn with water (5mL×3) and saturated brine (5mL), dried over anhydrous sodium sulfate, filtered, evaporated under reduced pressure. The residue was purified with silica column chromatography (PE:EA = 4:1) to give light yellow solid **2-b** (280mg, yield 91%). LC-MS (ESI): m/z =308 [M+H]⁺.

### Synthesis of compound 2-a

Under N₂ atmosphere, compound **2-b** (155mg, 0.5mmol), 4-cyanophenylboronic acid (75mg, 0.5mmol) and sodium carbonate (106mg, 1mmol) were suspended in a mixture of dioxane (4mL) and water (1mL), [1,1'-bis (diphenylphosphine)ferrocene]palladium dichloride (40mg, 0.05mmol) was added. The mixture was stirred at 80°C for 3hrs, then cooled to room temperature. The mixture was filtered through celite, the filtrate cake was washed with EA (50mL). The filtrate was in turn washed with water (20mL×3) and saturated brine (10mL), dried over anhydrous magnesium sulfate, filtered, evaporated under reduced pressure. The residue was purified with silica column chromatography (PE:EA = 1:1) to give compound **2-a** (100mg, yield 61%). LC-MS (ESI): m/z = 331 [M+H]⁺.

### Synthesis of compound 2

At room temperature, LiOH (42mg, 1.0mmol) was added to a mixed solution of compound **2-a** (100mg, 0.3mmol) in methanol (1mL), THF (4mL) and water (1mL), the mixture was further stirred for 1h, 2M HCl aqueous solution (1mL) and water (20mL) were added, solid was precipitated and filtered out. The solid was washed with water (10mL), dried under vacuum to give white solid **2** (61mg, yield 67%). LC-MS (ESI): m/z = 303 [M+H]⁺.
¹H-NMR (400MHz, DMSO-d6) δ: 12.18 (s, 1H), 9.34 (s, 1H), 8.44 (s, 1H), 8.18 (d, J=8.0 Hz, 1H), 8.04 (d, J=8.0 Hz, 2H), 7.77 (d, J=8.0 Hz, 2H), 7.68 (d, J=8.0 Hz, 1H), 2.99 (t, J=8.0 Hz, 2H), 2.59 (t, J=8.0 Hz, 1H) ppm.

### Embodiment 3

### 2-[4-(4-Cyanonaphthalen-1-yl)isoquinolin-6-yl]acetic acid (Compound 3)

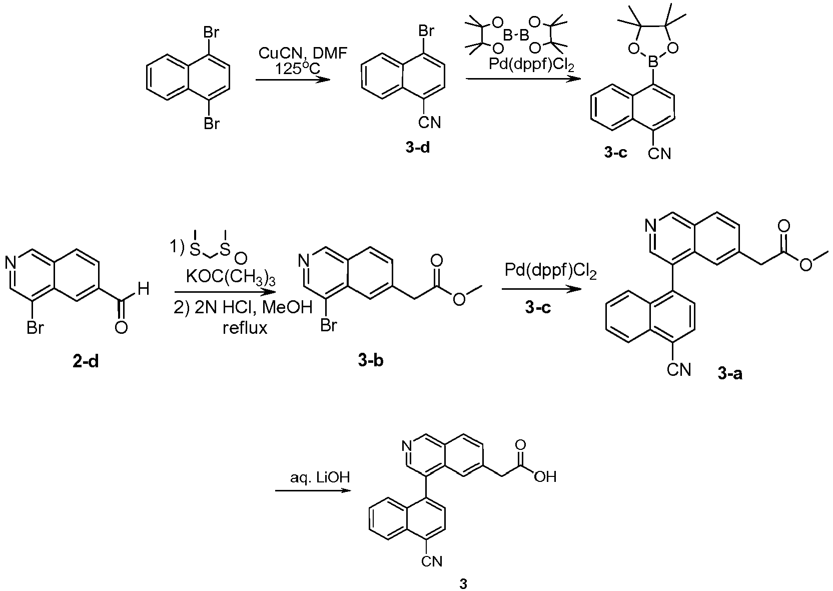

### Synthesis of compound 3-d

CuCN (5.0g, 56.3mmol) was added to a solution of 1,4-dibromonaphthalene (20g, 70.4mmol) in DMF (250mL), the mixture was reacted for 16hrs at 125°C, evaporated under reduced pressure. Aqueous ammonia (200mL) and EA (200mL) were added to the residue, the mixture was stirred for 1h and organic phase was seperated. The organic phase was in turn washed with water (100mL×3) and saturated brine (100mL), dried over anhydrous magnesium sulfate, filtered, evaporated under reduced pressure. The residue was purified with silica chromatography (PE:EA = 10:1) to give compound **3-d** (5.1g, yield 31%). LC-MS (ESI): m/z = 232 [M+H]⁺.

### Synthesis of compound 3-c

Under N₂ atmosphere, bis(pinacolato)diboron (8.4g, 33mmol), potassium acetate (6.5g, 66mmol) and [1,1'-bis(diphenylphosphine)ferrocene]palladium dichloride (1.2g, 1.76mmol) were respectively added to a solution of compound **3-d** (5.1g, 22mmol) in dioxane (150mL), the mixture was stirred at 80°C for 6hrs. The mixture was evaporated under reduced pressure, the residue was filtered through celite, the filtrate cake was washed with dioxane (50mL), the filtrate was evaporated under reduced pressure. The residue was purified with silica column chromatography (PE:EA = 10:1) to give compound **3-c** (6 g, yield 97%). LC-MS (ESI): m/z = 280 [M+H]⁺.

### Synthesis of compound 3-b

At 0°C, under N₂ atmosphere, potassium *tert*-butanolate (71mg, 0.63mmol) was added to a solution of methyl(methylthiomethyl)sulfoxide (78mg, 0.63mmol) in anhydrous THF (5mL). The mixture was stirred for 30mins, followed by adding compound **2-d** (100mg, 0.42mmol), stirred for 1h at room temperature, then evaporated under reduced pressure. 2M HCl in methanol (5mL) was added to the residue, the mixture was refluxed for 3hrs, then concentrated under reduced pressure. The residue was purified with silica column chromatography (PE:EA = 5:1) to give compound **3-b** (70 mg, yield 60%). LC-MS (ESI): m/z = 281 [M+H]⁺.

### Synthesis of compound 3-a

Under N₂ atmosphere, compound **3-c** (40mg, 0.14mmol), sodium carbonate (46mg, 0.43mmol) and [1,1'-bis(diphenylphosphine)ferrocene]palladium dichloride (30mg, 0.036mmol) were respectively added to a mixed solution of compound **3-b** (40mg, 0.14mmol) in ethylene glycol dimethyl ether (150mL) and water (1mL). The mixture was stirred at 75°C for 16hrs, and then concentrated under reduced pressure. The residue was purified by silica column chromatography (PE:EA = 2:1) to give compound **3-a** (27 mg, yield 53%). LC-MS (ESI): m/z = 353 [M+H]⁺.

### Synthesis of compound 3

At room temperature, 1M LiOH aqueous solution (1.0mL) was added to a mixed solution of compound **3-a** (27mg, 0.076mmol) in methanol (5mL) and THF (5mL), the mixture was stirred for 16hrs, and evaporated under reduced pressure. The residue was dissolved with water (6mL), adjusted to pH=3 with 1M citric acid aqueous solution, solid was precipitated and filtered out. The solid was washed with water (5mL), dried under vacuum to give white solid **3** (20mg, yield 77%). LC-MS (ESI): m/z = 339 [M+H]⁺.
¹H-NMR (400MHz, DMSO-d6) δ: 12.34 (s, br., 1H), 9.47 (s, 1H), 8.45 (s, 1H), 8.35 (d, J=8.0 Hz, 1H), 8.27 (d, J=8.0 Hz, 1H), 8.24 (d, J=8.0 Hz, 1H), 7.85 (t, J=8.0 Hz, 1H), 7.73 (d, J=8.0 Hz, 2H), 7.64 (d, J=8.0 Hz, 1H), 7.59 (t, J=8.0 Hz, 1H), 7.41 (d, J=8.0 Hz, 1H), 7.14 (s, 1H), 3.63 (s, 2H) ppm.

### Embodiment 4

### 2{[4-(4-Cyanophenyl)isoquinolin-6-yl]thio-2-methylpropionic acid (Compound 4)

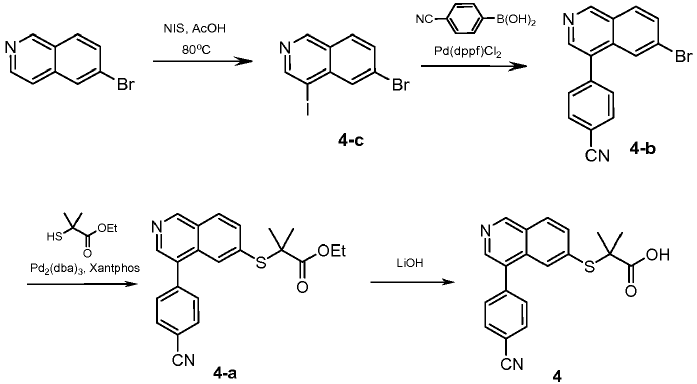

### Synthesis of compound 4-c

6-Bromoisoquinoline (10.4g, 50mmol) and N-iodosuccinimide (13.5g, 60mmol) were dissolved in acetic acid (100mL), the mixture was reacted for 8hrs at 80°C. The mixture was cooled to room temperature, followed by concentrating under reduced pressure to remove half of acetic acid, the residue was filtered through celite, the filtrate cake was washed with DCM (200mL), the organic phase was in turn washed with saturated sodium sulfite solution (200mL) and water (100mL), dried over anhydrous magnesium sulfate, filtered, concentrated under reduced pressure. The residue was purified with silica column chromatography (PE:EA = 5:1) to give compound **4-c** (11.6g, yield 70%). LC-MS (ESI): m/z = 334 [M+H]⁺.

### Synthesis of compound 4-b

Under N₂ atmosphere, compound **4-c** (2.33g, 10mmol), 4-cyanobenzene boronic acid (1.5g, 10mmol) and sodium carbonate (2.12g, 20mmol) were suspended in dioxane (40mL) and water (10mL), [1,1'-bis(diphenylphosphine)ferrocene]palladium dichloride (0.55g, 1mmol) was added. The mixture was stirred at 80°C for 3hrs, and then cooled to room temperature. The mixture was filtered through celite, the filtrate cake was washed with EA (50mL). The filtrate was in turn washed with water (100mL×3) and saturated brine (100mL), dried over magnesium sulfate, filtered, concentrated under reduced pressure. The residue was purified by silica column chromatography (PE:EA = 3:1) to give compound **4-b** (1.6g, yield 52%). LC-MS (ESI): m/z = 309 [M+H]⁺.

### Synthesis of compound 4-a

Under N₂ atmosphere, tris(dibenzylidene indene acetone)dipalladium (0.29g, 0.5mmol) and 4,5-bis(diphenylphosphine)-9,9-dimethyloxacanthracene (0.46mg, 0.5mmol) were added to a solution of compound **4-b** (1.5g, 5mmol), ethyl 2-methyl-2-mercaptopropionate (0.75g, 5mmol) and diisopropylethylamine (1.29g, 1mmol) in dioxane (8mL), the mixture was reacted in microwave at 110°C for 30mins. The mixture was cooled to room temperature, concentrated under reduced pressure to remove dioxane. The residue was filtered through celite, the filtrate cake was washed with EA (200mL). The filtrate was in turn washed with water (100mL×3) and saturated brine (100mL), dried over anhydrous magnesium sulfate, filtered, concentrated under reduced pressure. The residue was purified by silica column chromatography (PE:EA = 3:1) to give compound **4-a** (1.39g, yield 74%). LC-MS (ESI): m/z = 377 [M+H]⁺.

### Synthesis of compound 4

At room temperature, LiOH (0.178g, 0.74mmol) was added to a mixed solution of compound **4-a** (1.39g, 0.37mmol) in methanol (5mL), THF (20mL) and water (5mL), the mixture was stirred at room temperature for 1h, 2M HCl aqueous solution (2mL) and water (20mL) were added, solid was precipitated and filtered out. The solid was washed with water (10mL), dried under vacuum to give white solid **4** (1.1g, yield 85%). LC-MS (ESI): m/z = 349 [M+H]⁺.
¹H-NMR (400MHz, DMSO-d6) δ: 12.78 (s, 1H), 9.41 (s, 1H), 8.53 (s, 1H), 8.23 (d, J=8.0 Hz, 1H), 8.06 (d, J=8.0 Hz, 2H), 7.73 (m, 4H), 1.44 (s, 6H) ppm.

### Embodiment 5

### 2{[4-(4-Cyanonaphthalen)isoquinolin-6-yl]thio}-2-methylpropionic acid (Compound 5)

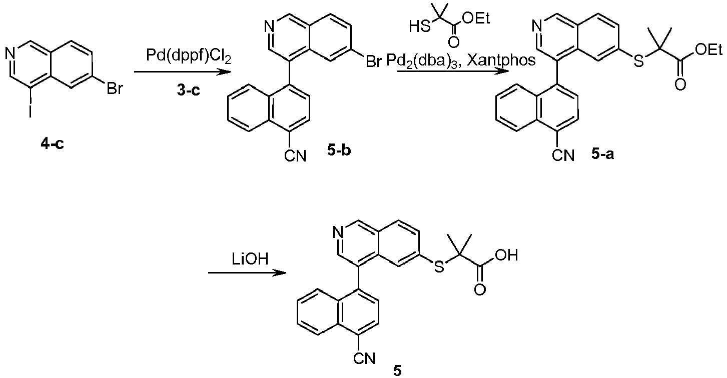

### Synthesis of compound 5-b

Under N₂ atmosphere, compound **4-c** (0.66g, 2mmol), compound **3-c** (0.56g, 2mmol) and sodium carbonate (0.42g, 0.4mmol) were suspended in dioxane (4mL) and water (1mL), [1,1'-bis(diphenylphosphine)ferrocene]palladium dichloride (0.12g, 0.2mmol) was added. The mixture was stirred at 80°C for 3hrs, and then cooled to room temperature, filtered through celite, washed with EA (20mL). The filtrate was in turn washed with water (10mL×3) and saturated brine (10mL), dried over anhydrous magnesium sulfate, filtered, concentrated under reduced pressure. The residue was purified by silica column chromatography (PE:EA = 3:1) to give compound **5-b** (0.46g, yield 64%). LC-MS (ESI): m/z = 359 [M+H]⁺.

### Synthesis of compound 5-a

Under N₂ atmosphere, tris(dibenzylidene indene acetone)dipalladium (0.06g, 0.1mmol) and 4,5-bis(diphenylphosphine)-9,9-dimethyloxacanthracene (0.1g, 0.1mmol) were added to a solution of compound **5-b** (0.36g, 1mmol), ethyl 2-methyl-2-mercaptopropionate (0.15g, 1mmol) and diisopropylethylamine (0.26g, 2mmol) in dioxane (8mL), the mixture was reacted in a microwave at 110°C for 30mins. The mixture was cooled to room temperature, and then concentrated under reduced pressure to remove dioxane, the residue was filtered through celite, the filtrate cake was washed with EA (50mL). The filtrate was in turn washed with water (50mL×3) and saturated brine (50mL), dried over anhydrous magnesium sulfate, filtered, concentrated under reduced pressure. The residue was purified by silica column chromatography (PE:EA = 3:1) to give compound **5-a** (0.37g, yield 87%). LC-MS (ESI): m/z = 427 [M+H]⁺.

### Synthesis of compound 5

At room temperature, LiOH (42mg, 1mmol) was added to a mixed solution of compound **5-a** (370mg, 0.86mmol) in methanol (1mL), THF (4mL) and water (1mL), the mixture was stirred for 1h, and then 2M HCl aqueous solution (2mL) and water (20mL) were added, solid was precipitated and filtered out. The solid was washed with water (10mL), dried under vacuum to give white solid **5** (280mg, yield 82%). LC-MS (ESI): m/z = 399 [M+H]⁺.
¹H-NMR (400MHz, DMSO-d6) δ: 12.67 (s, 1H), 9.51 (s, 1H), 8.57 (s, 1H), 8.35 (d, J=7.6 Hz, 1H), 8.27(m, 2H), 7.85 (t, J=7.6 Hz, 1H), 7.75 (d, J=7.6 Hz, 1H), 7.66 (d, J=8.0 Hz, 1H), 7.60 (t, J=8.0 Hz, 2H), 7.41 (d, J=8.0 Hz, 1H), 7.26 (s, 1H), 1.27 (s, 3H), 1.24 (s, 3H) ppm.

### Embodiment 6

### 2{[4-(4-Cyanophenyl)isoquinolin-6-yl]oxy}-2-methylpropionic acid (Compound 6)

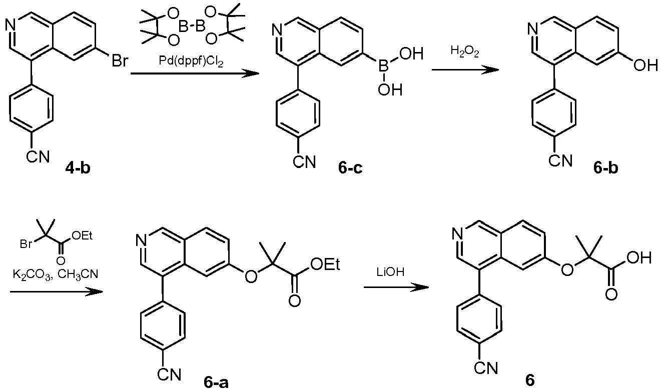

### Synthesis of compound 6-c

Under N₂ atmosphere, bis(pinacolato)diboron (3.1g, 12mmol), potassium acetate (2.0g, 20mmol) and [1,1'-bis(diphenylphosphine)ferrocene]palladium dichloride (0.56g, 1mmol) were respectively added to a solution of compound **4-b** (3.1g, 10mmol) in dioxane (15mL), the mixture was stirred at 80°C for 8hrs. The mixture was cooled to room temperature, filtered through celite, the filtrate cake was washed with EA (50mL). The filtrate was in turn washed with water (50mL×3) and saturated brine (50mL), dried over anhydrous magnesium sulfate, filtered, concentrated under reduced pressure. The residue was purified with silica column chromatography (PE:EA = 1:1) to give white solid **6-c** (1.76 g, yield 63%). LC-MS (ESI): m/z = 275 [M+H]⁺.

### Synthesis of compound 6-b

At 0°C, 30% H₂O₂ solution (2mL) was added to a solution of compound **6-c** (1.1g, 4mmol) in THF (20mL), the mixture was stirred for 4hrs before water (100mL) was added. The mixture was extracted with EA (100mL×3), the combined organic phases were washed in turn with water (50mL×3) and saturated brine (50mL), dried over anhydrous magnesium sulfate, filtered, concentrated under reduced pressure. The residue was purified by silica column chromatography (PE:EA = 1:1) to give white solid **6-b** (0.5g, yield 50%). LC-MS (ESI): m/z = 247 [M+H]⁺.

### Synthesis of compound 6-a

Under N₂ atmosphere, ethyl 2-bromoisobutyrate (190mg, 1mmol) and potassium carbonate (138mg, 1mmol) were added to a solution of compound **6-b** (75mg, 0.3mmol) in acetonitrile (4mL), the mixture was reacted for 3hrs at 80°C. The mixture was cooled to room temperature, concentrated under reduced pressure. The residue was filtered through celite, the filtrate cake was washed with EA (50mL). The filtrate was in turn washed with water (8mL×3) and saturated brine (10mL), dried over anhydrous magnesium sulfate, filtered, concentrated under reduced pressure. The residue was purified by silica column chromatography (PE:EA = 3:1) to give yellow liquid **6-a** (56 mg, yield 52%). LC-MS (ESI): m/z = 361 [M+H]⁺.

### Synthesis of compound 6

At room temperature, LiOH (42mg, 1mmol) was added to a mixed solution of compound **6-a** (56mg, 0.156mmol) in methanol (1mL), THF (2mL) and water (1mL), the mixture was stirred for 1h, followed by adding 2M HCl aqueous solution (2mL) and water (1mL), solid was precipitated and filtered. The solid was washed with water (5mL), dried under vacuum to give white solid **6** (32mg, yield 62%). LC-MS (ESI): m/z = 333 [M+H]⁺.
¹H-NMR (400MHz, DMSO-d6) δ: 13.27 (s, 1H), 9.24 (s, 1H), 8.39 (s, 1H), 8.16 (d, J=8.0 Hz, 1H), 8.02 (d, J=8.0Hz, 1H), 7.72 (d, J=8.0 Hz, 2H), 7.30 (d, J=8.0 Hz, 2H), 7.26 (s, 1H), 6.94 (s, 1H), 1.55 (s, 6H) ppm.

### Embodiment 7

### Compound 7A

### Compound 7B

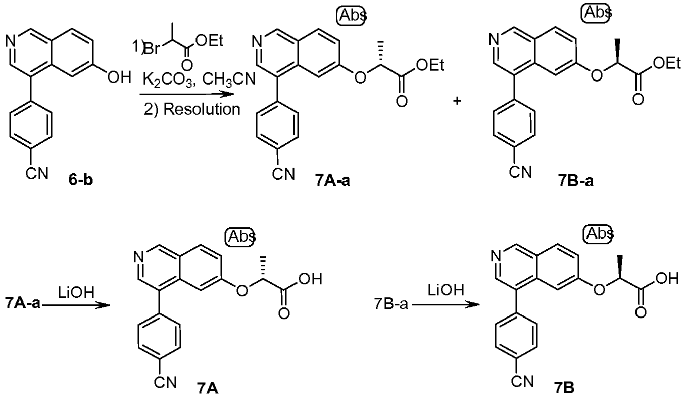

### Synthesis of compound 7A-a and 7B-a

Under N₂ atmosphere, ethyl 2-bromopropionate (540mg, 3mmol) and potassium carbonate (560mg, 4mmol) were added to a solution of compound **6-b** (500mg, 2mmol) in acetonitrile (20mL), the mixture was reacted for 6hrs at 80°C. The mixture was cooled to room temperature, concentrated under reduced pressure, the residue was filtered through celite, the filtrate cake was washed with EA (200mL). The filtrate was washed with water (50mL×3) and saturated brine (50mL), dried over anhydrous magnesium sulfate, filtered, concentrated under reduced pressure. The residue was purified by silica column chromatography (PE:EA = 3:1) to give yellow liquid, followed by separating with enantiomeric chromatographic column (process I, mobile phase: Hexane: EtOH: DEA =70:30:0.1) to give enantiomer **7A-a** which is obtained firstly (80 mg, yield 11.5%; LC-MS (ESI): m/z = 347 [M+H]⁺) (Tᵣ = 9.0 min) and enantiomer **7B-a** which is obtained later (90 mg, yield 13%; LC-MS (ESI): m/z = 347 [M+H]⁺) (Tᵣ = 11.0 min). The absolute configuration of **7A-a** and **7B-a** is unknown.

### Synthesis of compound 7A

At room temperature, LiOH (42mg, 1mmol) was added to a mixed solution of compound **7A-a** (70mg, 0.2mmol) in methanol (1mL), THF (2mL) and water (1mL), the mixture was stirred for 1h, followed by adding 2M HCl aqueous solution (2mL) and water (2mL), solid was precipitated and filtered out. The solid was washed with water (5mL), dried under vacuum to give white solid **7A** (51mg, yield 80%). LC-MS (ESI): m/z = 319 [M+H]⁺.
¹H-NMR (400MHz, DMSO-d6) δ: 13.15 (s, 1H), 9.24 (s, 1H), 8.40 (s, 1H), 8.19 (d, J=8.0 Hz, 1H), 8.01 (d, J=8.0Hz, 1H), 7.73 (d, J=8.0 Hz, 2H), 7.40 (d, J=8.0 Hz, 2H), 7.26 (s, 1H), 6.94 (s, 1H), 4.89 (m, 1H), 1.53 (d, J=8.0 Hz, 3H) ppm.

### Synthesis of compound 7B

At room temperature, LiOH (42mg, 1mmol) was added to a mixed solution of compound **7B-a** (70mg, 0.2mmol) in methanol (1mL), THF (2mL) and water (1mL), the mixture was stirred for 1h, followed by adding 2M HCl aqueous solution (2mL) and water (2mL), solid was precipitated and filtered out. The solid was washed with water (5mL), dried under vacuum to give white solid **7B** (46mg, yield 72%). LC-MS (ESI): m/z = 319 [M+H]⁺.
¹H-NMR (400MHz, DMSO-d6) δ: 13.15 (s, 1H), 9.24 (s, 1H), 8.40 (s, 1H), 8.19 (d, J=8.0 Hz, 1H), 8.01 (d, J=8.0Hz, 1H), 7.73 (d, J=8.0 Hz, 2H), 7.40 (d, J=8.0 Hz, 2H), 7.26 (s, 1H), 6.94 (s, 1H), 4.89 (m, 1H), 1.53 (d, J=8.0 Hz, 3H) ppm.

### Embodiment 8

### 2{[4-(4-Cyano-2-fluorophenyl)isoquinolin-6-yl]thio}-2-methylpropionic acid (Compound 8)

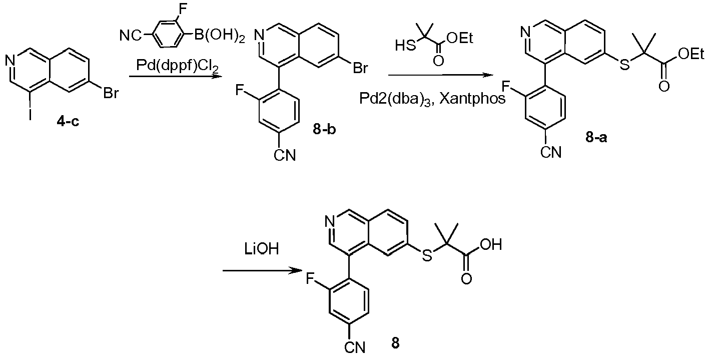

### Synthesis of compound 8-b

Under N₂ atmosphere, compound **4-c** (330mg, 1mmol), 2-fluoro-4-cyanophenylboronic acid (165mg, 1mmol) and sodium carbonate (212mg, 0.2mmol) were suspended in a mixed solution of dioxane (4mL) and water (1mL), [1,1'-bis(diphenylphosphine)ferrocene]palladium dichloride (56mg, 0.1mmol) was added. The mixture was stirred at 80°C for 3hrs, cooled to room temperature, filtered through celite, the filtrate cake was washed with EA (20mL). The filtrate was in turn washed with water (10mL×3) and saturated brine (10mL), dried over anhydrous magnesium sulfate, filtered, concentrated under reduced pressure. The residue was purified by silica column chromatography (PE:EA = 3:1) to give compound **8-b** (63mg, yield 19%). LC-MS (ESI): m/z = 387 [M+H]⁺.

### Synthesis of compound 8-a

Under N₂ atmosphere, tris(dibenzylidene indene acetone)dipalladium (12mg, 0.02mmol) and 4,5- bis(diphenylphosphine)-9,9-dimethyloxacanthracene (20mg, 0.02mmol) were added to a solution of compound **8-b** (63mg, 0.2mmol), ethyl 2-methyl-2-mercaptopropionate (30mg, 0.2mmol) and diisopropylethylamine (52mg, 0.4mmol) in dioxane (8mL), the mixture was reacted in a microwave at 110°C for 30mins. The mixture was cooled to room temperature, followed by evaporating dioxane under reduced pressure, the residue was filtered through celite, the filtrate cake was washed with EA (50mL). The filtrate was in turn washed with water (10mL×3) and saturated brine (10mL), dried over anhydrous magnesium sulfate, filtered, concentrated under reduced pressure. The residue was purified by silica column chromatography (PE:EA = 3:1) to give compound **8-a** (72mg, yield 91.3%). LC-MS (ESI): m/z = 395 [M+H]⁺.

### Synthesis of compound 8

At room temperature, LiOH (42mg, 1mmol) was added to a mixed solution of compound **8-a** (72mg, 0.18mmol) in methanol (1mL), THF (2mL) and water (1mL). The mixture was stirred for 1h, followed by adding 2M HCl aqueous solution (2mL) and water (2mL), solid was precipitated and filtered out. The solid was washed with water (5mL), dried under vacuum to give white solid **8** (16mg, yield 24%). LC-MS (ESI): m/z = 367 [M+H]⁺.
¹H-NMR (400MHz, DMSO-d6) δ: 12.8 (s, 1H), 9.44 (s, 1H), 8.54 (s, 1H), 8.23 (d, J=7.6 Hz, 1H), 8.10 (d, J=7.6 Hz, 1H), 7.92 (d, J=7.6 Hz, 1H), 7.78 (t, J=7.6 Hz, 1H), 7.70 (d, J=7.6 Hz, 1H), 7.59 (s, 1H), 1.44 (s, 6H) ppm.

### Embodiment 9

### 2{[4-(4-Cyanonaphthalen-1-yl)-8-fluoroisoquinolin-6-yl]thio}-2-methylpropionic acid (Compound 9)

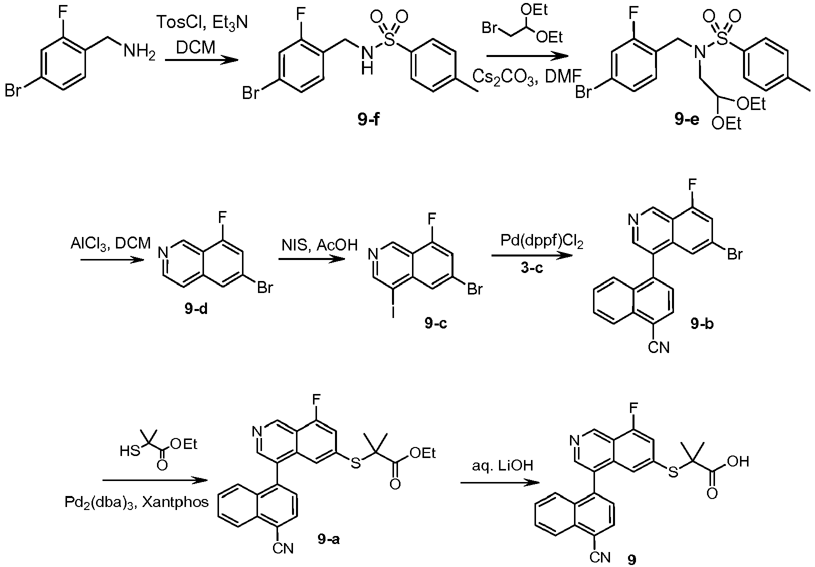

### Synthesis of compound 9-f

At 0°C, *p*-toluenesulfonyl chloride (4.00g, 21mmol) was added in portion to a solution of 5-bromo-2-fluorobenzylamine (4.08g, 20mmol) and triethylamine (4.04g, 40mmol) in DCM (60mL). The mixture was reacted at 0°C for 30mins, followed by removing the ice bath, further reacting at room temperature for 16hrs, then being concentrated under reduced pressure. The residue was purified by silica column chromatography (PE:EA = 5:1) to give compound **9-f** (5.30g, yield 74%). LC-MS (ESI): m/z = 358 [M+H]⁺.

### Synthesis of compound 9-e

At room temperature, 2-bromo-1,1-diethoxyethane (3.0g, 15mmol), cesium carbonate (6.5g, 20mmol) were added to a solution of compound **9-f** (3.57g, 10mmol) in DMF (15mL). The mixture was reacted for 16hrs at 80°C, concentrated under reduced pressure. The residue was purified by column chromatography (PE:EA = 8:1) to give compound **9-e** (3.80g, 80%).

### Synthesis of compound 9-d

At -5°C, compound **9-e** (1.50mg, 3.18mmol) was added to a mixture of AlCl₃ (2.0g, 15mmol) in DCM (20mL). The mixture was reacted for 16hrs at room temperature, followed by adding 2M HCl aqueous solution (20mL), extracted with DCM (30mL×3). The organic phases were combined, washed in turn with water (10mL×3) and saturated brine (20mL), dried over anhydrous magnesium sulfate, filtered, concentrated under reduced pressure. The residue was purified by silica column chromatography (PE:EA = 7:1) to give compound **9-d** (220mg, yield 31%). LC-MS (ESI): m/z = 226 [M+H]⁺.

### Synthesis of compound 9-c

Compound **9-d** (200mg, 0.89mmol) and N-iodosuccinimide (300mg, 1.33mmol) were dissolved in acetic acid (10mL) and trifluoroacetic acid (2mL), the mixture was reacted for 6hrs at 80°C. The mixture was cooled to room temperature, concentrated under reduced pressure to remove the solvent. The residue was purified by silica column chromatography (PE:EA = 8:1) to give compound **9-c** (200mg, yield 64%). LC-MS (ESI): m/z = 352 [M+H]⁺.

### Synthesis of compound 9-b

Under N₂ atmosphere, compound **9-c** (144mg, 0.4mmol), compound **3-c** (111mg, 0.4mmol) and sodium carbonate (170mg, 1.6mmol) were suspended in ethylene glycol dimethyl ether (10mL) and water (1mL), [1,1'-bis(diphenylphosphine)ferrocene]palladium dichloride (43mg, 0.05mmol) was added. The mixture was reacted for 4hrs at 50°C, cooled to room temperature, concentrated under reduced pressure. The residue was purified by silica column chromatography (PE:EA = 3:1) to give compound **9-b** (100mg, yield 66%). LC-MS (ESI): m/z = 377 [M+H]⁺.

### Synthesis of compound 9-a

Under N₂ atmosphere, tris(dibenzylidene indene acetone)dipalladium (30mg, 0.033mmol) and 4,5-bis(diphenylphosphine))-9,9-dimethyloxacanthracene (38mg, 0.066mmol) were added to a solution of compound **9-b** (110mg, 0.29mmol), ethyl 2-methyl-2-mercaptopropionate (64mg, 0.44mmol) and diisopropylethylamine (187mg, 1.45mmol) in dioxane (10mL). The mixture was reacted for 5hrs at 100°C, followed by cooling to room temperature, concentrating under reduced pressure to remove dioxane. The residue was purified by silica column chromatography (PE:EA = 4:1) to give compound **9-a** (120mg, yield 93%). LC-MS (ESI): m/z = 445 [M+H]⁺.

### Synthesis of compound 9

At room temperature, 1M LiOH aqueous solution (2.0mL) was added to a mixed solution of compound **9-a** (100mg, 0.22mmol) in methanol (8mL) and THF (8mL). The mixture was stirred at room temperature for 16hrs, followed by being concentrated under reduced pressure. The residue was dissolved with water (10mL), adjusted to pH=3 with 1M citric acid aqueous solution, solid was precipitated and filtered out. The solid was washed with water (10mL), dried under vacuum to give white solid **9** (70 mg, yield 76%). LC-MS (ESI): m/z = 417 [M+H]⁺.
¹H-NMR (400MHz, DMSO-d6) δ: 12.79 (s, br., 1H), 9.61 (s, 1H), 8.68 (s, 1H), 8.35 (d, J=8.0 Hz, 1H), 8.28 (d, J=8.0 Hz, 1H), 7.86 (d, J=7.2 Hz, 1H), 7.75 (d, J=8.0 Hz, 1H), 7.60 (t, J=8.0 Hz, 1H), 7.51 (d, J=10.4 Hz, 1H), 7.42 (d, J=11.2 Hz, 1H), 7.07 (s, 1H), 1.29 (s, 3H), 1.22 (s, 3H) ppm.

### Embodiment 10

### 2{[4-(5-Cyanonaphthalen-1-yl)isoquinolin-6-yl]thio}-2-methylpropionic acid (Compound 10)

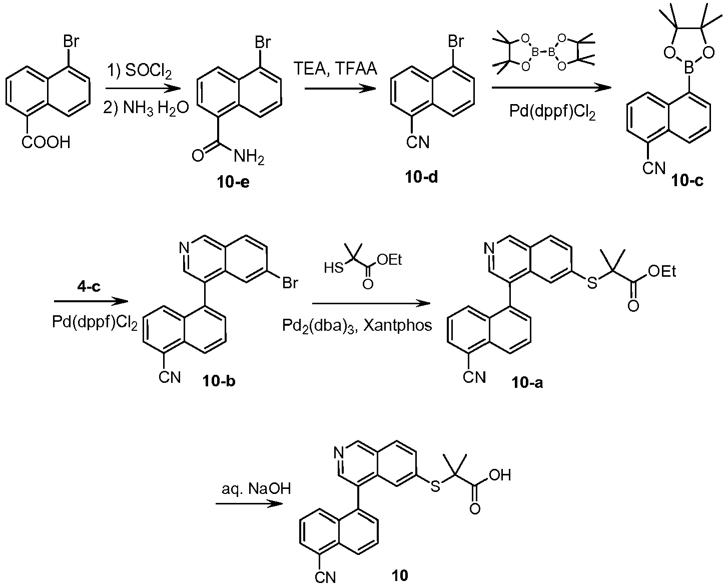

### Synthesis of compound 10-e

5-Bromo-1-naphthoic acid (980mg, 3.92mmol) was added to thionyl chloride (5mL). The mixture was stirred at 85°C for 2hrs, concentrated under reduced pressure. The residue was dissolved in anhydrous THF (10mL), the solution was added dropwise into 25%-28% aqueous ammonia (20mL) at 0°C. The mixture was warmed to room temperature and further stirred for 2hrs, followed by being extracted with EA (60mL×3). The organic phases were combined, dried over anhydrous sodium sulfate, filtered, and concentrated under reduced pressure to give white solid **10-e** (950mg, yield 97%). The product was used directly for the next step without further purification. LC-MS (ESI): m/z = 250 [M+H]⁺.

### Synthesis of compound 10-d

At 0°C, trifluoroacetic anhydride (3.2g, 15.1mmol) was added dropwise to a solution of compound **10-e** (0.94g, 3.78mmol) and triethylamine (1.53g, 15.1mmol) in THF (8mL). The mixture was slowly warmed to room temperature and further reacted for 3hrs, and then concentrated under reduced pressure. The residue was purified by silica column chromatography (PE:EA = 10:1) to give compound **10-d** (0.85g, yield 97%). LC-MS (ESI): m/z = 232 [M+H]⁺.

### Synthesis of compound 10-c

Under N₂ atmosphere, bis(pinacolato)diboron (1.32g, 5.2mmol), potassium acetate (1.0g, 10.38mmol) and [1,1'-bis(diphenylphosphine)ferrocene]palladium dichloride (0.253 g, 0.346 mmol) were added respectively to a solution of compound **10-d** (0.8g, 3.46mmol) in dioxane (15mL). The mixture was stirred at 80°C for 6hrs, concentrated under reduced pressure. The residue was purified by silica column chromatography (PE:EA = 10:1) to give compound **10-c** (0.85g, yield 88%). LC-MS (ESI): m/z = 280 [M+H]⁺.

### Synthesis of compound 10-b

Under N₂ atmosphere, compound **10-c** (200mg, 0.72mmol), compound **4-c** (200mg, 0.6mmol) and sodium carbonate (130mg, 1.2mmol) were suspended in dioxane (20mL) and water (2mL), [1,1'-bis(diphenylphosphine)ferrocene]palladium dichloride (50mg, 0.06mmol) was added. The mixture was reacted for 2hrs at 80°C, cooled to room temperature, filtered through celite, the filtrate cake was washed with EA (20mL), the filtrate was concentrated under reduced pressure. The residue was purified by silica column chromatography (PE:EA = 3:1) to give compound **10-b** (50mg, yield 81%). LC-MS (ESI): m/z = 359 [M+H]⁺.

### Synthesis of compound 10-a

Under N₂ atmosphere, tris(dibenzylidene indene acetone)dipalladium (16mg, 0.015mmol) and 4,5-bis(bis(diphenylphosphine)-9,9-dimethyloxacanthracene (17mg, 0.03mmol) were added to a solution of compound **10-b** (53mg, 0.15mmol), ethyl 2-methyl-2-mercaptopropionate (28mg, 0.19mmol) and diisopropylethylamine (38mg, 0.29mmol) in dioxane (5mL). The mixture was reacted for 6hrs at 100°C, cooled to room temperature, followed by being concentrated under reduced pressure to remove dioxane. The residue was purified by silica column chromatography (PE:EA = 2:1) to give compound **10-a** (45mg, yield 71%). LC-MS (ESI): m/z = 427 [M+H]⁺.

### Synthesis of compound 10

At room temperature, 1M NaOH aqueous solution (2.5mL) was added to a solution of compound **10-a** (59mg, 0.14mmol) in methanol (5mL), the mixture was stirred for 5hrs, followed by adding 1M HCl aqueous solution to adjust pH=6, being concentrated under reduced pressure to remove methanol, solid was precipitated and filtered out. The solid was washed with water (5mL), dried under vacuum to give white solid **10** (43mg, yield 78%). LC-MS (ESI): m/z = 399 [M+H]⁺.
¹H-NMR (400MHz, DMSO-d6) δ: 12.63 (s, 1H), 9.50 (s, 1H), 8.56 (s, 1H), 8.28 (m, 3H), 7.99 (m, 1H), 7.78 (d, J=6.3 Hz, 1H), 7.61 (m, 3H), 7.26 (s, 1H), 1.29 (s, 3H), 1.23 (s, 3H) ppm.

### Embodiment 11

### 2-{[4-(8-Cyano-2,3-dihydro-1,4-benzodioxan-5-yl)isoquinolin-6-yl]thio}-2-methylpropionic acid (Compound 11)

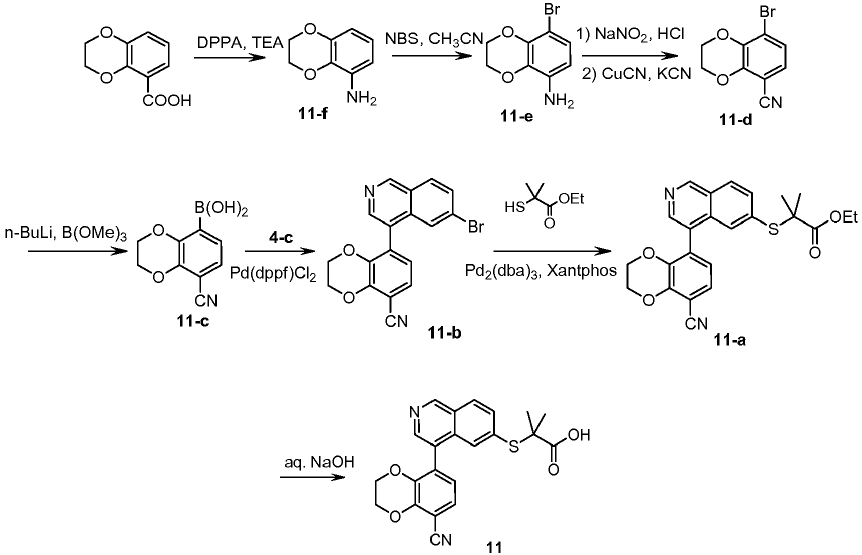

### Synthesis of compound 11-f

At room temperature, diphenyl phosphoryl azide (8.02g, 29mmol) and triethyl amine (4.2g, 42mmol) were added to a solution of 2,3-dihydro-1,4-benzodioxane-5-carboxylic acid (5.0g, 28mmol) in anhydrous THF (110mL). The mixture was stirred for 2hrs, followed by adding water (30mL), heating to 70°C and further reacting for 3hrs, then cooling to room temperature, being extracted with EA (100mL×3). The organic phases were combined, dried over anhydrous sodium sulfate, filtered, and concentrated under reduced pressure. The residue was purified by silica column chromatography (PE:EA = 5:1) to give compound **11-f** (1.58g, yield 37%). LC-MS (ESI): m/z= 152 [M+H]⁺.

### Synthesis of compound 11-e

At 0°C, a solution of N-bromosuccinimide (1.73g, 9.73mmol) in acetonitrile (5mL) was added to a solution of compound **11-f** (1.4g, 9.27mmol) in acetonitrile (35mL). The mixture was warmed to room temperature and reacted for 3hrs, evaporated under reduced pressure to remove the solvent. The residue was purified by silica column chromatography (PE:EA = 10:1 to 5:1) to give compound **11-e** (1.63g, yield 77%). LC-MS (ESI): m/z = 230 [M+H]⁺.

### Synthesis of compound 11-d

At 0°C, sodium nitrite (0.5g, 7.2mmol) was slowly added to a suspension of compound **11-e** (1.5g, 6.55mmol) in 3M HCl aqueous solution (12mL), reacted for 30mins, sodium bicarbonate solid was added to adjust the reaction mixture to pH=7. The mixture was heated to 60°C, a solution of CuCN (0.7g, 7.86mmol) and KCN (1.06g, 16.37mmol) in water (20mL) was added dropwise, and further stirred for 30mins. The reaction solution was cooled to room temperature, extracted with DCM (60mL×3). The organic phases were combined, dried over anhydrous sodium sulfate, filtered, and concentrated under reduced pressure. The residue was purified by silica column chromatography (PE: EA = 10:1) to give compound **11-d** (1.2g, yield 77%). LC-MS (ESI): m/z = 240 [M+H]⁺.

### Synthesis of compound 11-c

At -78°C, a solution of 2.5M *n*-butyl lithium in *n*-hexane (1.7mL, 4.2mmol) was added dropwise to a solution of compound **11-d** (910mg, 3.8mmol) in anhydrous THF (20mL), the mixture was stirred for 1h, followed by adding trimethyl borate (594mg, 5.7mmol) to the reaction solution. The reaction solution was slowly warmed to room temperature, further stirred for 16hrs, saturated NaCl aq. solution (20mL) was added. Organic phase was seperated, the aqueous phase was extracted with EA (60mL×3). The combined organic phases were dried over anhydrous sodium sulfate, filtered, concentrated under reduced pressure to give yellow solid **11-c** (700mg, yield 90%). The product was directly used for the next step without further purification. LC-MS (ESI): m/z = 206 [M+H]⁺.

### Synthesis of compound 11-b

Under N₂ atmosphere, compound **11-c** (130mg, 0.63mmol), compound **4-c** (200mg, 0.6mmol) and cesium carbonate (390mg, 1.2mmol) were suspended to a mixture of dioxane (10mL) and water (1mL), [1,1'-bis(diphenylphosphine)ferrocene]palladium dichloride (43mg, 0.06mmol) was added. The mixture was reacted for 10hrs at 80°C, cooled to room temperature, filtered through celite, the filtrate cake was washed with EA (20mL), the filtrate was concentrated under reduced pressure. The residue was purified by silica preparative plate chromatography (PE:EA = 3:1) to give compound **11-b** (146mg, yield 66%). LC-MS (ESI): m/z = 367 [M+H]⁺.

### Synthesis of compound 11-a

Under N₂ atmosphere, tris(dibenzylidene indene acetone)dipalladium (36mg, 0.04mmol) and 4,5- bis(diphenylphosphine)9,9-dimethyloxacanthracene (46mg, 0.08mmol) were added to a solution of compound **11-b** (146mg, 0.52mmol), ethyl 2-methyl-2-mercaptopropionate (77mg, 0.52mmol) and diisopropylethylamine (103mg, 0.8mmol) in dioxane (8mL). The mixture was stirred for 6hrs at 100°C, cooled to room temperature, concentrated under reduced pressure to remove dioxane. The residue was purified by silica preparative plate chromatography (PE:EA = 1:2) to give compound **11-a** (147mg, yield 85%). LC-MS (ESI): m/z = 435 [M+H]⁺.

### Synthesis of compound 11

At room temperature, 1M NaOH aq. solution (2.5mL) was added to a solution of compound **11-a** (146mg, 0.34mmol) in methanol (5mL), the mixture was stirred for 5hrs. The mixture was adjusted to pH=6 with 1M HCl aq. solution, concentrated under reduced pressure to remove methanol, solid was precipitated and filtered out. The solid was washed with water (5mL), dried under vacuum to give white solid **11** (95 mg, yield 69%). LC-MS (ESI): m/z = 407 [M+H]⁺.
¹H-NMR (400MHz, DMSO-d6) δ: 12.79 (s, 1H), 9.36 (s, 1H), 8.56 (s, 1H), 8.42 (s, 1H), 8.17 (d, J=8.0 Hz, 1H), 7.63 (m, 2H), 7.46 (d, J=8.0 Hz, 1H), 7.04 (d, J=8.0 Hz, 1H), 4.47 (m, 2H), 4.23 (m, 2H), 1.47 (s, 3H), 1.44 (s, 3H) ppm.

### Embodiment 12

### 2-{[4-(4-Cyano-7-fluoronaphthalen-1-yl)isoquinolin-6-yl]thio[-2-methyl propionic acid (Compound 12)

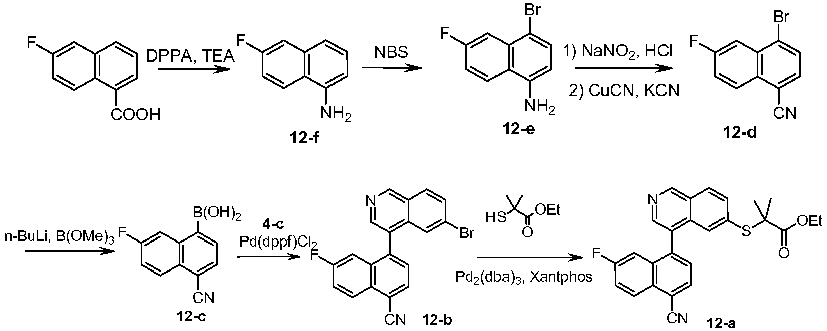

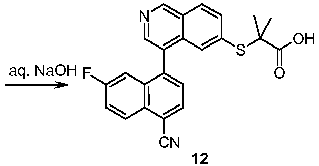

### Synthesis of compound 12-f

At room temperature, diphenyl phosphoryl azide (7.6g, 27.6mmol) and triethyl amine (4.0g, 54mmol) were added to a solution of 6-fluoronaphthalene-1-carboxylic acid (5.0g, 26.3mmol) in anhydrous THF (60mL). The mixture was stirred for 2hrs, followed by adding water (30mL), heating to 70°C and further stirring for 3hrs. The reaction solution was cooled to room temperature, extracted with EA (150mL×3). The organic phases were combined, dried over anhydrous sodium sulfate, filtered, and concentrated under reduced pressure. The residue was purified by silica column chromatography (PE:EA=15:1) to give compound **12-f** (1.0g, yield 16%). LC-MS (ESI): m/z = 162 [M+H]⁺.

### Synthesis of compound 12-e

At 0°C, a solution of N-bromosuccinimide (1.55g, 8.7mmol) in DCM (5mL) was added dropwise to a solution of compound **12-f** (1.4g, 8.7mmol) in DCM (50mL). The reaction solution was stirred for 30mins, concentrated under reduced pressure to remove the solvent. The residue was purified by silica column chromatography (PE:EA = 15:1) to give compound **12-e** (1.45g, yield 70%). LC-MS (ESI): m/z = 240 [M+H]⁺.

### Synthesis of compound 12-d

At 0°C, sodium nitrite (0.5g, 7.2mmol) was slowly added to a suspension of compound **12-e** (800mg, 3.3mmol) in 3M HCl aqueous solution (12mL), the mixture was reacted for 30mins, sodium bicarbonate solid was added to adjust the reaction solution to pH=7. At 60°C, the mixture was added to a solution of CuCN (357mg, 4.0mmol) and KCN (536mg, 8.25mmol) in water (20mL), the mixture was further reacted for 30mins. The reaction solution was cooled to room temperature, extracted with DCM (60mL×3). The organic phases were combined, dried over anhydrous sodium sulfate, filtered, and concentrated under reduced pressure. The residue was purified by silica column chromatography (PE:EA = 5:1) to give compound **12-d** (420mg, yield 50%). LC-MS (ESI): m/z = 250 [M+H]⁺.

### Synthesis of compound 12-c

At -78 °C, a solution of 2.5M *n*-butyl lithium in *n*-hexane (0.5mL, 1.17mmol) was added dropwise to a solution of compound **12-d** (226mg, 0.9mmol) in anhydrous THF (10mL). The mixture was stirred for 1h, followed by adding trimethyl borate (142mg, 1.36mmol) dropwise, then slowly warming to room temperature, and further stirring for 16hrs, 1M HCl aqueous solution (5mL) was added. The organic phase was seperated, the aqueous phase was extracted with EA (30mL×3). The organic phases were combined, dried over anhydrous sodium sulfate, filtered, and concentrated under reduced pressure to give yellow solid **12-c** (200mg, yield 100%). The product was directly used for the next step without further purification. LC-MS (ESI): m/z = 216 [M+H]⁺.

### Synthesis of compound 12-b

Under N₂ atmosphere, compound **12-c** (120mg, 0.93mmol), compound **4-c** (223mg, 1.11mmol) and cesium carbonate (363mg, 1.11mmol) were suspended in a mixture of dioxane (8mL) and water (0.8mL), [1,1'-bis(diphenylphosphine)ferrocene]palladium dichloride (41mg, 0.056mmol) was added. The mixture was reacted for 5hrs at 80°C, cooled to room temperature, filtered through celite, the filtrate cake was washed with EA (20mL). The filtrate was concentrated under reduced pressure, the residue was purified by silica preparative plate chromatography (PE:EA = 1:1) to give compound **12-b** (90mg, yield 43%). LC-MS (ESI): m/z = 377 [M+H]⁺.

### Synthesis of compound 12-a

Under N₂ atmosphere, tris(dibenzylidene indene acetone)dipalladium (22mg, 0.02mmol) and 4,5-bis(diphenylphosphine)-9,9-dimethyloxacanthracene (28mg, 0.05mmol) were added to a solution of compound **12-b** (90mg, 0.24mmol), ethyl 2-methyl-2-mercaptopropionate (46mg, 0.3mmol) and diisopropylethylamine (62mg, 0.48mmol) in dioxane (8mL). The mixture was stirred for 6hrs at 100°C, cooled to room temperature, concentrated under reduced pressure to remove dioxane. The residue was purified by silica preparative plate chromatography (PE:EA = 1:1) to give compound **12-a** (100mg, yield 94%). LC-MS (ESI): m/z = 445 [M+H]⁺.

### Synthesis of compound 12

At room temperature, 1M NaOH aq. solution (2.5mL) was added to a solution of compound **12-a** (100mg, 0.22mmol) in methanol (5mL), the mixture was stirred for 10hrs. The mixture was adjusted to pH=6 with 1M HCl aq. solution, concentrated under reduced pressure to remove methanol, solid was precipitated and filtered out. The solid was washed with water (5mL), dried under vacuum to give white solid **12** (70 mg, yield 75%). LC-MS (ESI): m/z = 417 [M+H]⁺.
¹H-NMR (400MHz, DMSO-d6) δ: 12.69 (s, 1H), 9.51 (s, 1H), 8.68 (s, 1H), 8.47 (d, J=8.5 Hz, 1H), 8.29 (m, 2H), 8.12 (m, 1H), 7.83 (m, 2H), 7.73 (d, J=8.5 Hz, 1H), 7.61 (s, 1H), 1.41 (s, 3H), 1.40 (s, 3H) ppm.

### Embodiment 13

### 3-[4-(4-Cyanophenyl)isoquinolin-6-yl]butyric acid (Compound 13)

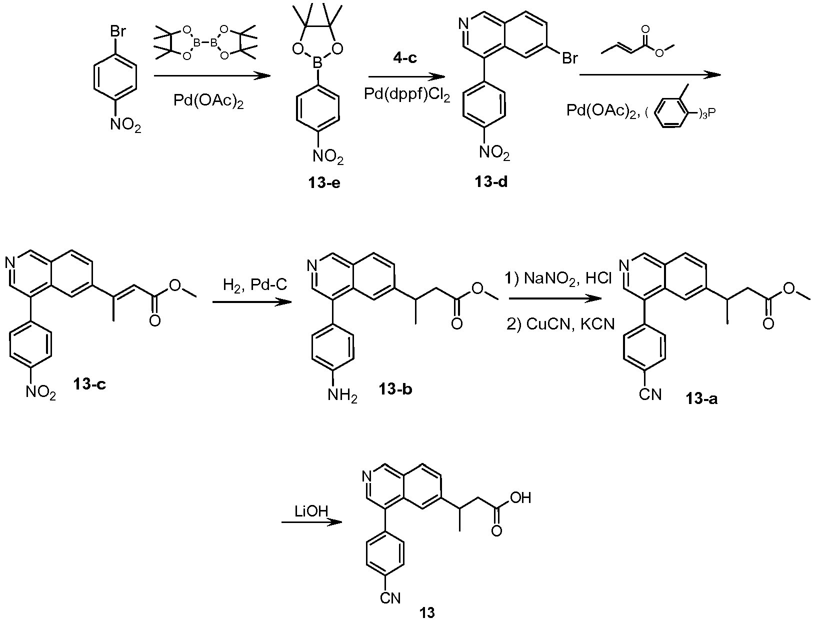

### Synthesis of compound 13-e

Under N₂ atmosphere, bis(pinacolato)diboron (4.53g, 17.82mmol), potassium acetate (4.37g, 44.55mmol) and palladium acetate (0.17g, 0.74mmol) were added respectively to a solution of 1-bromo-4-nitrobenzene (3.0g, 14.85mmol) in DMF (10mL). The mixture was stirred at 80°C for 2hrs, followed by adding water (20mL) and EA (20mL), the organic phase was in turn washed with water (10mL×3) and saturated brine (10mL), dried over anhydrous magnesium sulfate, filtered, concentrated under reduced pressure. The residue was purified by silica column chromatography (PE:EA = 10:1) to give compound **13-e** (2g, yield 54%). LC-MS (ESI): m/z = 250 [M+H]⁺.

### Synthesis of compound 13-d

Under N₂ atmosphere, compound **4-c** (1.0g, 3mmol), compound **13-e** (0.82g, 3.3mmol) and sodium carbonate (0.95g, 8.98mmol) were suspended in DMF (10mL) and water (5mL), [1,1'-bis(diphenylphosphine)ferrocene]palladium dichloride (0.245g, 0.3mmol) was added. The mixture was stirred at 80°C for 2hrs, cooled to room temperature, followed by adding water (15mL), being extracted with EA (30mL×3). The organic phase was in turn washed with water (20mL×3) and saturated brine (20mL), dried over anhydrous magnesium sulfate, filtered, concentrated under reduced pressure. The residue was purified by silica column chromatography (PE:EA = 2:1) to give compound **13-d** (0.9g, yield 90%). LC-MS (ESI): m/z = 329 [M+H]⁺.

### Synthesis of compound 13-c

Under N₂ atmosphere, methyl crotonate (0.29mL, 2.7mmol), palladium acetate (41mg, 0.18mmol), tri-o-methylphenylphosphine (111mg, 0.36mmol) and triethyl amine (0.5mL, 3.6mmol) were added to a solution of compound **13-d** (600mg, 1.8mmol) in DMF (5mL). The mixture was stirred for 16hrs at 80°C, cooled to room temperature, followed by adding water (15mL) and being extracted with EA (30mL×3). The organic phase was washed with water (20mL×3) and saturated brine (20mL), dried over anhydrous magnesium sulfate, filtered, concentrated under reduced pressure. The residue was purified by silica column chromatography (PE:EA = 1:1) to give compound **13-c** (360mg, yield 57%). LC-MS (ESI): m/z = 349 [M+H]⁺.

### Synthesis of compound 13-b

Under H₂ atmosphere (1 atm.), Pd-C (100mg) was added to a solution of compound **13-c** (180mg, 0.51mmol) in ethanol (20mL). The mixture was stirred for 16hrs at room temperature, filtered, concentrated under reduced pressure. The residue was purified by silica chromatography (PE:EA = 1:1) to give compound **13-b** (360 mg, yield 57%). LC-MS (ESI): m/z = 321 [M+H]⁺.

### Synthesis of compound 13-a

At 0°C, sodium nitrite (14.2mg, 0.2mmol) was slowly added to a suspension of compound **13-b** (60mg, 0.18mmol) in concentrated HCl aqueous solution (1mL), the mixture was stirred for 30mins, sodium bicarbonate solid was added to adjust the reaction solution to pH=7. The mixture was heated to 60°C, then was added to a solution of CuCN (20.1mg, 0.22mmol) and KCN (30.5mg, 0.46mmol) in water (3mL), further reacted for 30mins. The reaction solution was cooled to room temperature, extracted with DCM (20mL×3). The organic phases were combined, dried over anhydrous sodium sulfate, filtered, and concentrated under reduced pressure. The residue was purified by silica column chromatography (PE:EA = 5:1) to give compound **13-a** (30mg, yield 48%). LC-MS (ESI): m/z = 331 [M+H]⁺.

### Synthesis of compound 13

At room temperature, LiOH (50mg, 2mmol) was added to a mixed solution of compound **13-a** (30mg, 0.09mmol) in methanol (2mL), THF (2mL) and water (4mL). The mixture was stirred for 2hrs, followed by being adjusted to pH=7 with 2M HCl aq. solution and then extracted with EA (10 mL×3). The organic phases were combined, washed in turn with water (10mL×3) and saturated brine (10mL), dried over anhydrous magnesium sulfate, filtered, concentrated under reduced pressure to give compound **13** (10mg, yield 35%). LC-MS (ESI): m/z = 317 [M+H]⁺.
¹H-NMR (400MHz, DMSO-d6) δ: 12.10 (s, 1H), 9.34 (s, 1H), 8.44 (s, 1H), 8.20 (d, J=8.8 Hz, 1H), 8.05 (d, J=8.0Hz, 2H), 7.77 (d, J=8.0 Hz, 2H), 7.74 (d, J=8.8 Hz, 2H), 7.62 (s, 1H), 3.30 (m, 1H), 2.57 (d, J=7.6 Hz, 2H), 1.26 (d, J=8.8 Hz, 3H) ppm.

### Embodiment 14

### (2E)-3-[4-(4-Cyanonaphthalen-1-yl)isoquinolin-6-yl]acrylic acid (Compound 14)

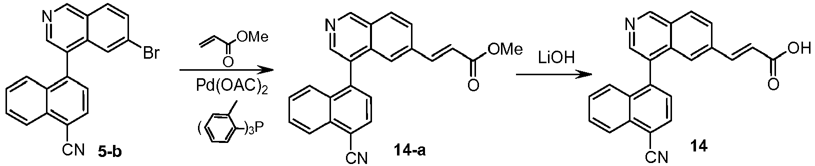

### Synthesis of compound 14-a

Under N₂ atmosphere, methyl acrylate (0.189mL, 2.09mmol), palladium acetate (31.2mg, 0.14mmol), tri-o-methylphenylphosphine (85mg, 0.27mmol) and triethyl amine (0.39mL, 2.78mmol) were added to a solution of compound **5-b** (500mg, 1.39mmol) in DMF (5mL). The mixture was stirred at 80°C for 16hrs, cooled to room temperature, followed by adding water (15mL), being extracted with EA (30mL×3). The organic phases were combined, washed in turn with water (20mL×3) and saturated brine (20mL), dried over anhydrous magnesium sulfate, filtered, and concentrated under reduced pressure. The residue was purified with silica column chromatography (PE:EA = 1:1) to give compound **14-a** (400mg, yield 79%). LC-MS (ESI): m/z = 379 [M+H]⁺.

### Synthesis of compound 14

At room temperature, LiOH (52.6mg, 2.19mmol) was added to a mixed solution of compound **14-a** (80mg, 0.22mmol) in methanol (2mL), THF (2mL) and water (4mL). The mixture was stirred for 2hrs, adjusted to pH=7 with 2M HCl aq. solution, extracted with EA (20mL×3). The organic phases were combined, washed in turn with water (10mL×3) and saturated brine (10mL), dried over anhydrous magnesium sulfate, filtered, concentrated under reduced pressure to give compound **14** (68mg, yield 88%). LC-MS (ESI): m/z = 351 [M+H]⁺.
¹H-NMR (400MHz, DMSO-d6) δ: 12.60 (s, 1H), 9.51 (s, 1H), 8.55 (s, 1H), 8.24 (m, 3H), 8.13 (d, J=8.4 Hz, 1H), 7.85 (t, J=7.6 Hz, 1H), 7.75 (d, J=7.6 Hz, 1H), 7.51 (m, 4H), 6.61 (d, J=15.6 Hz, 1H) ppm.

### Embodiment 15

### 3-[4-(4-Cyanonaphthalen-1-yl)isoquinolin-6-yl]butyric acid (Compound 15)

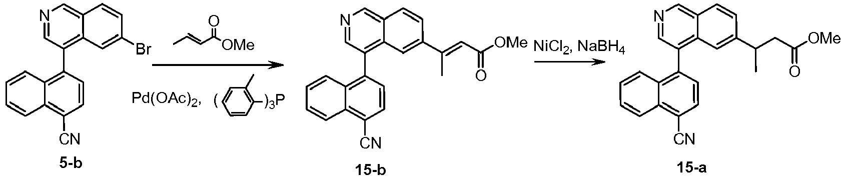

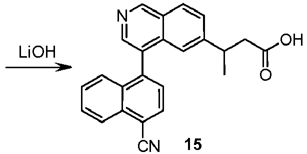

### Synthesis of compound 15-b

Under N₂ atmosphere, methyl crotonate (72mg, 0.56mmol), palladium acetate (12.5mg, 0.05mmol), tri-o-methylphenylphosphine (34mg, 0.11mmol) and triethyl amine (0.15mL, 1.1 1mmol) were added to a solution of compound **5-b** (200mg, 0.56mmol) in DMF (5mL). The mixture was stirred at 80°C for 16hrs, cooled to room temperature, followed by adding water (10mL), being extracted with EA (20mL×3). The organic phases were combined, washed in turn with water (10mL×3) and saturated brine (10mL), dried over anhydrous magnesium sulfate, filtered, and concentrated under reduced pressure. The residue was purified by silica column chromatography (PE:EA = 1:1) to give compound **15-b** (120mg, yield 59%). LC-MS (ESI): m/z = 379 [M+H]⁺.

### Synthesis of compound 15-a

At 0°C, NaBH₄ (120mg, 3.17mmol) was slowly added to a solution of compound **15-b** (120mg, 0.31mmol) and NiCl₂ (102mg, 0.79mmol) in methanol (150mL). The mixture was stirred at 0°C for 4.5hrs, warmed to room temperature, and concentrated under reduced pressure, followed by adding water (20mL) to the residue, being extracted with EA (50mL×3). The organic phases were combined, washed in turn with water (50mL×3) and saturated brine (50mL), dried over anhydrous sodium sulfate, filtered, and concentrated under reduced pressure. The residue was purified with silica column chromatography (PE:EA = 2:1) to give compound **15-a** (45mg, yield 37%). LC-MS (ESI): m/z =381 [M+H]⁺.

### Synthesis of compound 15

At room temperature, LiOH (28.3mg, 1.18mmol) was added to a mixed solution of compound **15-a** (45mg, 0.11mmol) in methanol (2mL), THF (2mL) and water (4mL). The mixture was stirred for 2hrs, and then adjusted to pH=7 with 2M HCl aq. solution, extracted with EA (20mL×3). The organic phases were combined, washed in turn with water (10mL×3) and saturated brine (10mL), dried over anhydrous magnesium sulfate, filtered, concentrated under reduced pressure to give compound **15** (18mg, yield 42%). LC-MS (ESI): m/z = 367 [M+H]⁺.
¹H-NMR (400MHz, CD₃OD) δ: 9.25 (s, 1H), 8.31 (s, 1H), 8.25 (d, J=8.4 Hz, 1H), 8.12 (d, J=4.4 Hz, 1H), 8.09 (d, J=7.2 Hz, 1H), 7.70 (t, J=7.2 Hz, 1H), 7.59 (m, 2H), 7.44 (m, 1H), 7.35 (t, J=8.8 Hz, 1H), 7.04 (d, J=10 Hz, 1H), 3.12 (m, 1H), 2.36 (m, 1H), 1.07 (m,3H) ppm.

### Embodiment 16

### 3-[4-(4-Cyanonaphthalen-1-yl)isoquinolin-6-yl]-2-methyl propionic acid (Compound 16)

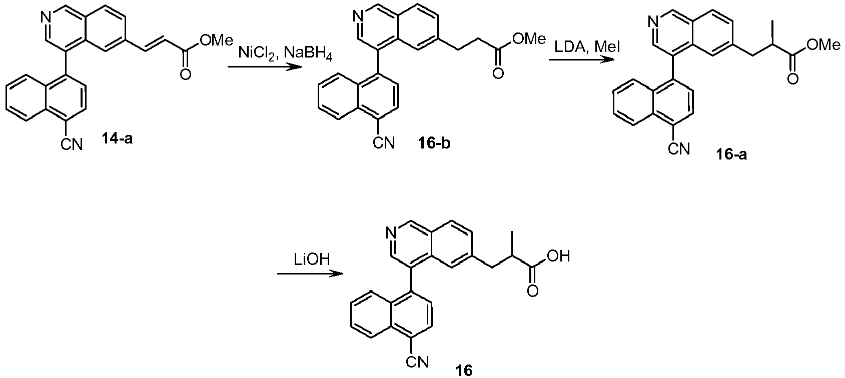

### Synthesis of compound 16-b

At 0°C, NaBH₄ (145mg, 3.84mmol) was slowly added to a solution of compound **14-a** (350mg, 0.96mmol) and NiCl₂ (62.2mg, 0.48mmol) in methanol (150mL). The mixture was stirred at 0°C for 4.5hrs, warmed to room temperature, and concentrated under reduced pressure, followed by adding water (20mL) to the residue, being extracted with EA (50mL×3). The organic phases were combined, washed in turn with water (50mL×3) and saturated brine (50mL), dried over anhydrous sodium sulfate, filtered, and concentrated under reduced pressure. The residue was purified with silica column chromatography (PE:EA = 2:1) to give compound **16-b** (200mg, yield 57%). LC-MS (ESI): m/z =367 [M+H]⁺.

### Synthesis of compound 16-a

Under N₂ atmosphere, at -78°C, a solution of 2.5M *n*-butyl lithium in n-hexane (0.87mL, 2.18mmol) was added dropwise in a solution of diisopropylamine (0.11mL, 2.18mmol) in anhydrous THF (10mL). The mixture was stirred for 30mins, a solution of compound **16-b** (200mg, 0.55mmol) in anhydrous THF (5mL) was added dropwise, and the mixture was further stirred for 30mins, a solution of CH₃I (139.5mg, 0.98mmol) in anhydrous THF (5mL) was added dropwise, the mixture was slowly warmed to room temperature and further stirred for 2hrs, saturated NH₄Cl aq. solution (10mL) was added, the mixture was extracted with EA (20mL×3). The organic phases were combined, dried over anhydrous sodium sulfate, filtered, and concentrated under reduced pressure. The residue was purified by silica column chromatography (PE:EA = 2:1) to give compound **16-a** (20mg, yield 19%). LC-MS (ESI): m/z =381 [M+H]⁺.

### Synthesis of compound 16

At room temperature, LiOH (25mg, 1.05mmol) was added to a mixed solution of compound **16-a** (20mg, 0.05mmol) in methanol (2mL), THF (2mL) and water (4mL). The mixture was stirred for 2hrs, adjusted to pH=7 with 2M HCl aq. solution, extracted with EA (20mL×3). The organic phases were combined, washed in turn with water (10mL×3) and saturated brine (10mL), dried over anhydrous magnesium sulfate, filtered, concentrated under reduced pressure to give compound **16** (10mg, yield 52%). LC-MS (ESI): m/z = 367 [M+H]⁺.
¹H-NMR (400MHz, CD₃OD) δ: 9.37 (s, 1H), 8.42 (d, J=2.4 Hz, 1H), 8.36 (d, J=8.4 Hz, 1H), 8.21 (m, 2H), 7.81 (t, J=7.6 Hz, 1H), 7.69 (d, J=7.6 Hz, 1H), 7.66 (d, J=8.4 Hz, 1H), 7.50 (m, 2H), 7.13 (d, J=7.6 Hz, 1H), 2.97 (m, 1H), 2.65 (m, 2H), 1.02 (m,3H) ppm.

### Embodiment 17

### 3-[4-(4-Cyanonaphthalen-1-yl)isoquinolin-6-yl]-2,2-dimethyl propionic acid (Compound 17)

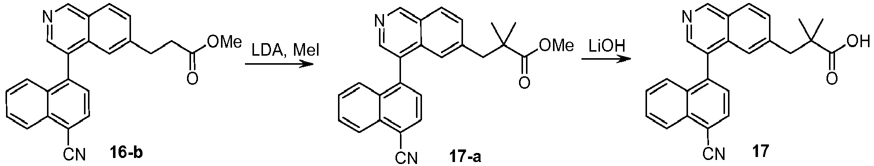

### Synthesis of compound 17-a

Under N₂ atmosphere, at -78°C, a solution of 2.5M *n*-butyl lithium in *n*-hexane (0.87mL, 2.18mmol) was slowly added to a solution of diisopropylamine (0.11mL, 2.18mmol) in anhydrous THF (10mL) dropwise. The mixture was stirred for 30mins, followed by adding a solution of compound **16-b** (200mg, 0.55mmol) in anhydrous THF (5mL) dropwise, further stirred for 30mins, followed by adding a solution of CH₃I (139.5mg, 0.98mmol) in anhydrous THF (5mL). The mixture was slowly warmed to room temperature, further stirred for 2hrs, followed by adding saturated NH₄Cl aq. solution (10mL) and being extracted with EA (20mL×3). The organic phases were combined, dried over anhydrous sodium sulfate, filtered, and concentrated under reduced pressure. The residue was purified by silica column chromatography (PE:EA = 2:1) to give compound **17-a** (20mg, yield 18.5%). LC-MS (ESI): m/z =395 [M+H]⁺.

### Synthesis of compound 17

At room temperature, LiOH (24mg, 1.01mmol) was added to a mixed solution of compound **17-a** (20mg, 0.05mmol) in methanol (2mL), THF (2mL) and water (4mL). The mixture was stirred for 2hrs, followed by adding 2M HCl aq. solution to adjust pH=7, and then extracted with EA (20mL×3). The organic phases were combined, washed in turn with water (10mL×3) and saturated brine (10mL), dried over magnesium sulfate, filtered, concentrated under reduced pressure to give compound **17** (5mg, yield 26%). LC-MS (ESI): m/z = 381 [M+H]⁺.
¹H-NMR (400MHz, CD₃OD) δ: 9.37 (s, 1H), 8.42 (s, 1H), 8.37 (d, J=8.8 Hz, 1H), 8.21 (d, J=7.6 Hz, 1H), 7.81 (m, 1H), 7.55 (m, 5H), 7.11 (s, 1H), 2.97 (m, 1H), 2.89 (m, 2H), 1.02 (s, 3H), 0.97 (s, 3H) ppm.

### Embodiment 18

### 2-{[8-(4-Cyanophenyl)isoqumolm-2-yl]thio}-2-methyl propionic acid (Compound 18)

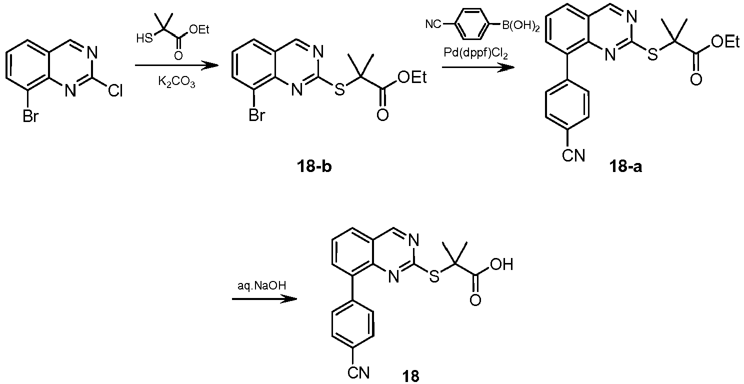

### Synthesis of compound 18-b

8-Bromo-2-chloroquinazoline (110mg, 0.45mmol) was added to a suspension of ethyl 2-methyl-2-mercaptopropionate (80mg, 0.54mmol), potassium carbonate (124mg, 0.9mmol) in DMF (3mL). The mixture was stirred for 3hrs at 130°C, cooled to room temperature, followed by adding water (20mL), being extracted with EA (30mL×3). The organic phases were combined, washed in turn with water (20mL×3) and saturated brine (20mL), dried over anhydrous sodium sulfate, filtered, and concentrated under reduced pressure. The residue was purified by silica column chromatography (PE:EA = 6:1) to give colorless oil **18-b** (108mg, yield 67.5%). LC-MS (ESI): m/z =355 [M+H]⁺.

### Synthesis of compound 18-a

Under N₂ atmosphere, compound **18-b** (108mg, 0.3mmol), 4-cyanophenyl boronic acid (54mg, 0.36mmol) and cesium carbonate (196mg, 0.6mmol) were suspended in dioxane (10mL) and water (1mL), [1,1'-bis(diphenylphosphine)ferrocene]palladium dichloride (40mg, 0.05mmol) was added. The mixture was stirred at 90°C for 16hrs, followed by cooling to room temperature, being concentrated under reduced pressure. The residue was purified by silica column chromatography (PE:EA = 5:1) to give colorless oil **18-a** (83mg, yield 72%). LC-MS (ESI): m/z = 378 [M+H]⁺.

### Synthesis of compound 18

At room temperature, 1M NaOH aq. solution (2.0mL) was added to a solution of compound **18-a** (83mg, 0.22mmol) in methanol (5mL). The mixture was stirred for 2hrs, followed by adding 1M HCl aq. solution to adjust pH=5-6, being extracted with EA (20mL×3). The organic phases were combined, dried over anhydrous sodium sulfate, filtered, and concentrated under reduced pressure. The residue was purified by HPLC (mobile phase: 10mM NH₄HCO₃ aq. solution: acetonitrile = 25%-55%) to give yellow solid **18** (7mg, yield 9%). LC-MS (ESI): m/z = 350 [M+H]⁺.
¹H-NMR (400MHz, CD₃OD) δ: 9.31 (s, 1H), 8.07 (dd, J=8.0 Hz, 1.6 Hz, 1H), 7.98 (dd, J=7.6 Hz, 2.4 Hz, 1H), 7.84 (d, J=7.6 Hz, 2H), 7.77 (d, J=7.6 Hz, 2H), 7.71 (dd, J=7.8 Hz, 3.6 Hz, 1H), 1.68 (s, 6H) ppm.

### Embodiment 19

### {[4-(4-Cyanophenyl)isoquinolin-6-yl](methyl)carbamoyl}formic acid (Compound 19)

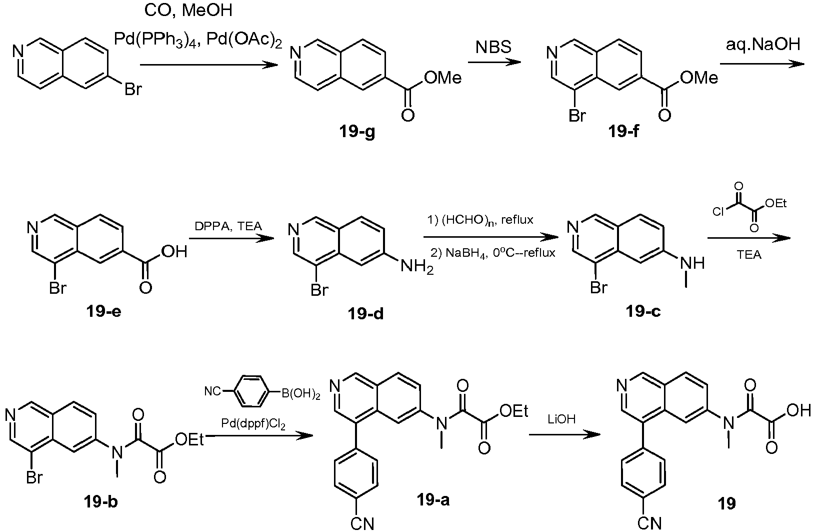

### Synthesis of compound 19-g

Under CO atmosphere (10 atm.), a mixture of 6-bromoisoquinoline (5.0g, 24mmol), sodium acetate (2.56g, 31mmol), tetrakis(triphenylphosphine)palladium (2.77g, 2.4mmol), palladium acetate (1.1g, 4.8mmol), DMF (50mL) and DCM (50mL) was heated to 100°C, stirred for 16hrs and then cooled to room temperature, concentrated under reduced pressure to remove methanol. Water (100mL) was added to the residue, EA (200mL×2) was used for extract. The organic phases were combined, washed in turn with water (100mL×3) and saturated brine (100mL), dried over anhydrous sodium sulfate, filtered, and concentrated under reduced pressure. The residue was purified by silica column chromatography (PE:EA = 5:1) to give white solid **19-g** (4.34g, yield 96%). LC-MS (ESI): m/z =188 [M+H]⁺.

### Synthesis of compound 19-f

N-Bromosuccinimide (6.19g, 34.8mmol), compound **19-g** (4.34g, 23.2mmol) and acetic acid (25mL) were added to 80°C and stirred for 16hrs, the mixture was cooled to room temperature, concentrated under reduced pressure. Saturated sodium bicarbonate solution was added to the residue (30mL), the mixture was extracted with EA (50mL×2). The organic phases were combined, dried over anhydrous sodium sulfate, filtered, and concentrated under reduced pressure. The residue was purified by silica chromatography (PE:EA = 5:1) to give compound **19-f** (3.59g, yield 58%). LC-MS (ESI): m/z = 268 [M+H]⁺.

### Synthesis of compound 19-e

At room temperature, 2M NaOH aq. solution (10mL) was added to a solution of compound **19-f** (3.59g, 13.5mmol) in methanol (30mL). The mixture was stirred for 16hrs, followed by adding 1M HCl aq. solution to adjust pH=5-6, solid was precipitated and filtered out. The solid was washed with water (10mL), dried under vacuum to give yellow solid **19-e** (3.29g, yield 96.8%). LC-MS (ESI): m/z = 254 [M+H]⁺.

### Synthesis of compound 19-d

At room temperature, diphenyl phosphoryl azide (5.2g, 25.2mmol) and triethyl amine (2.5g, 25.2mmol) were added to a solution of compound **19-e** (3.17g, 12.6mmol) in anhydrous THF (30mL). The mixture was stirred for 3hrs, followed by adding water (10mL), refluxing for 12hrs, then cooling to room temperature, being concentrated under reduced pressure. The residue was purified by silica column chromatography (PE:EA = 1:1) to give yellow solid **19-g** (0.2g, yield 7%). LC-MS (ESI): m/z = 225 [M+H]⁺.

### Synthesis of compound 19-c

Sodium methoxide (218mg, 4.03mmol) and polyformaldehyde (121mg, 4.03mmol) were added to a solution of compound **19-d** (180mg, 0.81mmol) in methanol (6mL). The mixture was refluxed for 1.5hrs, cooled to 0°C, NaBH₄ (185mg, 4.86mmol) was added in portions. The mixture was refluxed again for 1.5hrs, then cooled to room temperature, saturated NaHCO₃ (30mL) was added, the mixture was extracted with DCM (20mL×3). The organic phases were combined, dried over anhydrous sodium sulfate, filtered, concentrated under reduced pressure to give yellow solid **19-c** (175mg, yield 91.6%), the product was directly used for the next step without further purification. LC-MS (ESI): m/z = 237 [M+H]⁺.

### Synthesis of compound 19-b

At room temperature, ethyl oxalyl monochloride (151mg, 1.11mmol) was added to a solution of compound **19-c** (175mg, 0.74mmol) and triethyl amine (150mg, 1.48mmol) in DCM (10mL). The mixture was stirred for 1h, followed by adding water (10mL), being extracted with DCM (20mL×3). The organic phases were combined, dried over anhydrous sodium sulfate, filtered, concentrated under reduced pressure to give yellow oil **19-b** (249mg, yield 99%), the product was directly used for the next step without further purification. LC-MS (ESI): m/z = 339 [M+H]⁺.

### Synthesis of compound 19-a

Under N₂ atmosphere, compound **19-b** (249mg, 0.74mmol), 4-cyanophenylboronic acid (163mg, 1.1 1mmol) and sodium carbonate (157mg, 1.48mmol) were suspended in dioxane (15mL) and water (2mL), [1,1'-bis(diphenylphosphine)ferrocene]palladium dichloride (54mg, 0.07mmol) was added. The mixture was stirred at 90°C for 12hrs, followed by cooling to room temperature, being concentrated under reduced pressure. The residue was purified by silica column chromatography (PE:EA = 3:1-1:1) to give compound **19-a** (90 mg, yield 34%). LC-MS (ESI): m/z = 360 [M+H]⁺.

### Synthesis of compound 19

At room temperature, LiOH (2.0mL) was added to a solution of compound **19-a** (90mg, 0.25mmol) in methanol (5mL), THF (3mL) and water (1mL). The reaction solution was stirred for 2hrs, and then concentrated under reduced pressure. The residue was adjusted to pH=5-6 with 1M HCl aq. solution, then extracted with EA (20mL×3). The organic phases were combined, dried over anhydrous sodium sulfate, filtered, concentrated under reduce pressure. The residue underwent HPLC preparation (mobile phase: 10mM NH₄HCO₃ aq. solution: acetonitrile = 25%-55%) to give white solid **19** (5mg, yield 7%). LC-MS (ESI): m/z = 332 [M+H]⁺.
¹H-NMR (400MHz, CD₃OD) δ: 9.34 (s, 1H), 8.47 (s, 1H), 8.31 (d, J=8.6 Hz, 1H), 7.96 (d, J=8.6 Hz, 1H), 7.80 (m, 4H), 3.39 (s, 3H) ppm.

### Embodiment 20

### {[4-(4-Cyanonaphthalen-1-yl)isoquinolin-6-yl](methyl)carbamoyl} formic acid (Compound 20)

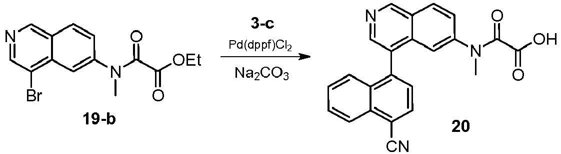

### Synthesis of compound 20

Under N₂ atmosphere, compound **19-b** (260mg, 0.77mmol), compound **3-c** (258mg, 0.93mmol) and sodium carbonate (163mg, 1.54mmol) were suspended in dioxane (8mL) and water (1mL), [1,1'-bis(diphenylphosphine)ferrocene]palladium dichloride (56mg, 0.07mmol) was added. The mixture was stirred at 90°C for 12hrs, followed by cooling to room temperature, being concentrated under reduced pressure. The residue underwent HPLC preparation (mobile phase: 10mM NH₄HCO₃ aq. solution: acetonitrile = 25%-45%) to give yellow solid **20** (40mg, yield 13.6%). LC-MS (ESI): m/z = 382 [M+H]⁺.
¹H-NMR (400MHz, CD₃OD) δ: 9.42 (s, 1H), 8.47 (s, 1H), 8.33 (d, J=8.7 Hz, 2H), 8.20 (d, J=7.3 Hz, 1H), 7.79 (dd, J=8.6 Hz, 4.8 Hz, 1H), 7.71 (d, J=7.3 Hz, 1H), 7.56 (m, 2H), 7.33 (d, J=1.4 Hz, 2H), 3.24 (s, 3H) ppm.

### Embodiment 21

### 2- {[4-(4-Cyanonaphthalen-1-yl)isoquinolin-6-yl]thio}propionic acid (Compound 21)

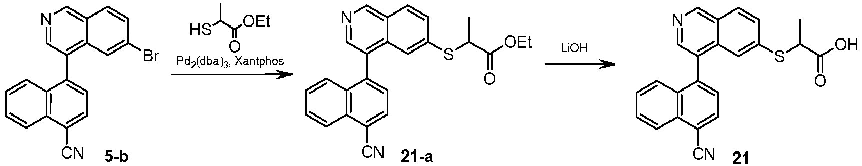

### Synthesis of compound 21-a

Under N₂ atmosphere, tris(dibenzylidene indene acetone)dipalladium (24mg, 0.05mmol) and 4,5-bis(diphenylphosphine)-9,9-dimethyloxacanthracene (30mg, 0.05mmol) were added to a solution of compound **5-b** (185mg, 0.51mmol), ethyl 2-mercaptopropionate (83mg, 0.61mmol) and diisopropylethylamine (133mg, 1.03mmol) in dioxane (6mL). The mixture was reacted in a microwave at 110°C for 1h, cooled to room temperature, and then concentrated under reduced pressure to remove dioxane. The residue was purified by silica column chromatography (PE:EA = 3:2) to give yellow solid **21-a** (163mg, yield 77%). LC-MS (ESI): m/z = 413 [M+H]⁺.

### Synthesis of compound 21

At room temperature, LiOH (12mg, 0.29mmol) was added to a solution of compound **21-a** (30mg, 0.07mmol) in methanol (1mL), THF (4mL) and water (1mL). The mixture was stirred for 1h, followed by adding 1M HCl aq. solution to adjust pH=5-6, the mixture was extracted with EA (15mL×2). The organic phases were combined, dried over anhydrous sodium sulfate, filtered, concentrated under reduced pressure to give white solid **21** (16mg, yield 57%). LC-MS (ESI): m/z = 385 [M+H]⁺.
¹H-NMR (400MHz, DMSO-d6) δ: 9.43 (s, 1H), 8.50 (s, 1H), 8.27 (m, 3H), 7.85 (s, 1H), 7.72 (dd, J=11.7 Hz, 7.5 Hz, 2H), 7.59 (d, J=7.2 Hz, 1H), 7.45 (d, J=9.0 Hz, 1H), 7.15 (d, J=10.1 Hz, 1H), 1.35 (s, 1H), 1.24 (m, 3H) ppm.

### Embodiment 22

### 2-{[4-(3-Chloro-4-cyanophenyl)isoquinolin-6-yl]thio}-2-methyl propionic acid (Compound 22)

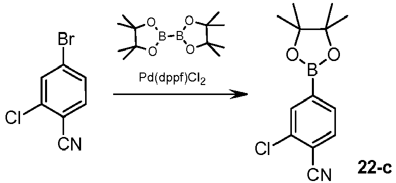

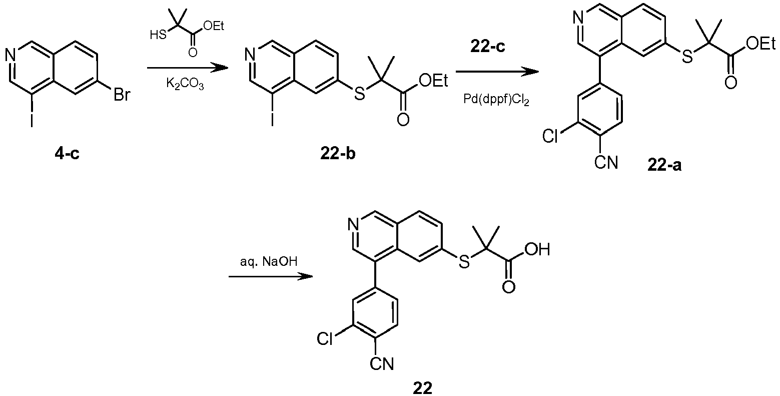

### Synthesis of compound 22-c

Under N₂ atmosphere, bis(pinacolato)diboron (391mg, 1.54mmol), potassium acetate (412mg, 4.2mmol) and [1,1'-bis(diphenylphosphine)ferrocene]palladium dichloride (102mg, 0.14mmol) were respectively added to a solution of 2-chloro-4-bromobenzonitrile (300mg, 1.4mmol) in dioxane (15mL). The mixture was stirred at 115 °C for 12hrs, cooled to room temperature, filtered through celite, washed with EA (50mL). The filtrate was evaporated under reduced pressure to give compound **22-c** (620mg, yield 100%). The product was directly used for the next step without further purification. LC-MS (ESI): m/z = 182 [M+H]⁺.

### Synthesis of compound 22-b

Compound **4-c** (80mg, 0.24mmol) was added to a suspension of ethyl 2-methyl-2-mercaptopropionate (71mg, 0.48mmol) and potassium carbonate (100mg, 0.72mmol) in DMF (2mL). The mixture was stirred at 130°C for 2hrs, cooled to room temperature, followed by adding water (20mL), being extracted with EA (20mL×3). The organic phases were combined, washed in turn with water (20mL×3) and saturated brine (20mL), dried over anhydrous sodium sulfate, filtered and concentrated under reduced pressure. The residue was purified with silica preparative plate (PE:EA = 3:1) to give yellow oil **22-b** (75mg, yield 78%). LC-MS (ESI): m/z = 402 [M+H]⁺.

### Synthesis of compound 22-a

Under N₂ atmosphere, compound **22-b** (60mg, 0.15mmol), compound **22-c** (120mg, 0.23mmol) and cesium carbonate (98mg, 0.3mmol) were suspended in dioxane (3mL) and water (0.3mL), [1,1'-bis(diphenylphosphine)ferrocene]palladium dichloride (11mg, 0.02mmol) was added. The mixture was stirred at 100°C for 12hrs, cooled to room temperature, followed by adding water (10mL), being extracted with EA (10mL×3). The organic phases were combined, washed in turn with water (10mL×3) and saturated brine (10mL), dried over anhydrous sodium sulfate, filtered, and concentrated under reduced pressure. The residue was purified with silica preparative plate (DCM: methanol = 20:1) to give brown solid **22-a** (43mg, yield 70%).

### Synthesis of compound 22

At room temperature, 1M NaOH aq. solution (1mL) was added to a solution of compound **22-a** (43mg, 0.1mmol) in methanol (1mL) and THF (1mL). The mixture was stirred for 4hrs, followed by evaporating under reduced pressure to remove methanol. The residue was adjusted to pH=5-6 with 1M HCl aq. solution, followed by being extracted with DCM (10mL×3). The organic phases were combined, dried over anhydrous sodium sulfate, filtered, and concentrated under reduced pressure. The residue was purified by silica preparative plate (DCM: methanol = 10:1) to give yellow solid **22** (21mg, yield 53%). LC-MS (ESI): m/z = 383 [M+H]⁺.
¹H-NMR (400MHz, CDCl₃) δ: 9.21 (s, 1H), 8.38 (s, 1H), 7.98 (d, J=8.4 Hz, 1H), 7.95 (s, 2H), 7.78 (d, J=8.8 Hz, 1H), 7.73 (d, J=8.0 Hz, 1H), 7.63 (s, 1H), 7.50 (d, J=8.0 Hz, 1H), 1.61 (s, 6H) ppm.

### Embodiment 23

### 2-{[4-(2-Chloro-4-cyanophenyl)isoquinolin-6-yl]thio}-2-methyl propionic acid (Compound 23)

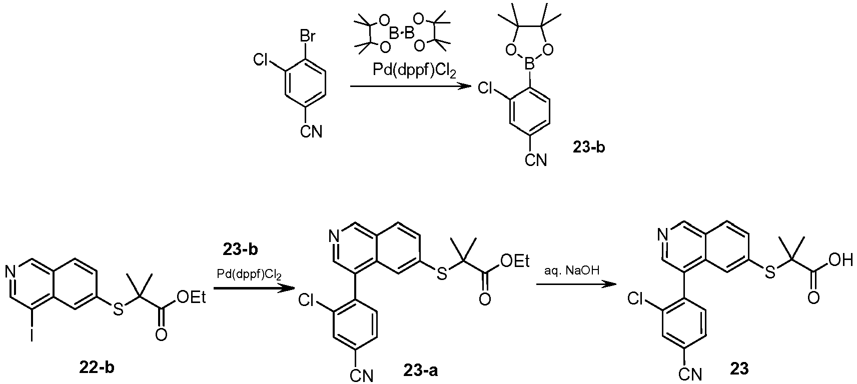

### Synthesis of compound 23-b

Under N₂ atmosphere, bis(pinacolato)diboron (391mg, 1.54mmol), potassium acetate (412mg, 4.5mmol) and [1,1'-bis(diphenylphosphine)ferrocene]palladium dichloride (102mg, 0.14mmol) were respectively added to a solution of 2-chloro-4-bromobenzonitrile (300mg, 1.4mmol) in dioxane (15mL). The mixture was stirred at 80 °C for 12hrs, cooled to room temperature, filtered through celite, washed with EA (50mL). The filtrate was evaporated under reduced pressure, and the residue was purified by silica preparative plate to give white solid **23-b** (73mg, yield 20%).
¹H-NMR (400MHz, CDCl₃) δ: 7.78 (d, J=7.6 Hz, 1H), 7.62 (d, J=1.2 Hz, 1H), 7.52 (dd, J=7.6 Hz, 1.2 Hz, 1H), 1.37 (s, 12H) ppm.

### Synthesis of compound 23-a

Under N₂ atmosphere, compound **22-b** (100mg, 0.15mmol), compound **23-b** (73mg, 0.27mmol) and cesium carbonate (163mg, 0.5mmol) were suspended in dioxane (3mL) and water (0.3mL), [1,1'-bis(diphenylphosphine)ferrocene]palladium dichloride (19mg, 0.03mmol) was added. The mixture was stirred at 100°C for 12hrs, cooled to room temperature, followed by adding water (10mL), being extracted with EA (10mL×3). The organic phases were combined, washed in turn with water (10mL×3) and saturated brine (10mL), dried over anhydrous sodium sulfate, filtered, and concentrated under reduced pressure. The residue was purified by silica preparative plate (DCM: methanol = 20:1) to give white solid **23-a** (72mg, yield 71%). LC-MS (ESI): m/z = 411 [M+H]⁺.

### Synthesis of compound 23

At room temperature, 1M NaOH aq. solution (1mL) was added to a solution of compound **23-a** (72mg, 0.18mmol) in methanol (1mL) and THF (1mL). The mixture was stirred for 2hrs, followed by evaporating under reduced pressure to remove methanol. The residue was adjusted to pH=5-6 with 1M HCl aq. solution, followed by being extracted with DCM (10mL×3). The organic phases were combined, dried over anhydrous sodium sulfate, filtered, and concentrated under reduced pressure. The residue was purified with silica preparative plate (DCM: methanol = 10:1) to give white solid **23** (31mg, yield 46%). LC-MS (ESI): m/z = 383 [M+H]⁺.
¹H-NMR (400MHz, DMSO-d6) δ: 9.42 (s, 1H), 8.44 (s, 1H), 8.31 (d, J=1.6 Hz, 1H), 8.21 (d, J=8.4 Hz, 1H), 8.03 (dd, J=8.0 Hz, 1.2 Hz, 1H), 7.73 (d, J=8.0 Hz, 1H), 7.39 (s, 1H), 1.42 (s, 3H), 1.41 (s, 3H) ppm.

### Embodiment 24

### 1-{[4-(4-cyanonaphthalen-1-yl)isoquinolin-6-yl]thio} cyclobutane-1 -carboxylic acid (Compound 24)

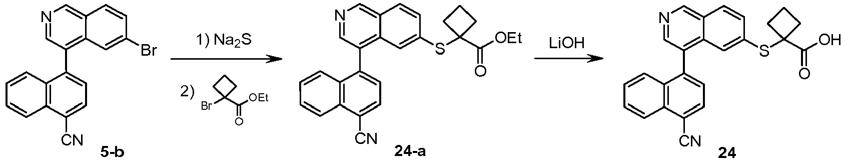

### Synthesis of compound 24-a

Na₂S•9H₂O (182mg, 0.75mmol) was added to a solution of compound **5-b** (180mg, 0.5mmol) in DMF (2mL). The mixture was reacted in a microwave at 130°C for 1h, cooled to room temperature, 1-bromo-cyclobutanoic acid ethyl ester (155mg, 0.75mmol) was added, the mixture was stirred at 50°C for 2hrs. The mixture was cooled to room temperature, followed by adding ice water (20mL), being extracted with EA (50mL). The organic phases were combined, dried over anhydrous sodium sulfate, filtered, and concentrated under reduced pressure. The residue was purified by silica preparative plate (PE:EA = 1:1) to give white solid **24-a** (89 mg, yield 40%). LC-MS (ESI): m/z = 439 [M+H]⁺.

### Synthesis of compound 24

At room temperature, LiOH•H₂O (26mg, 0.61mmol) was added to a mixed solution of compound **24-a** (89mg, 0.20mmol) in methanol (1mL), THF (1mL) and water (1mL). The mixture was stirred for 4hrs, concentrated under reduced pressure, followed by adding water (10mL) and EA (20mL). The aqueous phase was adjusted to pH=5-6 with 0.5M HCl aq. solution, solid turned out, the mixture was further stirred for 30mins and filtered. The solid was washed with water (10mL), dried under vacuum to give white solid **24** (65 mg, yield 78%). LC-MS (ESI): m/z = 411 [M+H]⁺.
¹H-NMR (400MHz, DMSO-d6) δ: 12.69 (s, 1H), 9.41 (s, 1H), 8.52 (s, 1H), 8.33 (d, J=8.0 Hz, 1H), 8.31 (d, J=8.0 Hz, 1H), 8.19 (d, J=8.0 Hz, 1H), 7.83 (m, 1H), 7.69 (d, J=8.0 Hz, 1H), 7.57 (m, 1H), 7.51 (d, J=8.0 Hz, 1H), 7.39 (d, J=8.0 Hz, 1H), 2.51 (m, 1H), 2.01 (m, 3H), 1.71 (m, 2H) ppm.

### Embodiment 25

### 3-{3-[(2, 6-dichlorophenyl)methyl]-1-methylimidazole[1,5-a]pyridine-6-yl}propionic acid (Compound 25)

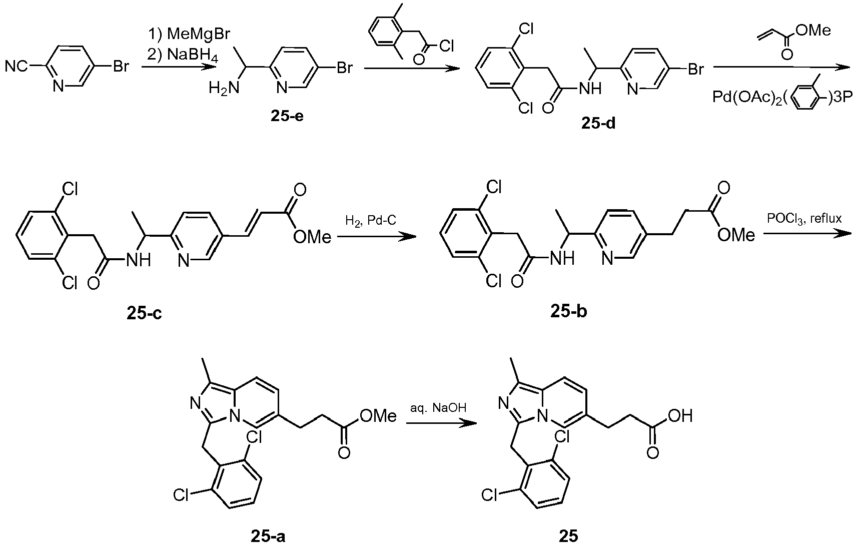

### Synthesis of compound 25-e

At 0°C, a solution of 3M methyl magnesium bromide in THF (2.09mL, 6.28mmol) was added into a solution of 5-bromo-2-cyanopyridine (1.0g, 5.46mmol) in anhydrous THF (10mL). The mixture was warmed to room temperature slowly, further stirred for 30mins, and followed by adding methanol (20mL), adding NaBH₄ (410mg, 10.93mmol) in portions. The mixture was further stirred for 10hrs, followed by adding water (10mL) and 2M NaOH aq. solution (10mL) in turn, being extracted with EA (50mL×3). The organic phases were combined, washed in turn with water (20mL×3) and saturated brine (20mL), dried over anhydrous sodium sulfate, filtered, and concentrated under reduced pressure. The residue was purified by silica preparative plate (PE:EA = 1:1) to give white solid **25-e** (1.0g, yield 91%). LC-MS (ESI): m/z = 201 [M+H]⁺.

### Synthesis of compound 25-d

At room temperature, oxalyl chloride (0.69g, 5.47mmol) and DMF (0.1mL) were added to a solution of 2,6-dichlorophenylacetic acid (1.02g, 4.97mmol) in DCM (10mL), the mixture was stirred for 2hrs and concentrated under reduced pressure. The residue was dissolved in DCM (10mL) again, at 0°C, the above solution was slowly added to a solution of compound **25-e** (1.0g, 4.97mmol) and triethyl amine (1.39mL, 9.95mmol) in DCM (10mL). The mixture was warmed to room temperature and further stirred for 2hrs, followed by adding water (20mL), and being extracted with DCM (50mL×3). The organic phases were combined, washed in turn with water (20mL×3) and saturated brine (20mL), dried over anhydrous sodium sulfate, filtered, and concentrated under reduced pressure. The residue was purified (PE:EA = 5:1) to give light yellow solid **25-d** (0.9g, yield 46%). LC-MS (ESI): m/z = 387 [M+H]⁺.

### Synthesis of compound 25-c

Under N₂ atmosphere, methyl acrylate (0.186mL, 2.06mmol), palladium acetate (23.1mg, 0.1mmol), tris(*o*-methylphenyl)phosphine (62.7mg, 0.2mmol) and triethyl amine (0.28mL, 2mmol) were added to a solution of compound **25-d** (400mg, 1.03mmol) in DMF (5mL). The mixture was reacted in a microwave at 120°C for 10mins, cooled to room temperature, followed by adding water (15mL), being extracted with EA (30mL×3). The organic phase was washed in turn with water (20mL×3) and saturated brine (20mL), dried over anhydrous magnesium sulfate, filtered, and concentrated under reduced pressure. The residue was purified by silica column chromatography (PE:EA = 1:1) to give compound **25-c** (400mg, yield 98%). LC-MS (ESI): m/z = 393 [M+H]⁺.

### Synthesis of compound 25-b

Under H₂ atmosphere (1 atm.), the Pd-C (50mg) was added to a solution of compound **25-c** (400mg, 1.02mmol) in ethanol (10mL). The mixture was stirred for 12hrs, filtered, and concentrated under reduced pressure to give compound **25-b** (350mg, yield 87%). The product was used directly for the next step without further purification. LC-MS (ESI): m/z = 393 [M+H]⁺.

### Synthesis of compound 25-a

Compound **25-b** (350mg, 0.89mmol) was dissolved in phosphorus oxychloride (8mL), stirred at 110°C for 5hrs. The mixture was cooled to room temperature, added to ice water (20mL), followed by adding sodium carbonate solid to adjust pH=8, and then extracted with EA (30mL×3). The organic phases were in turn washed with water (20mL) and saturated brine (20mL), dried over anhydrous magnesium sulfate, filtered, and concentrated under reduced pressure. The residue was purified by silica column chromatography (PE:EA = 2:1) to give yellow oil **25-a** (150mg, yield 45%). LC-MS (ESI): m/z = 377 [M+H]⁺.

### Synthesis of compound 25

At room temperature, 20% NaOH aq. solution (2mL) was added to a solution of compound **25-a** (120mg, 0.32mmol) in methanol (2mL). The reaction solution was stirred for 2hrs, concentrated under reduced pressure to remove methanol, 6M HCl aq. solution was added to adjust pH=7, solid was precipitated and filtered out. The solid was washed with water (10mL), dried under vacuum to give white solid **25** (65mg, yield 56%). LC-MS (ESI): m/z = 363 [M+H]⁺.
¹H-NMR (400MHz, CD₃OD) δ: 7.91 (s, 1H), 7.30-7.46 (m, 4H), 6.67 (d, J=9.6 Hz, 1H), 4.60 (s, 2H), 2.89 (t, J=7.6 Hz, 1H), 2.67 (t, J=7.6 Hz, 2H), 2.38 (s, 3H) ppm.

### Embodiment 26

### 2-({1-[(2,6-dichlorophenyl)methyl]-3-methyl-1H-indazol-6-yl}thio)-2-methyl propionic acid (Compound 26)

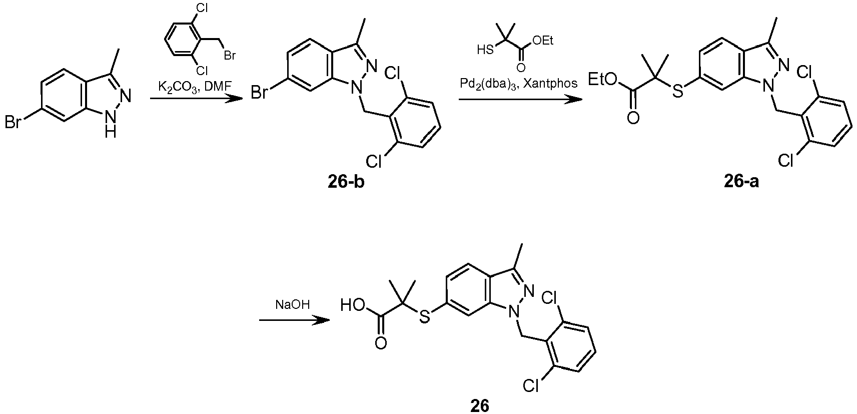

### Synthesis of compound 26-b

At room temperature, potassium carbonate (490mg, 3.55mmol) was added to a solution of 6-bromo-3-methyl-1H-indazole (500mg, 2.37mmol) and 2,6-dichlorobenzyl bromide (680mg, 2.84mmol) in DMF (5mL). The mixture was stirred for 12hrs, followed by adding water (10mL), being extracted with EA (20mL×3). The organic phases were combined, washed in turn with water (10mL) and saturated brine (10mL), dried over magnesium sulfate, filtered, and concentrated under reduced pressure. The residue was purified by silica column chromatography (PE:EA = 10:1) to give yellow oil **26-b** (400mg, yield 45%). LC-MS (ESI): m/z = 369 [M+H]⁺.

### Synthesis of compound 26-a

Under N₂ atmosphere, tris(dibenzylidene indene acetone)dipalladium (25.5mg, 0.02mmol), 4,5-bis(diphenylphosphine)-9,9-dimethyloxacanthracene (31mg, 0.05mmol) and CuI (5.1mg, 0.02mmol) were added to a solution of compound **26-b** (100mg, 0.27mmol), ethyl 2-methyl-2-mercaptopropionate (0.04mL, 0.27mmol) and diisopropylethylamine (0.14mL, 0.81mmol) in dioxane (2mL). The mixture was reacted in a microwave at 125°C for 1h, cooled to room temperature, concentrated under reduced pressure to remove dioxane. The residue was purified by silica preparative plate chromatography (PE:EA = 1:1) to give compound **26-a** (80mg, yield 67%). LC-MS (ESI): m/z = 437 [M+H]⁺.

### Synthesis of compound 26

At room temperature, NaOH (72mg, 1.8mmol) was added to a mixed solution of compound **26-a** (80mg, 0.18mmol) in methanol (2mL), THF (2mL) and water (2mL). The mixture was stirred for 2hrs, adjusted to pH=7 by 2M HCl aq. solution, extracted with EA (20mL×3). The organic phases were washed in turn with water (10mL) and saturated brine (10mL), dried over anhydrous magnesium sulfate, filtered, and concentrated under reduced pressure to give compound **26** (20mg, yield 27%). LC-MS (ESI): m/z = 409 [M+H]⁺.
¹H-NMR (400MHz, CD₃OD) δ: 7.60 (s, 1H), 7.52 (d, J=8.0 Hz, 1H), 7.34 (m, 2H), 7.24 (m, 1H), 7.13 (d, J=8.0 Hz, 1H), 5.64 (s, 1H), 2.37 (s, 3H), 1.35 (s, 6H) ppm.

### Embodiment 27

### 2- {[5-(4-Cyanophenyl)imidazo[1,2-a]pyridin-6-yl]thio}-2-methyl propionic acid (Compound 27)

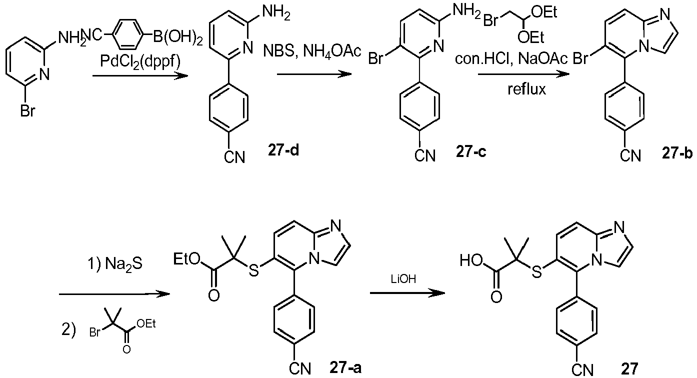

### Synthesis of compound 27-d

Under N₂ atmosphere, 2-amino-6-bromopyridine (500mg, 2.89mmol), 4-cyanophenylboronic acid (510mg, 3.47mmol) and sodium carbonate (920mg, 8.67mmol) were suspended in DMF (10mL) and water (5mL), [1,1'-bis(diphenylphosphine)ferrocene]palladium dichloride (240mg, 0.29mmol) was added. The mixture was stirred at 80 °C for 2hrs, cooled to room temperature, followed by adding water (15mL), being extracted with EA (30mL×3). The organic phase was washed in turn with water (20mL) and saturated brine (20mL), dried over anhydrous magnesium sulfate, filtered, and concentrated under reduced pressure. The residue was purified by silica column chromatography (PE:EA=2:1) to give compound **27-d** (400mg, yield 71%). LC-MS (ESI): m/z = 196 [M+H]⁺.

### Synthesis of compound 27-c

At 0°C, N-bromosuccinimide (360mg, 2.05mmol) was added to a solution of compound **27-d** (400mg, 2.05mmol) and ammonium acetate (160mg, 2.05mmol) in CH₃CN (10mL). The reaction solution was warmed to room temperature and stirred for 12hrs, and concentrated under reduced pressure to remove solvent. The residue was purified by silica column chromatography (PE:EA=2:1) to give compound **27-c** (500mg, yield 89%). LC-MS (ESI): m/z = 274 [M+H]⁺.

### Synthesis of compound 27-b

A solution of sodium acetate (42mg, 0.78mmol) and 2-bromo-1,1-diethoxyethane (0.28mL, 1.8mmol) in conc. HCl aqueous solution (0.1mL) and water (0.6mL) was heated to 110°C and refluxed for 10mins. The reaction solution was cooled to 60°C, the solution was added to a solution of compound **27-b** (250mg, 0.91mmol) and sodium acetate (83mg, 1.55mmol) in 60% ethanol aqueous solution (10mL). The mixture was heated to 100°C and refluxed for 2.5hrs, cooled to room temperature, and concentrated under reduced pressure. The residue was added to ice water (5mL), adjusted to pH=7 with saturated sodium bicarbonate solution, extracted with EA (30mL×3). The organic phase was washed in turn with water (20mL) and saturated brine (20mL), dried over anhydrous magnesium sulfate, filtered, and concentrated under reduced pressure. The residue was purified with silica column chromatography (PE:EA = 1:1) to give compound **27-b** (150mg, yield 55%). LC-MS (ESI): m/z = 298 [M+H]⁺.

### Synthesis of compound 27-a

Na₂S • 9H₂O (182mg, 0.75mmol) was added to a solution of compound **27-b** (180mg, 0.5mmol) in N-methyl pyrrolidone (2mL). The reaction solution was reacted in a microwave at 150°C for 1h, cooled to room temperature, followed by adding ethyl 2-bromo-2-methylpropionate (100mg, 0.67mmol) and potassium carbonate (90mg, 0.67mmol). The mixture was stirred at 50 for 2hrs, cooled to room temperature, followed by adding water (5mL), being extracted with EA (10mL). The organic phase was washed in turn with water (10mL) and saturated brine (10mL), dried over anhydrous magnesium sulfate, filtered, and concentrated under reduced pressure. The residue was purified by silica column chromatography (PE:EA = 1:1) to give compound **27-a** (10mg, yield 8%). LC-MS (ESI): m/z = 366 [M+H]⁺.

### Synthesis of compound 27

At room temperature, LiOH (13.1mg, 0.55mmol) was added to a solution of compound **27-a** (10mg, 0.02mmol) in methanol (2mL), THF (2mL) and water (4mL). The mixture was stirred for 2hrs, adjusted to pH=7 with 2M HCl aq. solution, extracted with EA (20mL×3). The organic phase was in turn washed with water (10mL) and saturated brine (10mL), dried over anhydrous magnesium sulfate, filtered, and concentrated under reduced pressure to give compound **27** (5mg, yield 54%). LC-MS (ESI): m/z = 338 [M+H]⁺.
¹H-NMR (400MHz, CD₃OD) δ: 7.97 (d, J=8.0 Hz, 1H), 7.59-7.70 (m, 5H), 7.26 (s, 1H), 1.38 (s, 6H) ppm.

### Embodiment 28

### 2-{[3-(4-Cyanophenyl)-[1,2,4]triazo[4,3-a]pyridin-6-yl]thio}-2-methyl propionic acid (Compound 28)

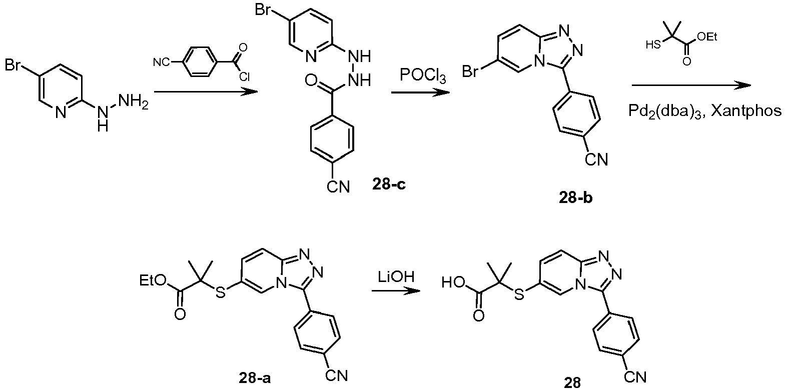

### Synthesis of compound 28-c

At room temperature, a mixture of 2-hydrazino-5-bromopyridine (1.0g, 5.3mmol), 4-cyanobenzoyl chloride (0.97g, 5.85mmol), triethyl amine (0.64g, 0.88mmol) and DCM (15mL) was stirred for 12hrs, and filtered. The solid was washed with DCM (5mL), dried under vacuum to give yellow solid **28-c** (1.13g, yield 67%). The produce was used directly for the next step without further purification. LC-MS (ESI): m/z = 319 [M+H]⁺.

### Synthesis of compound 28-b

Compound **28-c** (1.03g, 3.25mmol) was added to POCl₃ (10mL). The mixture was stirred at 100°C for 12hrs, cooled to room temperature, and concentrated under reduced pressure. Saturated NaHCO₃ aq. solution was added to the residue to adjust pH=7, the mixture was extracted with EA (50mL×2). The organic phase was in turn washed with water (30mL) and saturated NaHCO₃ aq. solution (30mL), dried over anhydrous sodium sulfate, filtered and concentrated under reduced pressure to give compound **28-b** (0.85g, yield 80%). The product was used directly for the next step without further purification. LC-MS (ESI): m/z = 301 [M+H]⁺.

### Synthesis of compound 28-a

Under N₂ atmosphere, tris(dibenzylidene indene acetone)dipalladium (54mg, 0.05mmol) and 4,5-bis(diphenylphosphine)-9,9-dimethyloxacanthracene (68mg, 0.11mmol) were added to a solution of compound **28-b** (350mg, 1.17mmol), ethyl 2-methyl-2-mercaptopropionate (208mg, 1.4mmol) and diisopropylethylamine (302mg, 2.34mmol) in dioxane (10mL). The mixture was reacted in a microwave at 110°C for 1h, cooled to room temperature, concentrated under reduced pressure to remove dioxane. The residue was purified by silica preparative plate chromatography (PE:EA = 3:1-1:1) to give compound **28-a** (268mg, yield 55%). LC-MS (ESI): m/z = 367 [M+H]⁺.

### Synthesis of compound 28

At room temperature, LiOH (51mg, 1.22mmol) was added to a solution of compound **28-a** (223mg, 0.61mmol) in methanol (2mL), THF (6mL) and water (2mL). The mixture was stirred for 3hrs, concentrated under reduced pressure, followed by adding water (10mL), being extracted with EA (30mL×2), the aqueous phase was adjusted to pH=5-6 with 2M HCl aq. solution and extracted with EA (30mL×2). The organic phases were combined, washed in turn with water (10mL) and saturated brine (10mL), dried over anhydrous sodium sulfate, filtered and concentrated under reduced pressure. The residue was recrystallized in EA (15mL) and PE (10mL) to give yellow solid **28** (108mg, yield 52%). LC-MS (ESI): m/z = 339 [M+H]⁺.
¹H-NMR (400MHz, DMSO-d6) δ: 12.92 (s, 1H), 8.56 (s, 1H), 8.12 (m, 4H), 7.92 (d, J=9.8 Hz, 1H), 7.45 (d, J=9.4 Hz, 1H), 1.44 (s, 6H) ppm.

### Embodiment 29

### 7-(4-Cyanonaphthalen-1-yl)thieno[3,2-c]pyridin-2-formic acid (Compound 29)

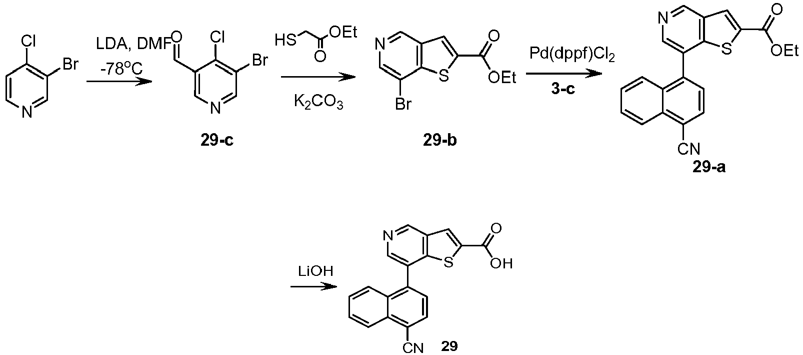

### Synthesis of compound 29-c

Under N₂ atmosphere, at -78 °C, a solution of 2.5M n-butyl lithium in n-hexane (24mL, 60mmol) was slowly added to a solution of diisopropylamine (6.1g, 60mmol) in anhydrous THF (100mL). The mixture was stirred for 15mins, followed by adding a solution of 3-bromo-4-chloropyridine (9.6g, 50mmol) in anhydrous THF (100mL), further stirred for 1h, followed by adding anhydrous DMF (10mL) and stirred for 30mins. The mixture was slowly warmed to room temperature, followed by adding saturated NH₄Cl aq. solution (300mL), the mixture was extracted with EA (300mL×3). The organic phases were combined, washed in turn with water (100mL) and saturated brine (100mL), dried over anhydrous sodium sulfate, filtered and concentrated under reduced pressure. The residue was purified by silica preparative plate chromatography (PE:EA = 2:1-1:1) to give light yellow solid **29-c** (5.9g, yield 54%).
¹H-NMR (400MHz, CDCl₃) δ: 10.44 (s, 1H), 8.80 (s, 1H), 7.67 (s, 1H) ppm.

### Synthesis of compound 29-b

Ethyl mercaptoacetate (2.4g, 20mmol) and potassium carbonate (3.0g, 24mmol) were added to a solution of compound **29-c** (4.4g, 20mmol) in DMF (40mL). The mixture was heated to 45°C and stirred for 12hrs, cooled to room temperature, followed by adding ice water (200mL), solid was precipitated and filtered out. The solid was washed with water (100mL×3) and dried under vacuum to give white solid **29-b** (5.1g, yield 89.5%). The product was used directly for the next step without further purification. LC-MS (ESI): m/z = 286 [M+H]⁺.

### Synthesis of compound 29-a

Under N₂ atmosphere, compound **29-b** (285mg, 1mmol), compound **3-c** (279mg, 1mmol) and sodium carbonate (212mg, 2mmol) were suspended in dioxane (6mL) and water (2mL), [1,1'-bis(diphenylphosphine)ferrocene]palladium dichloride (73mg, 0.1mmol) was added. The mixture was stirred at 80°C for 3hrs, cooled to room temperature and concentrated under reduced pressure. The residue was purified by silica preparative plate (PE:EA = 2:1) to give compound **29-a** (190mg, yield 53%). LC-MS (ESI): m/z = 359 [M+H]⁺.

### Synthesis of compound 29

At room temperature, LiOH (41mg, 1mmol) was added to a solution of compound **29-a** (190mg, 0.53mmol) in methanol (3mL), THF (3mL) and water (3mL). The mixture was stirred for 1h, adjusted to pH=5-6 with 2M HCl aq. solution, solid was precipitated and filtered out. The solid was washed with water (10mL), dried under vacuum to give light yellow solid **29** (130mg, yield 74%). LC-MS (ESI): m/z = 331 [M+H]⁺.
¹H-NMR (400MHz, DMSO-d6) δ: 9.42 (s, 1H), 8.64 (s, 1H), 8.40 (s, 1H), 8.35 (d, J=8.0 Hz, 1H), 8.29 (d, J=8.0 Hz, 1H), 7.90 (m, 2H), 7.66 (s, 2H) ppm.

### Embodiment 30

### 3-[7-(4-Cyanonaphthalen-1-yl)thieno[3,2-c]pyridin-2-yl]propionic acid (Compound 30)

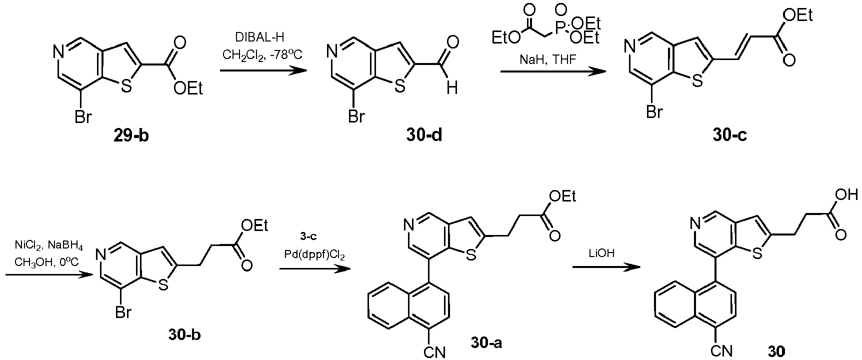

### Synthesis of compound 30-d

At -78°C, a solution of 1.0M diisobutylaluminum hydride in DCM (58mL, 58mmol) was slowly added to a solution of compound **29-b** (5.7g, 20mmol) in DCM (50mL). The mixture was stirred for 1h, warmed to room temperature, followed by adding saturated NH₄Cl aq. solution (300mL). The organic phase was seperated, the aqueous phase was extracted with DCM (50mL×3). The organic phases were combined, washed in turn with water (50mL) and saturated brine (50mL), dried over anhydrous sodium sulfate, filtered and concentrated under reduced pressure. The residue was purified by silica column chromatography (PE:EA = 3:1) to give light yellow solid **30-d** (4g, yield 83%). LC-MS (ESI): m/z = 242 [M+H]⁺.

### Synthesis of compound 30-c

At 0°C, triethyl phosphonoacetate (2.82mL, 10mmol) and sodium hydride (0.48g, 12mmol) were respectively added to a solution of compound **30-d** (2.42g, 10mmol) in THF (50mL). The mixture was further stirred for 1h, warmed to room temperature, followed by adding saturated NH₄Cl aq. solution (300mL), the mixture was extracted with EA (50mL×3). The organic phases were combined, washed in turn with water (30mL×3) and saturated brine (30mL), dried over anhydrous sodium sulfate, filtered, and concentrated under reduced pressure. The residue was purified by silica column chromatography (PE:EA = 5:1) to give light yellow solid **30-c** (2g, yield 64%). LC-MS (ESI): m/z =312 [M+H]⁺.

### Synthesis of compound 30-b

At 0°C, NaBH₄ (0.25g, 6.4mmol) was slowly added to a solution of compound **30-c** (2.0g, 6.4mmol) and NiCl (0.82g, 6.4mmol) in methanol (50mL). The mixture was stirred for 3hrs, warmed to room temperature, followed by adding NH₄Cl aq. solution (300mL), the mixture was extracted with EA (100mL×3). The organic phases were combined, washed in turn with water (50mL×3) and saturated brine (50mL), dried over anhydrous sodium sulfate, filtered and concentrated under reduced pressure. The residue was purified by silica column chromatography (PE:EA = 4:1) to give light yellow solid **30-b** (1.6g, yield 80%). LC-MS (ESI): m/z =314 [M+H]⁺.

### Synthesis of compound 30-a

Under N₂ atmosphere, compound **30-b** (155mg, 0.5mmol), compound **3-c** (140mg, 0.5mmol) and sodium carbonate (106mg, 1mmol) were suspended in dioxane (4mL) and water (1mL), [1,1'-bis(diphenylphosphine)ferrocene]palladium dichloride (40mg, 0.05mmol) was added. The mixture was stirred at 80°C for 3hrs, cooled to room temperature and concentrated under reduced pressure. The residue was filtered through celite, the filtrate cake was washed with EA (30mL). The filtrate was washed in turn with water (10mL×3) and saturated brine (10mL), dried over anhydrous sodium sulfate, filtered and concentrated under reduced pressure. The residue was purified by silica column chromatography (PE:EA = 1:1) to give compound **30-b** (120mg, yield 62%). LC-MS (ESI): m/z = 387 [M+H]⁺.

### Synthesis of compound 30

At room temperature, LiOH (42mg, 1mmol) was added to a solution of compound **30-a** (120mg, 0.31mmol) in methanol (1mL), THF (4mL) and water (1mL). The mixture was stirred for 1h, adjusted to pH=5-6 by 2M HCl aq. solution, solid was precipitated and filtered out. The solid was washed with water (10mL), dried under vacuum to give white solid **30** (93mg, yield 84%). LC-MS (ESI): m/z = 359 [M+H]⁺.
¹H-NMR (400MHz, DMSO-d6) δ: 9.15 (s, 1H), 8.42 (s, 1H), 8.32 (d, J=8.0 Hz, 1H), 8.27 (d, J=8.0 Hz, 1H), 7.90 (m, 1H), 7.81 (m, 1H), 7.68 (m, 2H), 3.10 (t, J=8.0 Hz, 2H), 3.07 (d, J=8.0 Hz, 2H) ppm.

### Embodiment 31

### 2-[7-(4-Cyanonaphthalen-1-yl)thieno[3,2-c]pyridin-2-yl]acetic acid (Compound 31)

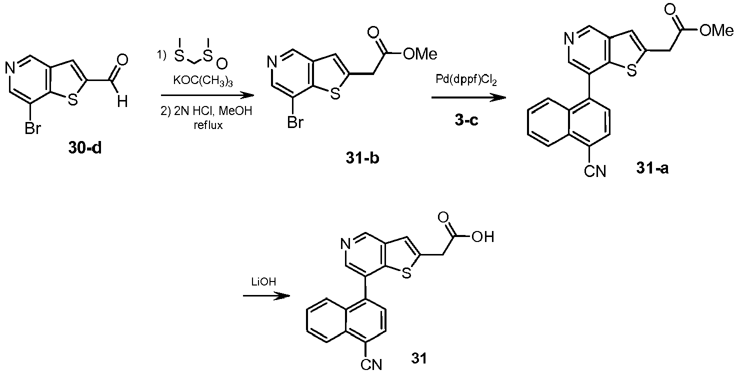

### Synthesis of compound 31-b

NaOH (40mg, 1mmol) was added to a solution of methyl(methylthiomethyl)sulfoxide (18mg, 1.5mmol) and compound **30-d** (240mg, 1mmol) in THF (6mL). The mixture was heated to 80 °C and stirred for 4hrs, cooled to room temperature, concentrated under reduced pressure to remove the solvent. The residue was added to a solution of 2M HCl in methanol (10mL), refluxed for 1h, concentrated under reduced pressure. The residue was added to saturated NaHCO₃ aq. solution (10mL), extracted with EA (10mL×3). The organic phases were combined, washed in turn with water (10mL×3) and saturated brine (10mL), dried over anhydrous sodium sulfate, filtered and concentrated under reduced pressure. The residue was purified by silica column chromatography (PE:EA = 4:1) to give compound **31-b** (0.15g, yield 88%). LC-MS (ESI): m/z =285 [M+H]⁺.

### Synthesis of compound 31-a

Under N₂ atmosphere, compound **31-b** (87mg, 0.3mmol), compound **3-c** (84mg, 0.3mmol) and sodium carbonate (60mg, 0.6mmol) were suspended in dioxane (4mL) and water (1mL), [1,1'-bis(diphenylphosphine)ferrocene]palladium dichloride (25mg, 0.03mmol) was added. The mixture was stirred at 80°C for 3hrs, cooled to room temperature and concentrated under reduced pressure. The residue was filtered through celite, the filtrate cake was washed with EA (30mL). The filtrate was washed in turn with water (20mL×3) and saturated brine (10mL), dried over anhydrous magnesium sulfate, filtered and concentrated under reduced pressure. The residue was purified by silica column chromatography (PE:EA = 1:1) to give compound **31-a** (76mg, yield 71%). LC-MS (ESI): m/z = 359 [M+H]⁺.

### Synthesis of compound 31

At room temperature, LiOH (42mg, 1mmol) was added to a solution of compound **31-a** (120mg, 0.31mmol) in methanol (1mL), THF (4mL) and water (1mL). The mixture was stirred for 1h, followed by adding 2M HCl aq. solution to adjust pH=5-6, solid was precipitated and filtered out. The solid was washed with water (10mL), dried under vacuum to give white solid **31** (44mg, yield 64%). LC-MS (ESI): m/z = 345 [M+H]⁺.
¹H-NMR (400MHz, DMSO-d6) δ: 9.30 (s, 1H), 8.55 (s, 1H), 8.36 (d, J=7.6 Hz, 1H), 8.29 (d, J=7.6 Hz, 1H), 7.90 (m, 2H), 7.72 (m, 3H), 4.04 (s, 2H) ppm.

### Embodiment 32

### 2-[7-(4-Cyanophenyl)thieno[3,2-c]pyridin-2-yl]acetic acid (Compound 32)

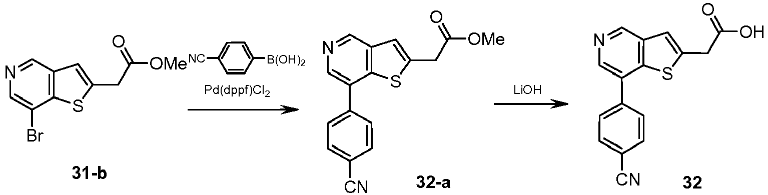

### Synthesis of compound 32-a

Under N₂ atmosphere, compound **31-b** (140mg, 0.5mmol), 4-cyanophenyl boronic acid (75mg, 0.5mmol) and sodium carbonate (60mg, 0.6mmol) were suspended in dioxane (4mL) and water (10mL), [1,1'-bis(diphenylphosphine)ferrocene]palladium dichloride (25mg, 0.03mmol) was added. The mixture was stirred at 80°C for 3hrs, cooled to room temperature and concentrated under reduced pressure. The residue was filtered through celite, the filtrate cake was washed with EA (30mL). The filtrate was in turn washed with water (20mL×3) and saturated brine (10mL), dried over anhydrous magnesium sulfate, filtered, concentrated under reduced pressure. The residue was purified by silica column chromatography (PE:EA = 1:1) to give compound **32-b** (86mg, yield 56%). LC-MS (ESI): m/z = 309 [M+H]⁺.

### Synthesis of compound 32

At room temperature, LiOH (42mg, 1mmol) was added to a solution of compound **32-a** (86mg, 0.28mmol) in methanol (1mL), THF (4mL) and water (1mL). The mixture was stirred for 1h, followed by adding 2M HCl aq. solution (2mL), solid was precipitated and filtered out. The solid was washed with water (10mL), dried under vacuum to give white solid **32** (28mg, yield 34%). LC-MS (ESI): m/z = 295 [M+H]⁺.
¹H-NMR (400MHz, DMSO-d6) δ: 12.87 (s, 1H), 9.11 (s, 1H), 8.64 (s, 1H), 8.06 (d, J=7.6 Hz, 1H), 7.91 (d, J=7.6 Hz, 1H), 7.57 (s, 1H), 4.06 (s, 2H) ppm.

### Embodiment 33

### 3-[7-(4-Cyanophenyl)thieno[3,2-c]pyridin-2-yl]-2,2-dimethylpropionic acid (Compound 33)

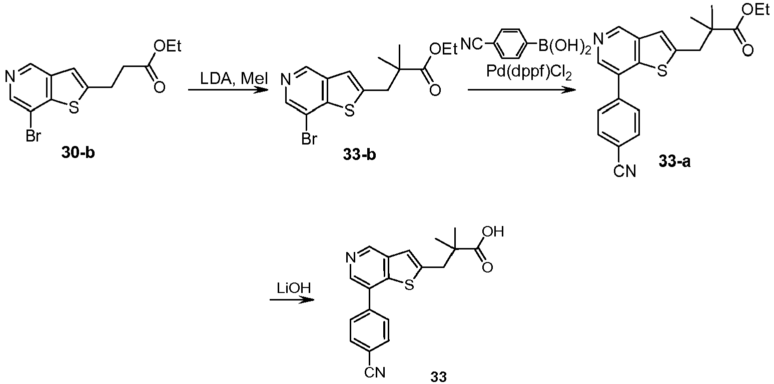

### Synthesis of compound 33-b

Under N₂ atmosphere, at -78 °C, a solution of 2.5M n-butyl lithium in n-hexane (2.0mL, 5mmol) was slowly added to a solution of diisopropylamine (505mg, 5mmol) in anhydrous THF (10mL). The mixture was stirred for 15mins, added dropwise to a solution of compound **30-b** (630mg, 2mmol) in anhydrous THF (10mL), stirred for 2hrs, followed by adding CH₃I (720mg, 5mmol) and the mixture was further stirred for 3hrs. The mixture was slowly warmed to room temperature, then added to saturated NH₄Cl aq. solution (30mL), extracted with EA (30mL×3). The organic phases were combined, washed in turn with water (10mL) and saturated brine (10mL), dried over anhydrous sodium sulfate, filtered and concentrated under reduced pressure. The residue was purified by silica preparative plate chromatography (PE:EA = 2:1-1:1) to give light yellow liquid **33-b** (310mg, yield 45%).

### Synthesis of compound 33-a

Under N₂ atmosphere, compound **33-b** (310mg, 0.91mmol), 4-cyanophenyl boronic acid (140mg, 0.91mmol) and sodium carbonate (212mg, 2mmol) were suspended in dioxane (4mL) and water (1mL), [1,1'-bis(diphenylphosphine)ferrocene]palladium dichloride (40mg, 0.05mmol) was added. The mixture was stirred at 80°C for 3hrs, and then cooled to room temperature, concentrated under reduced pressure to remove the solvent. The residue was filtered through celite, the filtrate cake was washed with EA (30mL). The filtrate was in turn washed with water (20mL×3) and saturated brine (10mL), dried over anhydrous magnesium sulfate, filtered, concentrated under reduced pressure. The residue was purified by silica column chromatography (PE:EA =1:1) to give yellow liquid **33-a** (76mg, yield 23%). LC-MS (ESI): m/z = 365 [M+H]⁺.

### Synthesis of compound 33

At room temperature, LiOH (42mg, 1mmol) was added to a solution of compound **33-a** (73mg, 0.19mmol) in methanol (1mL), THF (4mL) and water (1mL). The mixture was stirred for 1h, followed by adding 2M HCl aq. solution (2mL) and water (1mL), solid was precipitated and filtered out. The solid was washed with water (5mL), dried under vacuum to give white solid **33** (39mg, yield 61%). LC-MS (ESI): m/z = 337 [M+H]⁺.
¹H-NMR (400MHz, CD₃OD) δ: 9.34 (s, 1H), 8.64 (s, 1H), 8.02 (s, 4H), 7.71 (s, 1H), 7.57 (s, 1H), 3.34 (s, 2H), 1.26 (s, 6H) ppm.

### Embodiment 34

### 3-[7-(4-Cyanonaphthalen-1-yl)thieno[3,2-c]pyridin-2-yl]-2,2-dimethylpropionic acid (Compound 34)

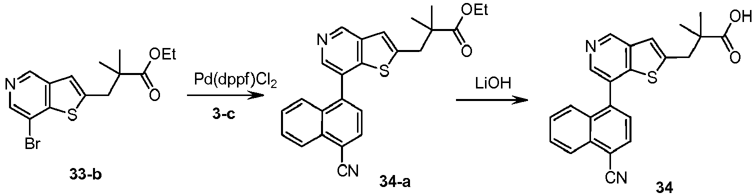

### Synthesis of compound 34-a

Under N₂ atmosphere, compound **33-b** (230mg, 0.7mmol), compound **3-c** (280mg, 0.5mmol) and sodium carbonate (150mg, 1.4mmol) were suspended in dioxane (4mL) and water (1mL), [1,1'-bis(diphenylphosphine)ferrocene]palladium dichloride (40mg, 0.05mmol) was added. The mixture was stirred at 80°C for 3hrs, cooled to room temperature and concentrated under reduced pressure. The residue was filtered through celite, the filtrate cake was washed with EA (30mL). The filtrate was in turn washed with water (20mL×3) and saturated brine (10mL), dried over anhydrous magnesium sulfate, filtered, concentrated under reduced pressure. The residue was prepared by HPLC (mobile phase: 10mM NH₄HCO₃ aq. solution: acetonitrile =35%-45%) to give compound **34-a** (53mg, yield 18%). LC-MS (ESI): m/z = 401 [M+H]⁺.

### Synthesis of compound 34

At room temperature, LiOH (42mg, 1mmol) was added to a solution of compound **34-a** (41mg, 0.1mmol) in methanol (1mL), THF (4mL) and water (1mL). The mixture was stirred for 1h, followed by adding 2M HCl aq. solution (2mL) and water (1mL), solid was precipitated and filtered out. The solid was washed with water (5mL), dried under vacuum to give white solid **34** (16mg, yield 41%). LC-MS (ESI): m/z = 387 [M+H]⁺.
¹H-NMR (400MHz, DMSO-d6) δ: 12.41 (s, 1H), 9.19 (s, 1H), 8.43 (s, 1H), 8.32 (d, J=7.6 Hz, 1H), 8.27 (d, J=7.6 Hz, 1H), 7.88 (m, 2H), 7.65 (m, 2H), 7.47 (s, 1H), 3.14 (s, 2H), 1.12 (s, 6H) ppm.

### Embodiment 35

### 3-{7-[(2,6-Dichlorophenyl)methyl]-1-benzothiophene-2-yl}propionic acid (Compound 35)

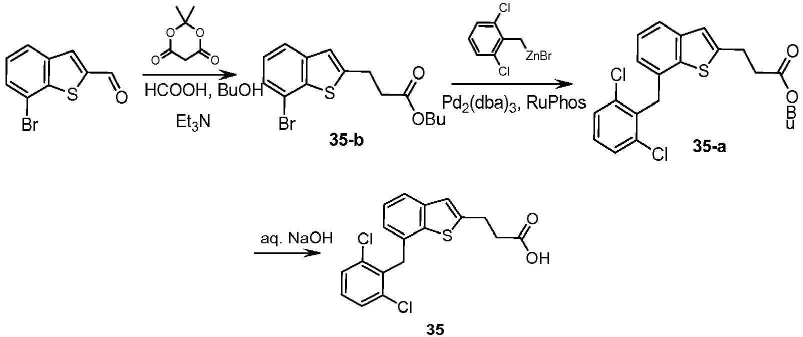

### Synthesis of compound 35-b

At 0°C, triethyl amine (3.6mL) was slowly added to a mixture of n-butanol (10mL) and formic acid (1mL). The mixture was stirred for 10mins, followed by adding 7-bromo-1-benzothiophene-2-carbaldehyde (241mg, 1mmol) and 2,2-dimethyl-1,3- dioxane-4,6-dione (216mg, 1.5mmol). The mixture was heated to reflux for 8hrs, cooled to room temperature and concentrated under reduced pressure. The residue was purified by silica column chromatography (PE:EA = 15:1) to give compound **35-b** (200mg, yield 59%). LC-MS (ESI): m/z = 341 [M+H]⁺.

### Synthesis of compound 35-a

Under N₂ atmosphere, tris(dibenzylidene indene acetone)dipalladium (54mg, 0.05mmol) and 4,5-bis(diphenylphosphine)-9,9-dimethyloxacanthracene (94mg, 0.02mmol) and a solution of 0.4M 2,6-dichlorobenzyl zinc bromide in THF solution (2.5mL, 1mmol) were added to a solution of compound **35-b** (170mg, 0.5mmol) in anhydrous THF (10mL). The mixture was reacted at 60°C for 16hrs, cooled to room temperature, concentrated under reduced pressure. The residue was purified by silica column chromatography (PE:EA = 10:1) to give compound **35-a** (170mg, yield 80%). LC-MS (ESI): m/z = 421 [M+H]⁺.

### Synthesis of compound 35

At room temperature, 1.0M NaOH aq. solution (3mL) was added to a solution of compound **35-a** (84mg, 0.2mmol) in methanol (4mL) and THF (8mL). The mixture was stirred for 16hrs, and concentrated under reduced pressure. The residue was adjusted to pH=3 with 1M HCl aq. solution, solid was precipitated and filtered out. The solid was washed with water (5mL), dried under vacuum to give white solid **35** (60mg, yield 82%). LC-MS (ESI): m/z = 365 [M+H]⁺.
¹H-NMR (400MHz, DMSO-d6) δ: 12.35 (s, br. 1H), 7.61 (d, J=8.0 Hz, 1H), 7.58 (d, J=8.0 Hz, 2H), 7.42 (t, J=8.0 Hz, 1H), 7.25 (s, 1H), 7.21 (t, J=8.0 Hz, 1H), 6.47 (d, J=7.2 Hz, 1H), 4.35 (s, 2H), 3.16 (t, J=7.2 Hz, 2H), 2.71 (t, J=7.2 Hz, 2H) ppm.

### Embodiment 36

### 3-{4-[(2,6-Dichlorophenyl)methyl]-1-benzothiophene-2-yl}propionic acid (Compound 36)

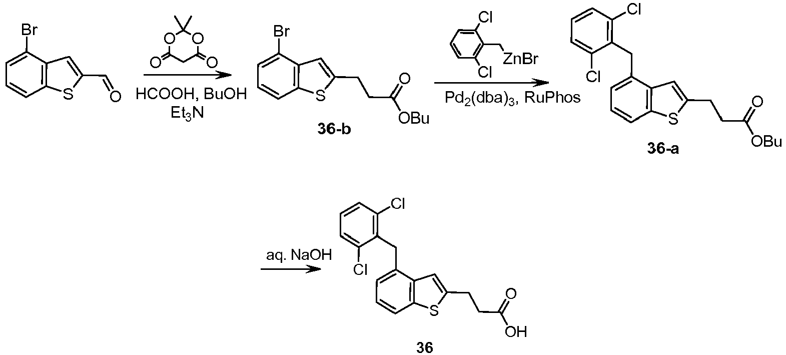

### Synthesis of compound 36-b

At 0°C, triethyl amine (3.6mL) was slowly added to a mixture of n-butanol (10mL) and formic acid (1mL). The mixture was stirred for 10mins, followed by adding 4-bromo-1-benzothiophene-2-carbaldehyde (241mg, 1mmol) and 2,2-dimethyl-1,3-dioxane-4,6-dione (216mg, 1.5mmol). The mixture was heated to reflux for 8hrs, cooled to room temperature, concentrated under reduced pressure. The residue was purified by silica column chromatography (PE:EA = 15:1) to give compound **36-b** (221mg, yield 65%). LC-MS (ESI): m/z = 341 [M+H]⁺.

### Synthesis of compound 36-a

Under N₂ atmosphere, tris(dibenzylidene indene acetone)dipalladium (47mg, 0.05mmol) and 2-dicyclohexylphospho-2',6'-diisopropoxy-1,1'-biphenyl (94mg, 0.02mmol) and a solution of 0.4M 2,6-dichlorobenzyl zinc bromide in THF solution (2.5mL, 1mmol) were added to a solution of compound **36-b** (170mg, 0.5mmol) in anhydrous THF (10mL). The mixture was reacted at 60°C for 16hrs, cooled to room temperature, concentrated under reduced pressure. The residue was purified by silica column chromatography (PE:EA = 10:1) to give compound **36-a** (180mg, yield 86%). LC-MS (ESI): m/z = 421 [M+H]⁺.

### Synthesis of compound 36

At room temperature, a solution of 1.0M NaOH aq. solution (3mL) was added to a solution of compound **36-a** (84mg, 0.2mmol) in methanol (4mL) and THF (8mL). The mixture was stirred for 16hrs, and concentrated under reduced pressure. The residue was adjusted to pH=3 with 1M HCl aq. solution, solid was precipitated and filtered out. The solid was washed with water (5mL), dried under vacuum to give white solid **36** (70mg, yield 95%). LC-MS (ESI): m/z = 365 [M+H]⁺.
¹H-NMR (400MHz, DMSO-d6) δ: 12.30 (s, br. 1H), 7.73 (d, J=8.0 Hz, 1H), 7.57 (d, J=8.0 Hz, 2H), 7.51 (s, 1H), 7.41 (t, J=8.0 Hz, 1H), 7.13 (t, J=8.0 Hz, 1H), 6.41 (d, J=7.2 Hz, 1H), 4.54 (s, 2H), 3.18 (t, J=7.2 Hz, 2H), 2.72 (t, J=7.2 Hz, 2H) ppm.

### Embodiment 37

### 3-[4-[(4-Cyanonaphthalen-1-yl)thieno[2,3-c]pyridine-2-yl]propionic acid (Compound 37)

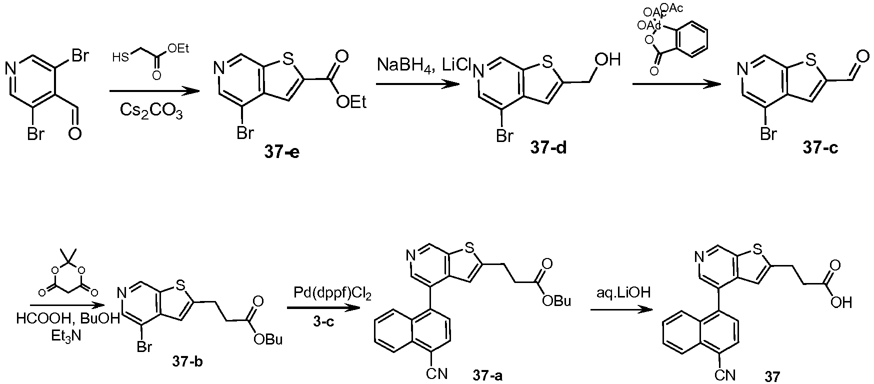

### Synthesis of compound 37-e

Ethyl mercaptoacetate (1.81g, 15.1mmol) and cesium carbonate (6.0g, 18.6mmol) were added to a solution of 3,5-dibromo-4-pyridinecarboxaldehyde (4.0g, 15. 1mmol) in THF (100mL). The mixture was stirred at 60°C for 3hrs, cooled to room temperature, concentrated under reduced pressure. The residue was purified by silica column chromatography (PE:EA = 15:1) to give compound **37-e** (3.7g, yield 85%). LC-MS (ESI): m/z = 286 [M+H]⁺.

### Synthesis of compound 37-d

At 0°C, NaBH₄ (530mg, 13.9mmol) was added into a solution of compound **37-e** (1.0g, 3.48mmol), LiCl (590mg, 13.9mmol) in THF (40mL) and methanol (20mL). The mixture was warmed to room temperature and stirred for 4hrs, concentrated under reduced pressure. Water (40mL) and DCM (40mL) were added to the residue, the organic phase was seperated, aqueous phase was extracted with DCM (20mL×3). The organic phases were combined, washed in turn with water (20mL×3) and saturated brine (10mL), dried over anhydrous sodium sulfate, filtered and concentrated under reduced pressure to give compound **37-d** (750mg, yield 89%). The product was used directly for the next step without further purification. LC-MS (ESI): m/z = 244 [M+H]⁺.

### Synthesis of compound 37-c

At 0°C, (1,1,1-triacetoxy)-1,1-dihydro-1,2-benzoiodooxol-3(1H)-one (1.39g, 3.27mmol) was added to a solution of compound **37-d** (750mg, 2.18mmol) in DCM (30mL). The reaction solution was warmed to room temperature and further stirred for 2hrs, followed by adding saturated sodium bicarbonate aq. solution (10mL) and saturated sodium thiosulfate aq. solution (10mL). The mixture was stirred for 10mins, organic phase was seperated. The organic phase was in turn washed with water (20mL×3) and saturated brine (20mL), dried over anhydrous magnesium sulfate, filtered, concentrated under reduced pressure. The residue was purified by silica column chromatography (PE:EA = 10:1) to give compound **37-c** (680mg, yield 91%). LC-MS (ESI): m/z = 342 [M+H]⁺.

### Synthesis of compound 37-b

At 0°C, triethyl amine (3.6mL) was slowly added to a mixture of n-butanol (10mL) and formic acid (1mL). The mixture was stirred for 10mins, followed by adding compound **37-c** (300mg, 1.24mmol) and 2,2-dimethyl-1,3-dioxane-4,6-dione (300mg, 2.08mmol), refluxing for 8hrs. The mixture was cooled to room temperature, concentrated under reduced pressure. The residue was purified by silica column chromatography (PE:EA = 12:1) to give compound **37-b** (280mg, yield 66%). LC-MS (ESI): m/z = 342 [M+H]⁺.

### Synthesis of compound 37-a

Under N₂ atmosphere, compound **37-b** (120mg, 0.35mmol), compound **3-c** (110mg, 0.39mmol) and sodium carbonate (150mg, 1.4mmol) were suspended in ethylene glycol dimethyl ether (10mL) and water (1mL), [1,1'-bis(diphenylphosphine)ferrocene]palladium dichloride (40mg, 0.05mmol) was added. The mixture was stirred at 75°C for 16hrs, cooled to room temperature, and concentrated under reduced pressure. The residue was purified by silica column chromatography (PE:EA = 8:1) to give compound **37-a** (90mg, yield 62%). LC-MS (ESI): m/z = 415 [M+H]⁺.

### Synthesis of compound 37

At room temperature, a solution of 1.0M LiOH aq. solution (2mL) was added to a solution of compound **37-a** (100mg, 0.25mmol) in methanol (10mL) and THF (10mL). The mixture was stirred for 16hrs, concentrated under reduced pressure. The residue was dissolved in water (10mL), adjusted to pH=3 with 1M citric acid aq. solution, solid was precipitated and filtered out. The solid was washed with water (5mL), dried under vacuum to give white solid **37** (50 mg, yield 55%). LC-MS (ESI): m/z = 359 [M+H]⁺.
¹H-NMR (400MHz, DMSO-d6) δ: 12.30 (s, br. 1H), 9.30 (s, 1H), 8.44 (s, 1H), 8.32 (d, J=8.0 Hz, 1H), 8.26 (d, J=8.0 Hz, 1H), 7.87 (t, J=8.8 Hz, 1H), 7.73 (d, J=7.2 Hz, 1H), 7.68 (t, J=7.2 Hz, 1H), 7.57 (d, J=8.8 Hz, 1H), 6.74 (s, 1H), 3.10 (t, J=7.2 Hz, 2H), 2.59 (t, J=7.2 Hz, 2H) ppm.

### Embodiment 38

### 3-[4-(4-Cyanonaphthalen-1-yl)-1-benzothiophen-2-yl]-2,2-dimethyl propionicacid (Compound 38)

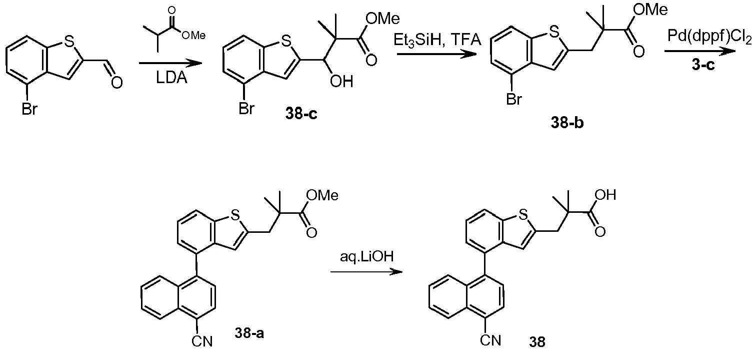

### Synthesis of compound 38-c

At -78 °C, a solution of 1M lithium diisopropylamide in THF (3mL, 3mmol) was added slowly to a solution of methyl isobutyrate (714mg, 7.1mmol) in anhydrous THF (5mL). The mixture was stirred for 1h, followed by adding 4-bromo-1-benzothiophene-2-carbaldehyde (500mg, 2.38mmol). The mixture was slowly warmed to room temperature, followed by adding NH₄Cl aq. solution (20mL), being extracted with EA (30mL×3). The organic phases were combined, dried over anhydrous sodium sulfate, filtered and concentrated under reduced pressure. The residue was purified by silica column chromatography (PE:EA = 10:1) to give compound **38-c** (700mg, yield 86%). LC-MS (ESI): m/z = 365 [M+Na]⁺.

### Synthesis of compound 38-b

At 0°C, trifluoroacetic acid (2mL) was added to a solution of compound **38-c** (170mg, 0.5mmol) and triethylsilane (392mg, 4mmol) in DCM (10mL). The mixture was warmed to room temperature and further stirred for 16hrs, and then concentrated under reduced pressure. DCM (30mL) was added to the residue. The mixture was in turn washed with saturated sodium bicarbonate solution (10mL) and saturated brine (10mL), dried over anhydrous sodium sulfate, filtered, concentrated under reduced pressure. The residue was purified by silica column chromatography (PE:EA = 10:1) to give compound **38-b** (150mg, yield 92%). LC-MS (ESI): m/z = 349 [M+Na]⁺.

### Synthesis of compound 38-a

Under N₂ atmosphere, compound **38-b** (150mg, 0.46mmol), compound **3-c** (150mg, 0.54mmol) and sodium carbonate (300mg, 2.8mmol) were suspended in ethylene glycol dimethyl ether (12mL) and water (1mL), [1,1'-bis(diphenylphosphine)ferrocene]palladium dichloride (50mg, 0.06mmol) was added. The mixture was stirred at 75°C for 16hrs, cooled to room temperature, concentrated under reduced pressure. The residue was purified by column chromatography (PE:EA = 4:1) to give compound **38-a** (105mg, yield 57%). LC-MS (ESI): m/z = 400 [M+H]⁺.

### Synthesis of compound 38

At room temperature, 1.0M LiOH aq. solution (2.5mL) was added to a solution of compound **38-a** (100mg, 0.25mmol) in methanol (10mL) and THF (10mL). The mixture was stirred for 16hrs, concentrated under reduced pressure. Water (10mL) was added to the residue. The mixture was adjusted to pH=3 with 1M citric acid aq. solution, solid was precipitated and filtered out. The solid was washed with water (5mL), dried under vacuum to give white solid **37** (80 mg, yield 83%). LC-MS (ESI): m/z = 408 [M+Na]⁺.
¹H-NMR (400MHz, DMSO-d6) δ: 12.37 (s, br. 1H), 8.29 (d, J=8.0 Hz, 1H), 8.24 (d, J=8.0 Hz, 1H), 8.06 (d, J=8.0 Hz, 1H), 7.85 (t, J=8.0 Hz, 1H), 7.67 (d, J=8.0 Hz, 1H), 7.58 -7.62 (m, 2H), 7.49 (t, J=7.2 Hz, 2H), 7.36 (d, J=7.2 Hz, 1H), 2.99 (s, 2H), 1.06 (s, 6H) ppm.

### Embodiment 39

### 3-[4-(4-Cyanonaphthalen-1-yl)-1-benzothiophen-2-yl]-2,2-difluoro-3-hydroxyl propionic acid (Compound 39)

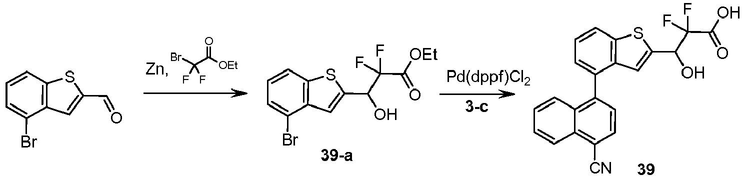

### Synthesis of compound 39-a

Under N₂ atmosphere, Zn powder (130mg, 2mmol) was added to a solution of 4-bromo-1-benzothiophene-2-carbaldehyde (500mg, 2.38mmol) and ethyl difluorobromoacetate (808mg, 4mmol) in anhydrous THF (10mL). The mixture was heated to 45°C and further stirred for 16hrs, cooled to room temperature, followed by adding saturated NH₄Cl aq. solution (20mL), being extracted with EA (30mL×3). The organic phases were combined, dried over anhydrous sodium sulfate, filtered, and concentrated under reduced pressure. The residue was purified by silica column chromatography (PE:EA = 10:1) to give compound **39-a** (253mg, yield 71%). LC-MS (ESI): m/z = 388 [M+Na]⁺.

### Synthesis of compound 39

Under N₂ atmosphere, compound **39-a** (190mg, 0.5mmol), compound **3-c** (140mg, 0.5mmol) and sodium carbonate (106mg, 1mmol) were suspended in ethylene glycol dimethyl ether (20mL) and water (2mL), [1,1'-bis(diphenylphosphine)ferrocene] palladium dichloride (50mg, 0.06mmol) was added. The mixture was stirred at 75°C for 16hrs, cooled to room temperature, concentrated under reduced pressure. Water (20mL) was added to the residue, the mixture was extracted with EA (30mL×3). The aqueous phase was adjusted to pH=3 with 1M HCl aqueous solution, solid was precipitated and filtered out. The solid was washed with water (5mL), dried under vacuum to give white solid **39** (120mg, yield 58%). LC-MS (ESI): m/z = 432 [M+Na]⁺.
¹H-NMR (400MHz, DMSO-d6) δ: 8.30 (d, J=7.2 Hz, 1H), 8.25 (d, J=8.0 Hz, 1H), 8.14 (d, J=8.0 Hz, 1H), 7.85 (t, J=7.0 Hz, 1H), 7.65-7.68 (m, 1H), 7.60 -7.63 (m, 1H), 7.55 (t, J=8.0 Hz, 2H), 7.39-7.42 (m, 1H), 6.82 (d, J=16.8 Hz, 1H), 5.23-5.30 (m, 1H) ppm.

### Embodiment 40

### 3- {4-[(2,6-Dichlorophenyl)methyl]-1-benzothiophen-2-yl]-2,2-dimethyl propionic acid (Compound 40)

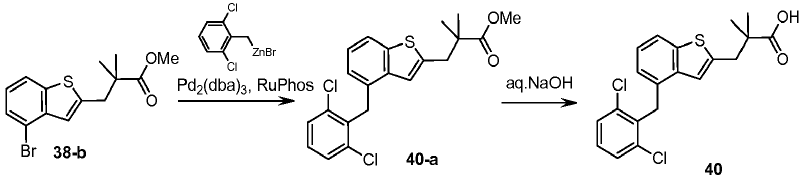

### Synthesis of compound 40-a

Under N₂ atmosphere, tris(dibenzylidene indene acetone)dipalladium (47mg, 0.05mmol) and 2-dicyclohexylphospho-2',6'-diisopropoxy-1,1'-biphenyl (94mg, 0.02mmol) and a solution of 0.4M 2,6-dichlorobenzyl zinc bromide in THF solution (2.5mL, 1mmol) were added to a solution of compound **38-b** (130mg, 0.4mmol) in anhydrous THF (10mL). The mixture was reacted at 60°C for 16hrs, cooled to room temperature, concentrated under reduced pressure. The residue was purified by silica column chromatography (PE:EA = 8:1) to give compound **40-a** (150mg, yield 72%). LC-MS (ESI): m/z = 407 [M+H]⁺.

### Synthesis of compound 40

At room temperature, 1.0M NaOH aq. solution (1mL) was added to a solution of compound **40-a** (84mg, 0.2mmol) in methanol (5mL) and THF (5mL). The mixture was stirred for 16hrs, concentrated under reduced pressure. Water (10mL) was added to the residue, 1M HCl aq. solution was added to adjust pH=3, solid was precipitated and filtered out. The solid was washed with water (5mL), dried under vacuum to give white solid **40** (40mg, yield 50%). LC-MS (ESI): m/z = 393 [M+H]⁺.
¹H-NMR (400MHz, DMSO-d6) δ: 12.49 (s, br. 1H), 7.73(d, J=8.0 Hz, 1H), 7.56 (d, J=8.0 Hz, 1H), 7.45 (s, 1H), 7.41 (t, J=8.0 Hz, 2H), 6.45 (d, J=10.4 Hz, 1H), 4.54 (s, 2H), 3.16 (s, 3H), 1.20 (s, 6H) ppm.

### Embodiment 41

### 3-[4-(4-Cyanonaphthalen-1-yl)thieno[2,3-c]pyridin-2-yl]-3-hydroxyl butyric acid (Compound 41)

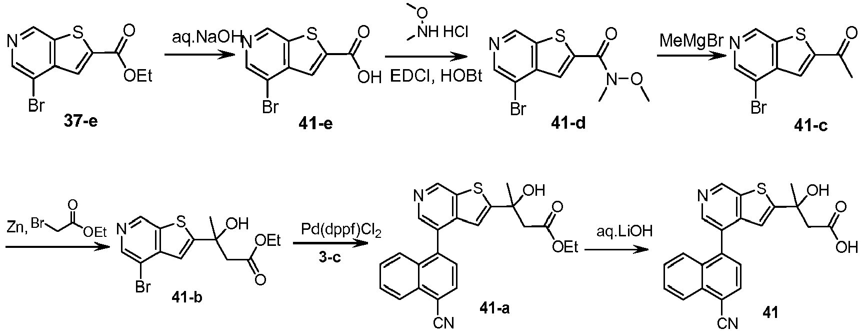

### Synthesis of compound 41-e

At room temperature, 7.0M NaOH aq. solution (2mL) was added to a solution of compound **37-e** (1.0g, 3.5mmol) in methanol (4mL) and THF (10mL). The mixture was stirred for 2hrs, concentrated under reduced pressure. Water (30mL) was added to the residue, 1M citric acid aq. solution was added to adjust pH=3, solid was precipitated and filtered out. The solid was washed with water (5mL), dried under vacuum to give compound **41-e** (740mg, yield 82%).

### Synthesis of compound 41-d

At room temperature, N,O-dimethylhydroxylamine hydrochloride (546mg, 5.6mmol), 1-(3-dimethylaminopropyl)-3-ethylcarbodiimide hydrochloride (1.08g, 5.6mmol), 1-hydroxybenzotriazole (378mg, 2.8mmol) and triethyl amine (1.13g, 11.2mmol) were added to a solution of compound **41-e** (720mg, 2.8mmol) in DMF (10mL) and DCM (30mL). The mixture was stirred for 24hrs, concentrated under reduced pressure. The residue was purified by silica column chromatography (PE:EA = 3:1) to give compound **41-d** (740mg, yield 82%). LC-MS (ESI): m/z = 301 [M+H]⁺.

### Synthesis of compound 41-c

At -78°C, 3M methyl magnesium bromide in ether (1mL, 3mmol) was added to a solution of compound **41-d** (604mg, 2mmol) in anhydrous THF (20mL). The mixture was slowly warmed to room temperature and further stirred for 20mins, followed by adding saturated NH₄Cl aq. solution (5mL), water (20mL) and EA (30mL) in turn. The organic phase was seperated, the aqueous phase was extracted with EA (20mL×2). The organic phases were combined, concentrated under reduced pressure. The residue was purified by silica column chromatography (PE:EA=8:1) to give compound **41-c** (740mg, yield 82%). LC-MS (ESI): m/z = 256 [M+H]⁺.

### Synthesis of compound 41-b

Under N₂ atmosphere, Zn powder (65mg, 1mmol) was added to a solution of compound **41-c** (257mg, 1mmol) and ethyl bromoacetate (217mg, 1.3mmol) in anhydrous THF (10mL). The mixture was heated to 50°C and further stirred for 16hrs, cooled to room temperature, followed by adding saturated NH₄Cl aq. solution (5mL) and water (20mL) in turn. The mixture was extracted with EA (30mL×3). The organic phases were combined, dried over anhydrous sodium sulfate, filtered, and concentrated under reduced pressure. The residue was purified by silica column chromatography (PE:EA=4:1) to give compound **41-b** (281mg, yield 82%). LC-MS (ESI): m/z = 344 [M+H]⁺.

### Synthesis of compound 41-a

Under N₂ atmosphere, compound **41-b** (137mg, 1mmol), compound **3-c** (140mg, 0.5mmol) and sodium carbonate (106mg, 1mmol) were suspended in ethylene glycol dimethyl ether (15mL) and water (1mL), [1,1'-bis(diphenylphosphine)ferrocene] palladium dichloride (50mg, 0.06mmol) was added. The mixture was stirred at 75°C for 16hrs, cooled to room temperature, concentrated under reduced pressure. The residue was purified by silica column chromatography (PE:EA = 3:1) to give compound **41-a** (160mg, yield 96%). LC-MS (ESI): m/z = 417 [M+H]⁺.

### Synthesis of compound 41

At room temperature, 1.0M LiOH aq. solution (5mL) was added to a solution of compound **41-a** (42mg, 0.1mmol) in methanol (5mL) and THF (5mL). The mixture was stirred for 16hrs, concentrated under reduced pressure. Water (10mL) was added to the residue, 1M citric acid aq. solution was added to adjust pH=3, solid was precipitated and filtered out. The solid was washed with water (5mL), dried under vacuum to give white solid **41** (30mg, yield 77%). LC-MS (ESI): m/z = 389 [M+H]⁺.
¹H-NMR (400MHz, DMSO-d6) δ: 12.08 (s, br. 1H), 9.31 (s, 1H), 8.44 (s, 1H), 8.32 (d, J=7.2 Hz, 1H), 8.27 (d, J=8.0 Hz, 1H), 7.87 (t, J=8.0 Hz, 1H), 7.73 (t, J=7.2 Hz, 1H), 7.62 -7.67 (m, 1H), 7.59 (d, J=8.0 Hz, 1H), 6.79 (d, J=10.0 Hz, 1H), 2.65-2.76 (m, 2H), 1.56 (d, J=6.0 Hz, 3H) ppm.

### Embodiment 42

### 3-[4-(4-Cyanonaphthalen-1-yl)thieno[2,3-c]pyridin-2-yl] butyric acid (Compound 42)

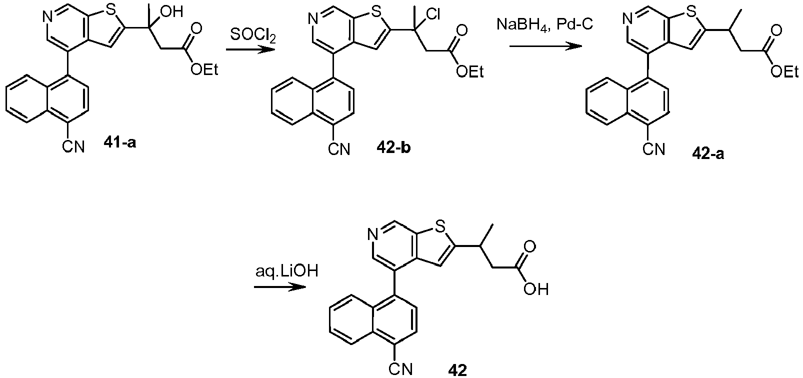

### Synthesis of compound 42-b

At 20°C, thionyl chloride (2mL) was added to a solution of compound **41-a** (160mg, 0.4mmol) in DCM (10mL). The mixture was stirred for 16hrs, concentrated under reduced pressure to give compound **42-b.** The product was used directly for the next step without further purification.

### Synthesis of compound 42-a

At 0°C, NaBH₄ (114mg, 3mmol) was added to a solution of compound **42-b,** 10% Pd-C (30mg) and ethanol (10mL) in portions. The mixture was warmed to room temperature and stirred for 16hrs, filtered through celite. Water (10mL) was added to the filtrate, the mixture was extracted with DCM (10mL×3). The organic phases were combined, dried over anhydrous sodium sulfate, filtered, and concentrated under reduced pressure. The residue was purified by silica column chromatography (PE:EA = 10:1) to give compound **42-a** (62mg, yield 42%). LC-MS (ESI): m/z = 401 [M+H]⁺.

### Synthesis of compound 42

At room temperature, 1M LiOH aq. solution (5mL) was added to a solution of compound **42-a** (40mg, 0.1mmol) in methanol (5mL) and THF (5mL). The mixture was stirred for 16hrs, and concentrated under reduced pressure. Water (10mL) was added to the residue, 1M citric acid aq. solution was added to adjust pH=3, solid was precipitated and filtered out. The solid was washed with water (5mL), dried under vacuum to give white solid **42** (20mg, yield 53%). LC-MS (ESI): m/z = 373 [M+H]⁺.
¹H-NMR (400MHz, CDCl₃) δ: 9.12 (s, 1H), 8.43 (s, 1H), 8.36 (d, J=8.8 Hz, 1H), 8.01 (t, J=8.0 Hz, 1H), 7.69-7.74 (m, 1H), 7.61 (d, J=8.8 Hz, 1H), 7.47 -7.55 (m, 2H), 6.67 (d, J=4.4 Hz, 1H), 3.59-3.64 (m, 1H), 2.58-2.74 (m, 2H), 1.24 (d, J=9.0 Hz, 3H) ppm.

### Embodiment 43

### Compound 43A

### Compound 43B

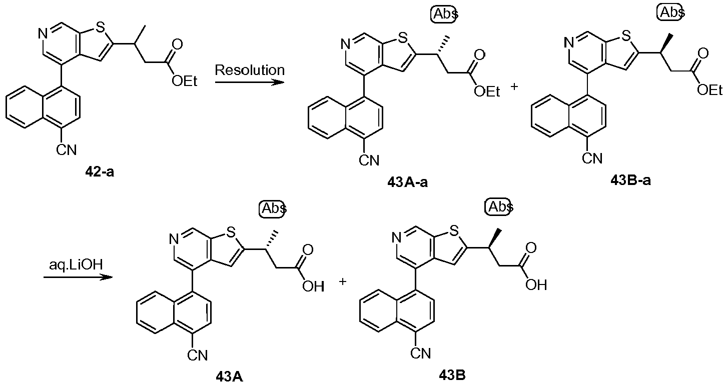

### Synthesis of compound 43A

Compound **42-a** (170mg) underwent enantiomeric chromatographic column (process II, mobile phase: n-Hexane (0.1% DEA): EtOH (0.1% DEA) = 80:20), compound **43A-a** (59mg) (Tᵣ = 18.0min) was eluted firstly and compound **43B-a** (46mg) (Tᵣ = 20.0min) was eluted later, the absolute configuration of **43A-a** and **43B-a** remains unknown. At room temperature, 1M LiOH aq. solution (2.5mL) was added to a solution of **43A-a** (59mg, 0.14mmol) in methanol (5mL). The mixture was stirred for 4hrs, concentrated under reduced pressure to remove the solvent. Water (10mL) was added to the residue, 1M citric acid aq. solution was added to adjust pH=6, solid was precipitated and filtered out. The solid was washed with water (5mL), dried under vacuum to give white solid **43A** (38mg, yield 69%). LC-MS (ESI): m/z = 373 [M+H]⁺.
¹H-NMR (400MHz, DMSO-d6) δ: 12.20 (s, 1H), 9.31 (s, 1H), 8.43 (s, 1H), 8.45 (s, 1H), 8.30 (dd, J=20.3 Hz, 7.9 Hz, 1H), 7.87 (t, J=7.4 Hz, 1H), 7.81 -7.52 (m, 3H), 6.67 (d, J=5.7 Hz, 1H), 3.50 (dd, J=13.4 Hz, 6.4 Hz, 1H), 2.57 (dd, J=9.7 Hz, 6.30 Hz, 1H), 1.28 (d, J=9.0 Hz, 3H) ppm.

### Synthesis of compound 43B

At room temperature, 1M LiOH aq. solution (2.5mL) was added to a solution of **43B-a** (46mg, 0.11mmol) in methanol (5mL). The mixture was stirred for 4hrs, concentrated under reduced pressure to remove the solvent. Water (10mL) was added to the residue, 1M citric acid aq. solution was added to adjust pH=6, solid was precipitated and filtered out. The solid was washed with water (5mL), dried under vacuum to give white solid **43B** (25mg, yield 58%). LC-MS (ESI): m/z = 373 [M+H]⁺.
¹H-NMR (400MHz, CD₃OD) δ: 9.19 (s, 1H), 8.47-8.28 (m, 2H), 8.18 (d, J=7.4 Hz, 1H), 7.93-7.78 (m, 1H), 7.70 (d, J=7.4 Hz, 1H), 7.63 (t, J=3.7 Hz, 1H), 6.75 (d, J=8.6 Hz, 1H), 3.61 (dd, J=13.6 Hz, 6.9 Hz, 1H), 2.73-2.56 (m, 2H), 1.40 (d, J=9.0 Hz, 3H) ppm.

### Embodiment 44

### Compound 44A

### Compound 44B

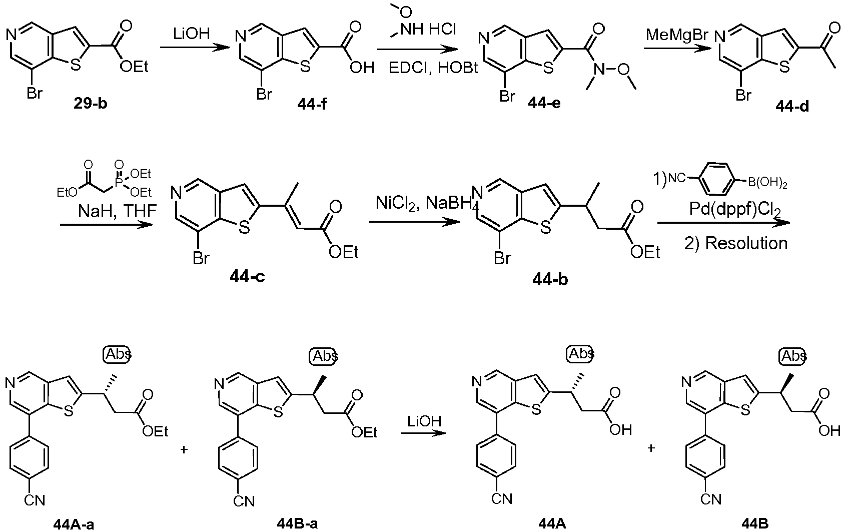

### Synthesis of compound 44-f

At room temperature, LiOH (1.68g, 40mmol) was added to a solution of compound **29-b** (5.7g, 20mmol) in methanol (10mL), THF (40mL) and water (10mL). The mixture was stirred for 1h, followed by adding 2M HCl (20mL) and water (20mL), solid was precipitated and filtered out. The solid was washed with water (50mL), dried under vacuum to give compound **44-f** (4.5g, yield 100%). LC-MS (ESI): m/z = 258 [M+H]⁺.

### Synthesis of compound 44-e

At room temperature, N,O-dimethylhydroxylamine hydrochloride (1.6g, 3mmol), 1-(3-dimethylaminopropyl)-3-ethylcarbodiimide hydrochloride (5.6g, 3mmol), 1-hydroxybenzotriazole (4.04g, 3mmol) and diisopropylethylamine (3.9g, 3mmol) were added to a solution of compound **44-f** (4.9g, 3mmol) in DCM (100mL). The mixture was stirred for 8hrs, followed by adding 2M HCl (50mL) and water (20mL), being extracted with DCM (80mL×3). The organic phases were combined, dried over anhydrous magnesium sulfate, filtered and concentrated under reduced pressure. The residue was purified by silica column chromatography (PE:EA = 1:1) to give compound **44-e** (6g, yield 100%). LC-MS (ESI): m/z = 301 [M+H]⁺.

### Synthesis of compound 44-d

At -78 °C, a solution of 1.5M methyl magnesium bromide in ether (20mL, 30mmol) was added dropwise to a solution of compound **44-e** (6.0g, 20mmol) in anhydrous THF (100mL). The mixture was slowly warmed to room temperature and further stirred for 20mins, saturated NH₄Cl aq. solution (30mL) was added, the mixture was extracted with EA (30mL×3). The organic phases were combined, concentrated under reduced pressure. The residue was purified by silica column chromatography (PE:EA = 2:1-1:1) to give compound **44-d** (4.8g, yield 94%). LC-MS (ESI): m/z = 256 [M+H]⁺.

### Synthesis of compound 44-c

At 0°C, triethyl phosphonoacetate (5.6mL, 20mmol) and sodium hydride (1.6g, 20mmol) were added to a solution of compound **44-d** (4.8g, 18.9mmol) in THF (100mL) respectively. The mixture was stirred for 1h, warmed to room temperature, followed by adding NH₄Cl aq. solution (100mL), being extracted with EA (100mL×3). The organic phases were combined, washed in turn with water (100mL×3) and saturated brine (100mL), dried over anhydrous sodium sulfate, filtered and concentrated under reduced pressure. The residue was purified by silica column chromatography (PE:EA = 5:1) to give light yellow solid **44-c** (5.2g, yield 89%). LC-MS (ESI): m/z =326 [M+H]⁺.

### Synthesis of compound 44-b

At 0°C, NaBH₄ (0.38g, 10mmol) was added into a solution of compound **44-c** (5.2g, 16mmol) and NiCl₂ (1.3g, 10mmol) in methanol (50mL). The mixture was stirred for 3hrs, warmed to room temperature, followed by adding saturated NH₄Cl aqueous solution (100mL), being extracted with EA (10mL×3). The organic phases were combined, washed in turn with water (50mL×3) and saturated brine (50mL), dried over anhydride sodium sulfate, filtered, and evaporated under reduced pressure. The residue was purified with silica column chromatography (PE:EA = 4:1) to give light yellow solid **44-b** (2.24g, yield 43%). LC-MS (ESI): m/z =328 [M+H]⁺.

### Synthesis of compound 44A-a and 44B-a

Under N₂ atmosphere, compound **44-b** (327mg, 1mmol), 4-cyanophenyl boronic acid (150mg, 1mmol) and sodium carbonate (212mg, 2mmol) were suspended in dioxane (8mL) and water (2mL), [1,1'-bis(diphenylphosphine)ferrocene]palladium dichloride (60mg, 0.1mmol) was added. The mixture was stirred at 80°C for 3hrs, and then cooled to room temperature, filtered through celite, the filtrate cake was washed with EA (50mL). The filtrate was in turn washed with water (20mL×3) and saturated brine (10mL), dried over anhydrous magnesium sulfate, filtered, concentrated under reduced pressure. The residue was purified by silica column chromatography (PE:EA =1:1) to give racemic compound, followed by separating by enantiomeric chromatographic column (process I, mobile phase: Hexane:EtOH:DEA =70:30:0.1), compound **44A-a** (80mg, yield 22.8%; LC-MS (ESI): m/z = 351 [M+H]⁺) (Tᵣ = 6.0min) was diluted firstly and compound **44B-a** (90mg, yield 25.6%; LC-MS (ESI): m/z = 351 [M+H]⁺) (Tᵣ = 7.0 min) was diluted later. Absolute configuration of **44A-a** and **44B-a** remains unknown.

### Synthesis of compound 44A

At room temperature, LiOH (42mg, 1mmol) was added to a solution of compound **44A-a** (70mg, 0.2mmol) in methanol (1mL), THF (2mL) and water (1mL). The mixture was stirred for 1h, followed by adding 1M HCl aq. solution (1mL) and water (2mL), solid was precipitated and filtered out. The solid was washed with water (5mL), dried under vacuum to give compound **44A** (53mg, yield 82%). LC-MS (ESI): m/z = 323 [M+H]⁺.
¹H-NMR (400MHz, DMSO-d6) δ: 12.30 (s, 1H), 9.07 (s, 1H), 8.52 (s, 1H), 8.06 (d, J=8.0 Hz, 2H), 7.97 (d, J=8.0 Hz, 2H), 7.51 (s, 1H), 3.58 (m, 1H), 2.66 (t, J=8.0 Hz, 2H), 1.37 (d, J=8.0 Hz, 3H) ppm.

### Synthesis of compound 44B

At room temperature, LiOH (42mg, 1mmol) was added to a solution of compound **44B-a** (70mg, 0.2mmol) in methanol (1mL), THF (2mL) and water (1mL). The mixture was stirred for 1h, followed by adding 1M HCl aq. solution (1mL) and water (2mL), solid was precipitated and filtered out. The solid was washed with water (5mL), dried under vacuum to give compound **44B** (39mg, yield 60.6%). LC-MS (ESI): m/z = 323 [M+H]⁺.
¹H-NMR (400MHz, DMSO-d6) δ: 12.30 (s, 1H), 9.07 (s, 1H), 8.52 (s, 1H), 8.06 (d, J=8.0 Hz, 2H), 7.97 (d, J=8.0 Hz, 2H), 7.51 (s, 1H), 3.58 (m, 1H), 2.66 (t, J=8.0 Hz, 2H), 1.37 (d, J=8.0 Hz, 3H) ppm.

### Embodiment 45

### Compound 45A

### Compound 45B

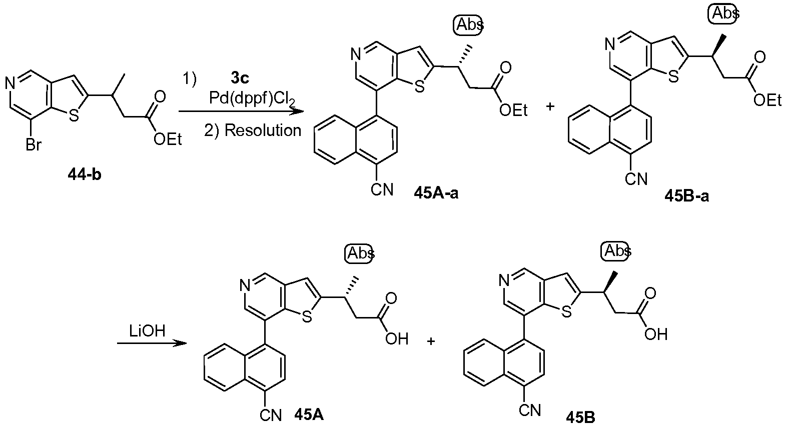

### Synthesis of compound 45A-a and 45B-a

Under N₂ atmosphere, compound **44-b** (800mg, 2.5mmol), compound **3-c** (750mg, 2.5mmol) and sodium carbonate (510mg, 5mmol) were suspended in dioxane (8mL) and water (2mL), [1,1'-bis(diphenylphosphine)ferrocene]palladium dichloride (140mg, 0.25mmol) was added. The mixture was stirred for 3hrs at 80°C, cooled to room temperature, filtered through celite, the filtrate cake was washed with EA (50mL). The filtrate was washed in turn with water (20mL×3) and saturated brine (10mL), dried over anhydrous magnesium sulfate, filtered, and concentrated under reduced pressure. The residue was purified by silica column chromatography (PE:EA=1:1) to give racemic compound, followed by separating by enantiomeric chromatographic column (process II, mobile phase: CO₂:Methanol (0.1% NH₄OH) = 65:35), compound **45A-a** was diluted firstly (260 mg, yield 26%; LC-MS (ESI): m/z = 401 [M+H]⁺) (Tᵣ = 8.5min), and compound **45B-a** (230mg, yield 23%; LC-MS (ESI): m/z = 401 [M+H]⁺) (Tᵣ = 10.5min) was diluted later. Absolute configuration of **45A-a** and **45B-a** remains unknown.

### Synthesis of compound 45A

At room temperature, LiOH (42mg, 1mmol) was added to a solution of compound **45A-a** (80mg, 0.2mmol) in methanol (1mL), THF (2mL) and water (1mL). The mixture was stirred for 1h, followed by adding 1M HCl aq. solution (1mL) and water (2mL), solid was precipitated and filtered out. The solid was washed with water (5mL), dried under vacuum to give compound **45A** (61mg, yield 82%). [α]²⁵D = +26.248 (c=1.1018 MeOH), LC-MS (ESI): m/z = 373 [M+H]⁺.
¹H-NMR (400MHz, DMSO-d6) δ: 12.27 (s, 1H), 9.17 (s, 1H), 8.43 (s, 1H), 8.33 (d, J=7.2 Hz, 1H), 8.27 (d, J=8.4 Hz, 1H), 7.90 (dd, J=7.2 Hz, 5.6 Hz, 1H), 7.83 (d, J=7.2 Hz, 1H), 7.68 (dd, J=6.8 Hz, 6.0 Hz, 1H), 7.64 (d, J=8.4 Hz, 1H), 7.52 (s, 1H), 3.50 (m, 1H), 2.60 (m, 2H), 1.31 (dd, J=7.6 Hz, 6.8 Hz, 3H) ppm.

### Synthesis of compound 45B

At room temperature, LiOH (42mg, 1mmol) was added to a solution of compound **45B-a** (80mg, 0.2mmol) in methanol (1mL), THF (2mL) and water (1mL). The mixture was stirred for 1h, followed by adding 1M HCl aq. solution (1mL) and water (2mL), solid was precipitated and filtered out. The solid was washed with water (5mL), dried under vacuum to give compound **45B** (56mg, yield 75%). [α]²⁵D = -25.594 (c=1.002 MeOH), LC-MS (ESI): m/z = 373 [M+H]⁺.
¹H-NMR (400MHz, DMSO-d6) δ: 12.27 (s, 1H), 9.17 (s, 1H), 8.43 (s, 1H), 8.33 (d, J=7.2 Hz, 1H), 8.27 (d, J=8.4 Hz, 1H), 7.90 (dd, J=7.2 Hz, 5.6 Hz, 1H), 7.83 (d, J=7.2 Hz, 1H), 7.68 (dd, J=6.8 Hz, 6.0 Hz, 1H), 7.64 (d, J=8.4 Hz, 1H), 7.52 (s, 1H), 3.50 (m, 1H), 2.60 (m, 2H), 1.31 (dd, J=7.6 Hz, 6.8 Hz, 3H) ppm.

### Embodiment 46

### Compound 46A

### Compound 46B

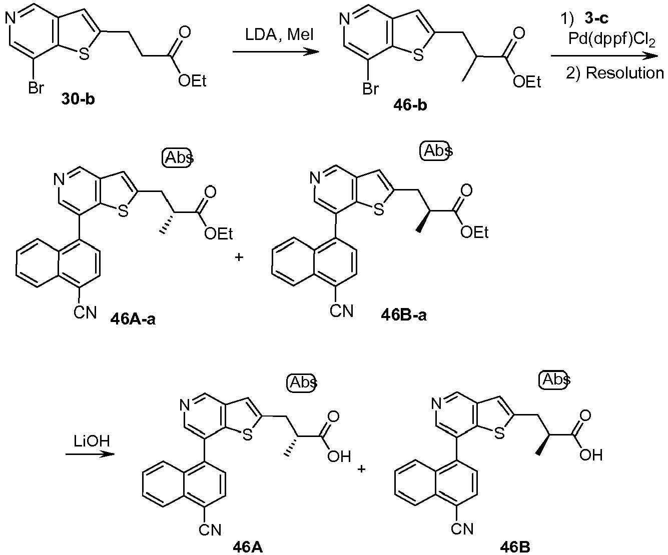

### Synthesis of compound 46-b

Under N2 atmosphere, at -78 °C, 2.5M *n*-butyl lithium in n-hexane (2.0mL, 5mmol) was slowly added to a solution of diisopropylamine (505mg, 5mmol) in anhydrous THF (10mL) dropwise. The mixture was stirred for 15mins, followed by adding a solution of compound **30-b** (630mg, 2mmol) in anhydrous THF (10mL) dropwise, the mixture was stirred for 2hrs, followed by adding CH₃I (720mg, 5mmol) and the mixture was further stirred for 3hrs. The mixture was slowly warmed to room temperature, followed by adding saturated NH₄Cl aq. solution (30mL), being extracted with EA (30mL×3). The organic phases were combined, washed in turn with water (10mL) and saturated brine (10mL), dried over anhydrous sodium sulfate, filtered, and concentrated under reduced pressure. The residue was purified by silica preparative plate chromatography (PE:EA = 2:1-1:1) to give compound **46-b** (170mg, yield 26%). LC-MS (ESI): m/z = 328 [M+H]⁺.

### Synthesis of compound 46A-a and 46B-a

Under N₂ atmosphere, compound **46-b** (170mg, 0.52mmol), compound **3-c** (145mg, 0.52mmol) and sodium carbonate (120mg, 1.13mmol) were suspended in dioxane (4mL) and water (1mL), [1,1'-bis(diphenylphosphine)ferrocene]palladium dichloride (43mg, 0.05mmol) was added. The mixture was stirred at 80°C for 3hrs, cooled to room temperature, concentrated under reduced pressure. The residue was filtered through celite, the filtrate cake was washed with EA (30mL). The filtrate was washed in turn with water (20mL×3) and saturated brine (10mL), dried over anhydrous magnesium sulfate, filtered, concentrated under reduced pressure. The residue was purified by silica column chromatography (PE:EA = 1:1) to give racemic compound, followed by separating by enantiomeric chromatographic column (process I, mobile phase: Hexane : EtOH:DEA = 80:20:0.1), compound **46A-a** was eluted firstly (66mg, yield 31%; LC-MS (ESI): m/z = 401 [M+H]⁺) (Tᵣ = 14.0min) and compound **46B-a** was eluted later (61mg, yield 29%; LC-MS (ESI): m/z = 401 [M+H]⁺) (Tᵣ = 18.0min). Absolute configuration of **46A-a** and **46B-a** remains unknown.

### Synthesis of compound 46A

At room temperature, LiOH (42mg, 1mmol) was added to a solution of compound **46A-a** (60mg, 0.15mmol) in methanol (1mL), THF (4mL) and water (1mL). The mixture was stirred for 1h, followed by adding 1M HCl aq. solution (1mL) and water (10mL), solid was precipitated and filtered out. The solid was washed with water (5mL), dried under vacuum to give compound **46A** (26mg, yield 46%). LC-MS (ESI): m/z = 373 [M+H]⁺.
¹H-NMR (400MHz, DMSO-d₆) δ: 12.31 (s, 1H), 9.17 (s, 1H), 8.44 (s, 1H), 8.33 (d, J=8.0 Hz, 1H), 8.27 (d, J=8.0 Hz, 1H), 7.88 (m, 2H), 7.66 (m, 2H), 7.49 (s, 1H), 3.16 (m, 1H), 3.02 (m, 1H), 2.68 (m, 1H), 1.23 (d, J=6.8 Hz, 1H) ppm.

### Synthesis of compound 46B

At room temperature, LiOH (42mg, 1mmol) was added to a solution of compound **46B-a** (60mg, 0.15mmol) in methanol (1mL), THF (4mL) and water (1mL). The mixture was stirred for 1h, followed by adding 1M HCl aq. solution (1mL) and water (10mL), solid was precipitated and filtered out. The solid was washed with water (5mL), dried under vacuum to give compound **46B** (26mg, yield 46%). LC-MS (ESI): m/z = 373 [M+H]⁺.
¹H-NMR (400MHz, DMSO-d₆) δ: 12.31 (s, 1H), 9.17 (s, 1H), 8.44 (s, 1H), 8.33 (d, J=8.0 Hz, 1H), 8.27 (d, J=8.0 Hz, 1H), 7.88 (m, 2H), 7.66 (m, 2H), 7.49 (s, 1H), 3.16 (m, 1H), 3.02 (m, 1H), 2.68 (m, 1H), 1.23 (d, J=6.8 Hz, 1H) ppm.

### Embodiment 47

### 3-[4-(4-Cyanonaphthalen-1-yl)thieno[2,3-c]pyridin-2-yl]-2,2-dimethyl propionic acid (Compound 47)

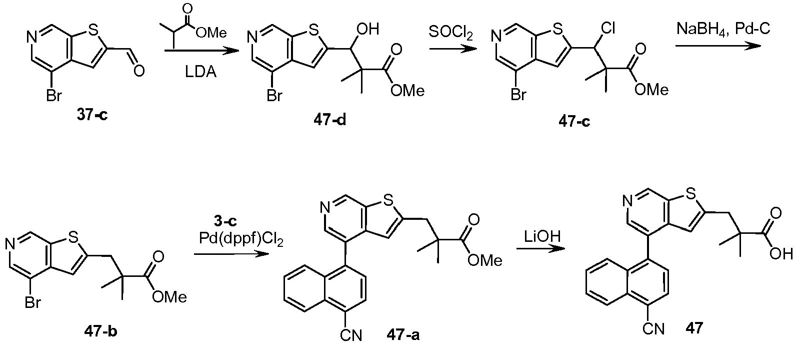

### Synthesis of compound 47-d

At -78°C, 1M Lithium diisopropylamide in THF (3mL, 3mmol) was slowly added to a solution of methyl isobutyrate (306mg, 3mmol) in anhydrous THF (4mL). The mixture was stirred for 1h, followed by adding compound **37-c** (242mg, 1mmol), the mixture was further stirred for 1h. The mixture was slowly warmed to room temperature, followed by adding saturated NaHCO₃ aq. solution (20mL), being extracted with EA (20mL×3). The organic phases were combined, dried over anhydrous sodium sulfate, filtered, and concentrated under reduced pressure to give compound **47-d** (425mg). The product was used directly for the next step without further purification. LC-MS (ESI): m/z = 344 [M+H]⁺.

### Synthesis of compound 47-c

Thionyl chloride (6mL) was added to a solution of compound **47-d** (425mg) in DCM (10mL). The mixture was heated to 40°C, stirred for 16hrs and concentrated under reduced pressure to remove the solvent. Water (15mL) was added to the residue, the mixture was extracted with EA (15mL×3). The organic phases were combined, dried over anhydrous sodium sulfate, filtered and concentrated under reduced pressure to give compound **47-c** (487mg). The product was used directly for the next step without further purification. LC-MS (ESI): m/z = 364 [M+H]⁺.

### Synthesis of compound 47-b

At room temperature, NaBH₄ (204mg, 5.37mmol) was added to a mixture of compound **47-c** (487mg), 10% Pd-C (50mg) and ethanol (20mL) in portions, the mixture was stirred for 16hrs. The mixture was filtered through celite, the filtrate cake was washed with ethanol (10mL×3). The filtrate was concentrated under reduced pressure. The residue was purified by silica column chromatography (PE:EA = 5:1) to give compound **47-b** (135 mg, yield 31%). LC-MS (ESI): m/z = 328 [M+H]⁺.

### Synthesis of compound 47-a

Under N₂ atmosphere, compound **47-b** (135mg, 0.41mmol), compound **3-c** (121mg, 0.43mmol) and sodium sulfate (106mg, 1mmol) were suspended in dioxane (8mL) and water (1mL), [1,1'-bis(diphenylphosphine)ferrocene]palladium dichloride (30mg, 0.04mmol) was added. The mixture was stirred at 90°C for 16hrs, cooled to room temperature, and concentrated under reduced pressure. The residue was purified by silica column chromatography (PE:EA=3:1) to give compound **47-a** (130mg, yield 79%). LC-MS (ESI): m/z = 401 [M+H]⁺.

### Synthesis of compound 47

At room temperature, LiOH (55mg, 1.3mmol) was added to a solution of compound **47-a** (130mg, 0.32mmol) in methanol (1mL), THF (5mL) and water (1mL). The mixture was stirred for 1h, followed by adding 1M HCl aq. solution to adjust pH=5-6, being extracted with EA (15mL×3). The organic phases were combined, dried over anhydrous sodium sulfate, filtered, and concentrated under reduced pressure. The residue was prepared by HPLC (mobile phase: 10mM NH₄HCO₃ aq. solution: acetonitrile =35%-45%) to give white solid **47** (13mg, yield 10.5%). LC-MS (ESI): m/z = 387 [M+H]⁺.
¹H-NMR (400MHz, CD₃OD) δ: 9.22 (s, 1H), 8.39 (s, 1H), 8.22 (d, J=8.1 Hz, 1H), 8.10-8.01 (m, 1H), 7.71 (dd, J=7.9 Hz, 3.7 Hz, 1H), 7.60 (d, J=7.1 Hz, 1H), 7.50 (s, 2H), 6.68 (s, 1H), 3.13-3.02 (m, 2H), 1.19 (s, 3H), 1.18 (s, 3H) ppm.

### Embodiment 48

### 3-[4-(4-Cyanophenyl)thieno[2,3-c]pyridin-2-yl]-2,2-dimethyl propionic acid (Compound 48)

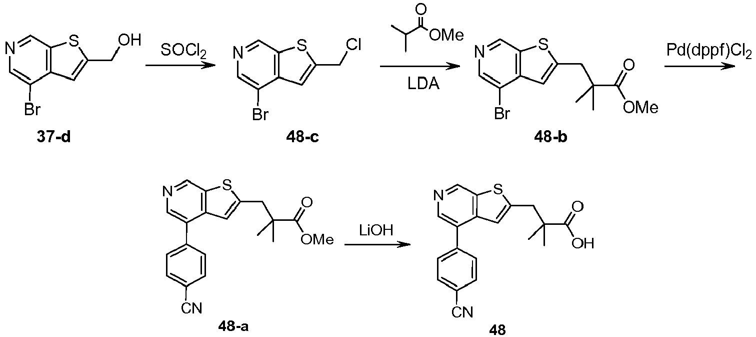

### Synthesis of compound 48-c

Thionyl chloride (5mL) was added to a solution of compound **37-d** (200mg, 0.82mmol) in DCM (10mL). The mixture was heated to 30°C, stirred for 16hrs, and concentrated under reduced pressure to give compound **48-c** (236mg). The product was used directly for the next step without further purification. LC-MS (ESI): m/z = 264 [M+H]⁺.

### Synthesis of compound 48-b

Under N₂ atmosphere, at -78°C, a solution of 2.5M n-butyl lithium in n-hexane (1.37mL, 3.43mmol) was slowly added to a solution of diisopropylamine (347mg, 3.43mmol) in anhydrous THF (10mL). The mixture was warmed to 0°C and further stirred for 1h, then cooled again to -78°C, methyl isobutyrate (350mg, 3.43mmol) was added, the mixture was stirred for 1h, followed by adding compound **48-c** (180mg, 0.69mmol) and further stirred for 1h. The mixture was slowly warmed to room temperature, stirred for 2hrs, followed by adding saturated NH₄Cl aq. solution (20mL), being extracted with EA (50mL×2). The organic phases were combined, dried over anhydrous sodium sulfate, filtered, and concentrated under reduced pressure. The residue was purified by silica column chromatography (PE:EA = 3:1) to give compound **48-b** (280mg, yield 97%). LC-MS (ESI): m/z = 329 [M+H]⁺.

### Synthesis of compound 48-a

Under N₂ atmosphere, compound **48-b** (280mg, 0.85mmol), 4-cyanophenylboronic acid (138mg, 0.94mmol) and sodium sulfate (180mg, 1.7mmol) were suspended in dioxane (15mL) and water (2mL), [1,1'-bis(diphenylphosphine)ferrocene]palladium dichloride (62mg, 0.08mmol) was added. The mixture was added at 90°C for 16hrs, cooled to room temperature, concentrated under reduced pressure. The residue was purified by silica column chromatography (PE:EA = 3:1) to give compound **48-a** (135mg, yield 45%). LC-MS (ESI): m/z = 351 [M+H]⁺.

### Synthesis of compound 48

At room temperature, LiOH (65mg, 1.54mmol) was added to a solution of compound **48-a** (135mg, 0.38mmol) in methanol (1mL), THF (5mL) and water (1mL). The mixture was stirred for 6hrs, 1M HCl aq. solution was added to adjust pH=5-6, and concentrated under reduced pressure. The residue was adjusted to pH=7-8 with 2M NaOH aq. solution, and then extracted with EA (10mL) to remove the impurities. The aqueous phase was adjusted to pH=5-6 with 1M HCl aq. solution, extracted with EA (15mL×2). The organic phases were combined, dried over anhydrous sodium sulfate, filtered, and concentrated under reduced pressure to give yellow solid **48** (75 mg, yield 58%). LC-MS (ESI): m/z = 337 [M+H]⁺.
¹H-NMR (400MHz, DMSO-d6) δ: 12.52 (s, 1H), 9.21 (s, 1H), 8.48 (s, 1H), 8.03 (d, J=8.2 Hz, 2H), 7.84 (d, J=8.2 Hz, 2H), 7.30 (s, 1H), 3.21 (s, 2H), 1.16 (s, 6H) ppm.

### Embodiment 49

### 2-[4-(4-Cyanonaphthalen-1-yl)isoquinolin-6-yl]-2-methyl propionic acid (Compound 49)

### Synthetic route

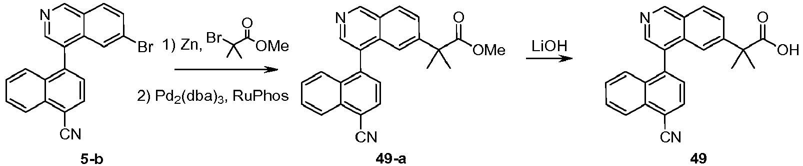

### Synthesis of compound 49-a

Under N₂ atmosphere, trimethylchlorosilane (11mg, 0.1mmol) was added dropwise to a solution of Zn powder (130mg, 2mmol) and THF (4mL). The mixture was stirred for 15mins at room temperature, heated to 40°C, followed by adding a solution of methyl 2-bromoisobutyrate (181mg, 1mmol) in THF (2mL). The mixture was further stirred at 40°C for 30mins, added to a mixture of compound **5-b** (90mg, 0.25mmol), LiCl (11mg, 0.25mmol), tris(dibenzylidene indene acetone)dipalladium (23mg, 0.025mmol), 2-dicyclohexylphospho-2',6'-diisopropoxy-1,1'-biphenyl (12mg, 0.025mmol) and THF (4mL). The mixture was heated to 80°C and further stirred for 1h, and then cooled to room temperature, and concentrated under reduced pressure to remove the solvent. The residue was dissolved in DCM (50mL), washed in turn with water (20mL×3) and saturated brine (10mL), dried over anhydrous sodium sulfate, filtered, concentrated under reduced pressure. The residue was purified by silica preparative plate chromatography (DCM: methanol = 20:1) to give compound **49-a** (40mg, yield 42%). LC-MS (ESI): m/z = 381 [M+H]⁺.

### Synthesis of compound 49

At room temperature, LiOH (22mg, 0.5mmol) was added to a solution of compound **49-a** (40mg, 0.1mmol) in methanol (1mL) and THF (3mL). The mixture was stirred for 16hrs, evaporated to remove the solvent, followed by adding water (5mL), being extracted with EA (10mL×3). 1M HCl aq. solution was added to the aqueous phase to adjust pH=5-6, extracted with EA (15mL×3), the organic phases were combined, dried over anhydrous sodium sulfate, filtered and concentrated under reduced pressure. The residue was prepared by HPLC (mobile phase: water (0.05% trifluoroacetic acid): nitrile =25%-40%) to give compound **49** (15mg, yield 39%). LC-MS (ESI): m/z = 367 [M+H]⁺.
¹H-NMR (400MHz, CDCl₃) δ: 9.39 (s, 1H), 8.50 (s, 1H), 8.36 (d, J=8.4 Hz, 1H), 8.13 (d, J=8.4 Hz, 1H), 7.77 (d, J=8.4 Hz, 1H), 7.72-7.67 (m, 1H), 7.58 (d, J=7.2 Hz, 1H), 7.48-7.46 (m, 2H), 7.34 (s, 1H), 1.45 (s, 6H) ppm.

### Embodiment 50

### 2-{[4-(4-Cyanonaphthalen-1-yl)phthalazin-6-yl]thio}-2-methyl propionic acid (Compound 50)

### Synthetic route

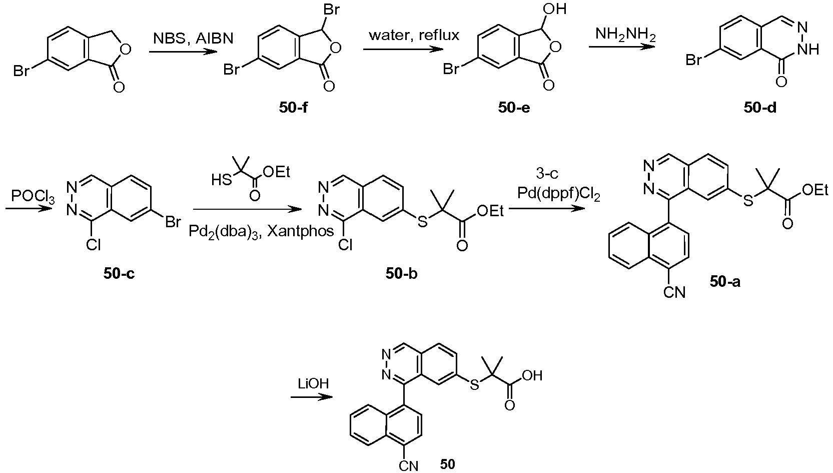

### Synthesis of compound 50-f

6-Bromo-phthalide (2.30g, 10.9mmol) was added to a solution of N-bromosuccinimide (2.1g, 11.8mmol), azobisisobutyronitrile (0.1g, 0.06mmol) in 1,2-dichloroethane (60mL). The mixture was heated to reflux for 2hrs, cooled to room temperature, and concentrated under reduced pressure. The residue was washed with water (10mL×3) to give compound **50-f.** The product was used directly for the next step without further purification.

### Synthesis of compound 50-e

A mixture of compound **50-f** and water (40mL) was heated to reflux for 2hrs, cooled to room temperature, white solid was precipitated and filtered out. Solid was washed with water (20mL×3), dried under vacuum to give compound **50-e** (1.6g, yield 64%). The product was used directly for the next step without further purification.

### Synthesis of compound 50-d

85% Hydrazine hydrate (2mL) was added to a solution of compound **50-e** (1.60g, 7mmol) in isopropyl alcohol (40mL). The mixture was heated to reflux for 2hrs, cooled to room temperature, white solid was precipitated and filtered out. The solid was washed with water (20mL×3), dried under vacuum to give compound **50-d** (1.2g, yield 76%). The product was used directly for the next step without further purification.

### Synthesis of compound 50-c

A mixture of compound **50-d** (600mg, 2.67mmol) and POCl₃ (8mL) was heated to reflux for 1.5h, cooled to room temperature and concentrated under reduced pressure. The residue was dissolved in DCM (40mL), washed in turn with saturated sodium bicarbonate (40mL) and saturated brine (10mL), dried over anhydrous sodium sulfate, filtered and concentrated under reduced pressure. The residue was purified by silica column chromatography (PE:EA = 3:1) to give compound **50-c** (500mg, yield 75%). LC-MS (ESI): m/z = 243 [M+H]⁺.

### Synthesis of compound 50-b

Under N₂ atmosphere, tris(dibenzylidene indene acetone)dipalladium (30mg, 0.03mmol) and 4,5-bis(diphenylphosphine)-9,9-dimethyloxacanthracene (38mg, 0.06mmol) were added to a solution of compound **50-c** (131mg, 0.5mmol), ethyl 2-methyl-2-mercaptopropionate (73mg, 0.5mmol) and diisopropylethylamine (193mg, 1.5mmol) in dioxane (10mL). The mixture was stirred at 100°C for 16hrs, cooled to room temperature, concentrated under reduced pressure. The residue was purified by silica column chromatography (PE:EA = 3:1) to give compound **50-b** (120mg, yield 77%). LC-MS (ESI): m/z = 311 [M+H]⁺.

### Synthesis of compound 50-a

Under N₂ atmosphere, compound **50-b** (120mg, 0.38mmol), compound **3-c** (111mg, 0. 4mmol) and sodium carbonate (170mg, 2.8mmol) were suspended in ethylene glycol dimethyl ether (10mL) and water (1mL), [1,1'-bis(diphenylphosphine)ferrocene] palladium dichloride (43mg, 0.05mmol) was added. The mixture was stirred at 80°C for 4hrs, cooled to room temperature and concentrated under reduced pressure. The residue was purified by silica column chromatography (PE:EA = 2:1) to give compound **50-a** (96mg, yield 60%). LC-MS (ESI): m/z = 428 [M+H]⁺.

### Synthesis of compound 50

At room temperature, 1M LiOH aq. solution (3.0mL) was added to a solution of compound **50-a** (86mg, 0.2mmol) in methanol (4mL) and THF (8mL). The mixture was stirred for 16hrs and concentrated under reduced pressure. The residue was dissolved in water (10mL), adjusted to pH=3 with 1M HCl aq. solution, solid was precipitated and filtered out. The solid was washed with water (5mL), dried under vacuum to give white solid **50** (60mg, yield 75%). LC-MS (ESI): m/z = 400 [M+H]⁺.
¹H-NMR (400MHz, DMSO-d6) δ: 12.77 (s, br. 1H,), 9.87 (s, 1H), 8.39 (d, J=8.0 Hz, 1H), 8.30 (d, J=8.0 Hz, 2H), 7.98-8.00 (m, 1H), 7.85-7.90 (m, 2H), 7.62 (t, J=8.0 Hz, 1H), 7.47 (d, J=8.8 Hz, 1H), 7.42 (s, 1H), 1.32 (s, 3H), 1.26 (s, 3H) ppm.

### Embodiment 51

### 3-[4-(4-Cyanophenyl)isoquinolin-6-yl]-2,2-dimethyl propionic acid (Compound 51) Synthetic route

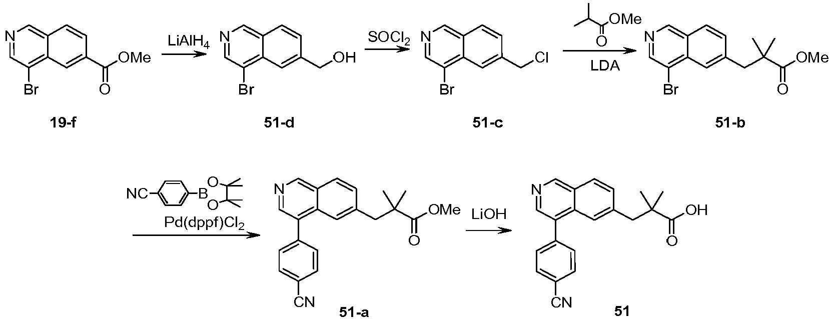

### Synthesis of compound 51-d

At 0°C, lithium aluminum hydride (214mg, 5.64mmol) was suspended in anhydrous THF (100mL), a solution of compound **19-f** (1.5g, 5.64mmol) in THF (10mL) was slowly added. The mixture was stirred for 10mins, Na₂SO₄ • 10H₂O (2.0g) was added in portions, then the mixture was warmed to room temperature and further stirred for 30mins. The mixture was filtered, the filtrate cake was washed with EA (20mL). The filtrate was concentrated under reduced pressure, the residue was purified by silica column chromatography (PE:EA = 3:1) to give compound **51-d** (600mg, yield 44%). LC-MS (ESI): m/z = 238 [M+H]⁺.

### Synthesis of compound 51-c

Thionyl chloride (1.84mL) was added to a solution of compound **51-d** (600mg, 2.52mmol) in DCM (25mL). The mixture was heated to 30°C, stirred for 16hrs and concentrated under reduced pressure to give compound **51-c** (720mg). The product was used directly for the next step without further purification. LC-MS (ESI): m/z = 256 [M+H]⁺.

### Synthesis of compound 51-b

Under N₂ atmosphere, at -78°C, a solution of 2.5M *n*-butyl lithium in n-hexane (2.05mL, 5.1mmol) was added to a solution of diisopropylamine (0.72mL, 5.1mmol) in anhydrous THF (20mL). The mixture was warmed to room temperature, stirred for 1h, cooled again to -78°C. The mixture was added to methyl isobutyrate (0.59mL, 5.1mmol), stirred for 1h, followed by adding compound **51-c** (300mg, 1.02mmol) and further stirred for 1h. The mixture was slowly warmed to room temperature, stirred for 2hrs, followed by adding saturated NH₄Cl aq. solution (20mL), being extracted with EA (50mL×2). The organic phases were combined, dried over anhydrous sodium sulfate, filtered and concentrated under reduced pressure. The residue was purified by silica column chromatography (PE:EA = 3:1) to give yellow oil **51-b** (300mg, yield 91%). LC-MS (ESI): m/z = 322 [M+H]⁺.

### Synthesis of compound 51-a

Under N₂ atmosphere, compound **51-b** (300mg, 0.93mmol), 4-cyanophenylboronic acid (215mg, 0.93mmol) and sodium carbonate (296mg, 2.79mmol) were suspended in DMF (10mL) and water (5mL), [1,1'-bis(diphenylphosphine)ferrocene] palladium dichloride (76mg, 0.09mmol) was added. The mixture was stirred at 80°C for 16hrs, cooled to room temperature, and concentrated under reduced pressure. The residue was purified by silica column chromatography (PE:EA = 3:1-2:1) to give compound **51-a** (250mg, yield 78%). LC-MS (ESI): m/z = 345 [M+H]⁺.

### Synthesis of compound 51

At room temperature, LiOH (152mg, 3.6mmol) was added to a solution of compound **51-a** (250mg, 0.72mmol) in methanol (1mL), THF (5mL) and water (2mL). The mixture was stirred for 6hrs, 1M HCl aq. solution was added to adjust pH=5-6, the mixture was concentrated under reduced pressure, extracted with EA (20mL×3). The organic phases were combined, dried over anhydrous sodium sulfate, filtered, and concentrated under reduced pressure. The residue was prepared by HPLC (mobile phase: water (0.01% NH₃+10 mm NH₄HCO₃) : nitrile = 45%-75%) to give compound **51** (33mg, yield 14%). LC-MS (ESI): m/z = 331 [M+H]⁺.
¹H-NMR (400MHz, CDCl₃) δ: 9.19 (s, 1H), 8.36 (s, 1H), 7.94 (m, 1H), 7.51-7.65 (m, 6H), 3.06 (s, 2H), 1.27 (s, 6H) ppm.

### Embodiment 52

### 3-[7-(4-Cyanonaphthalen-1-yl)thieno[3,2-c]pyridine-2-yl]-butyric acid (Compound 52)

### Synthetic route

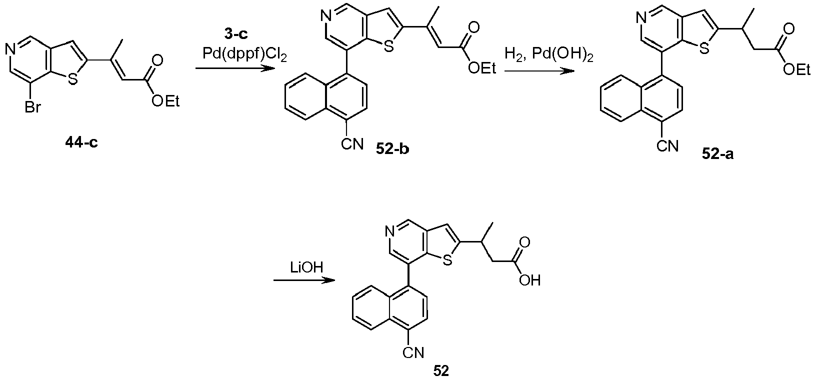

### Synthesis of compound 52-b

Under N₂ atmosphere, compound **44-c** (9.0g, 27.6mmol), compound **3-c** (15.4g, 55.2mmol) and sodium carbonate (5.85g, 55.2mmol) were suspended in dioxane (240mL) and water (40mL), [1,1'-bis(diphenylphosphine)ferrocene] palladium dichloride (1.0g, 1.38mmol) was added. The mixture was stirred at 80°C for 16hrs, cooled to room temperature, and concentrated under reduced pressure. The residue was purified by silica column chromatography (PE:EA = 10:1) to give yellow solid **52-b** (9.1g, yield 82.8%). LC-MS (ESI): m/z = 399 [M+H]⁺.

### Synthesis of compound 52-a

Under H₂ (1atm.) atmosphere, palladium hydroxide (3.0g) was added to a solution of compound **52-b** (9.1g, 22.8mmol) in THF (100mL) and methanol (280mL). The mixture was stirred for 16hrs, filtered, and concentrated under reduced pressure. The residue was purified by silica column chromatography (PE:EA = 1:1) to give yellow oil **52-a** (8.0g, yield 87.5%). LC-MS (ESI): m/z = 401 [M+H]⁺.

### Synthesis of compound 52

At room temperature, LiOH (1.51g, 36mmol) was added to a solution of compound **52-a** (8.0g, 20mmol) in methanol (15mL), THF (30mL) and water (5mL). The mixture was stirred for 8hrs, followed by adding 1M HCl aq. solution to adjust pH=5-6, solid was precipitated and filtered out. The solid was washed with water (20mL×3), dried under vacuum to give white solid **52** (6.18g, yield 83%). LC-MS (ESI): m/z = 373 [M+H]⁺.
¹H-NMR (400MHz, DMSO-d6) δ: 12.27 (s, 1H), 9.17 (s, 1H), 8.43 (s, 1H), 8.33 (d, J=7.2 Hz, 1H), 8.27 (d, J=8.4 Hz, 1H), 7.90 (dd, J=7.2 Hz, 5.6 Hz, 1H), 7.83 (d, J=7.2 Hz, 1H), 7.68 (dd, J=6.8 Hz, 6.0 Hz, 1H), 7.64 (d, J=8.4 Hz, 1H), 7.52 (s, 1H), 3.50 (m, 1H), 2.60 (m, 2H), 1.31 (dd, J=7.6 Hz, 6.8 Hz, 3H) ppm.

### Embodiment 53

### Compound 53A

### Synthetic route

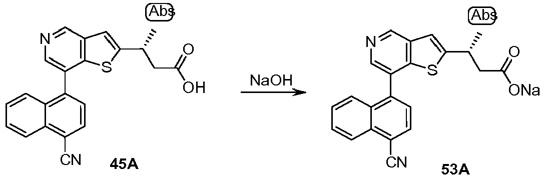

### Synthesis of compound 53A

At room temperature, NaOH (8mg, 0.02mmol) was added to a solution of compound **45A** (74mg, 0.02mmol) in water (1mL). The mixture was stirred for 2hrs, freeze-dried to give white solid **53A** (79mg, yield 100%). LC-MS (ESI): m/z = 373 [M-Na+2H]⁺.
¹H-NMR (400MHz, DMSO-d6) δ: 9.11 (s, 1H), 8.38 (s, 1H), 8.32 (d, J=7.2 Hz, 1H), 8.27 (d, J=8.0 Hz, 1H), 7.88 (t, J=6.4 Hz, 1H), 7.82 (d, J=7.2 Hz, 1H), 7.65 (m, 2H), 7.42 (s, 1H), 3.47 (m, 1H), 2.23 (m, 1H), 2.11 (m, 1H), 1.25 (dd, J=7.2 Hz, 6.8 Hz, 3H) ppm.

### Embodiment 54

### 3-[4-(4-Cyanonaphthalen-1-yl)thieno[3,2-c]pyridine-2-yl]-2,2-bis(triadecylmethyl) propionic acid (Compound 54)

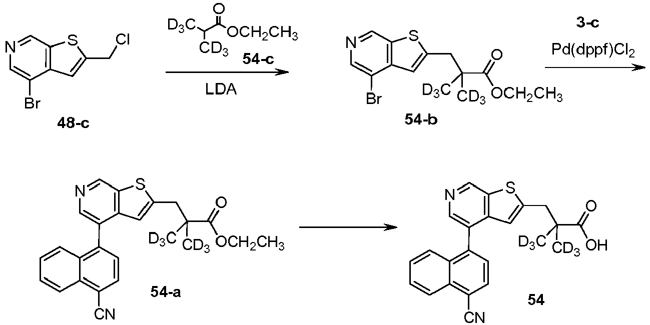

### Synthesis of compound 54-b

According to the process for preparing compound **48-b,** compound **54-b** (1000mg, 46%) was prepared by using commercially available compound **54-c.** LC-MS (ESI): m/z = 349 [M+H]⁺.

According to the process for preparing compound **47-a,** compound **54-a** (500mg, 72%) was prepared by using compound **54-b.** LC-MS (ESI): m/z = 421 [M+H]⁺.

### Synthesis of compound 54

According to the process for preparing compound **47,** white solid compound **54** (63mg, 32%) was prepared by compound **47-a.** LC-MS (ESI): m/z = 394 [M+H]⁺. ¹H-NMR (400MHz, DMSO-d6) δ: 12.46(s, 1H), 9.30 (s, 1H), 8.45(s, 1H), 8.32(d, J=8Hz, 1H), 8.26(d, J=8Hz, 1H), 7.89(d, J=8Hz, 1H), 7.74(d, J=8Hz, 1H), 7.65 (m, 1H), 7.59 (m, 1H), 6.67(s, 1H), 3.09(s, 2H) ppm

### Effect Example Biological assessment

### Example 1: the inhibitory activity against URAT1 of the compound of the present invention

Human embryonic kidney cells (HEK293) was incubated in DMEM tissue culture medium, at 37°C, under 5% CO₂ and 95% air atmosphere. TransIT-293 transfection agent (MIRUS BIO, Cat. No. MIR2706) and model URAT1 were used to construct transfected HEK293 cells. Transfected HEK293/hURAT1 cells were used to the test for ¹⁴C-uric acid transport activity.

HEK293/hURAT1 cells were seeded in a 96-well plate (BD, Cat. No. 356461) fully coating with poly-D-lysine at a density of 6×10⁴ cells per well. Cells were incubated at 37°C for at least 12hrs in the calorstat, and then washed with pre-heated washing buffer (125mM sodium gluconate, 10mM HEPES pH=7.4) at an amount of 200µL per well to wash out the culture medium. The uric acid [8-14C] (ARC, Cat. No. ARC0513-250UCI) containing or not containing the compound was added to 50µL HBSS buffer which was free of chloric ion each well (HBSS buffer: 125mM sodium gluconate, 4.8mM potassium gluconate, 1.3mM calcium gluconate, 1.2mM potassium dihydrogen phosphate, 1.2mM magnesium sulfate, 5.6mM glucose, 25mM HEPES pH=7.4) to make the specific concentration of the uric acid 1µCi per well. The incubating solution was removed after 10mins incubation, followed by adding 100µL cold washing buffer, after washing with this buffer for 3 times, the buffer was completely removed from the well. 50µL Lysis buffer (0.1mM NaOH) was added to each well, and transferred to a 96-well plate (PERKIN ELMER, Cat. No. 6005040) containing scintillation fluid after 5mins, and counted by MicroBeta Trilux (PerkinElmer) to give IC₅₀ value eventually.

The inhibitory activity of the compound of the present invention against hURAT1 was tested according to the assessment above, the results were listed below (Table 1):

**Table 1 IC₅₀ value of partial compounds of the present invention against hURAT1**

| Compound | IC₅₀ (µM) | Compound | IC₅₀ (µM) |
|---|---|---|---|
| Verinurad (RDEA3170) | 0.113 | 30 | 0.087 |
| 1 | 2.534 | 31 | 3.418 |
| 2 | 0.161 | 32 | 5.405 |
| 3 | 0.327 | 33 | 0.042 |
| 4 | 0.015 | 34 | 0.139 |
| 5 | 0.008 | 35 | 2.408 |
| 6 | 0.214 | 36 | 0.542 |
| 7A | 5.524 | 37 | 0.035 |
| 7B | 2.687 | 38 | 0.209 |
| 8 | 0.057 | 40 | 3.467 |
| 9 | 0.127 | 41 | 0.934 |
| 10 | 0.019 | 42 | 0.024 |
| 11 | 0.062 | 43A | 0.011 |
| 12 | 0.071 | 43B | 0.037 |
| 13 | 0.189 | 44A | 0.038 |
| 14 | 0.385 | 44B | 1.858 |
| 15 | 0.024 | 45A | 0.580 |
| 16 | 0.015 | 45B | 0.010 |
| 17 | 0.012 | 46A | 0.148 |
| 21 | 0.100 | 46B | 0.051 |
| 22 | 0.055 | 47 | 0.018 |
| 23 | 0.018 | 48 | 0.012 |
| 24 | 0.018 | 49 | 0.116 |
| 25 | 0.289 | 50 | 0.971 |
| 26 | 0.119 | 51 | 0.019 |
| 27 | 1.731 | 52 | 0.037 |
| 29 | 2.858 | / | / |

Compound Verinurad (RDEA3170, CAS No.:1352792-74-5) was a known hURAT1 inhibitor having a structure shown as below:

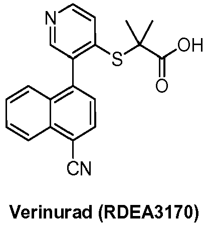

What can be concluded from Table 1 was that compounds of the present invention are of significantly inhibitory effects against hURAT1.

## Claims

1. A condensed ring derivative having a structure of formula II-1 or IV-1, a tautomer, a mesomer, a racemate, an enantiomer, a diastereoisomer, or a pharmaceutically acceptable salt thereof, 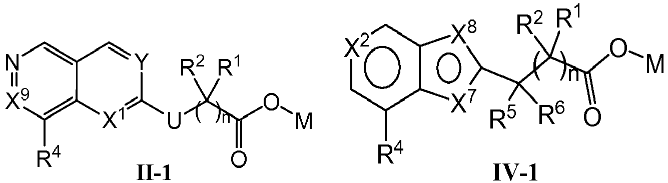
M is H, D or a pharmaceutically acceptable cation;
U is 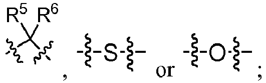
X¹ is CH or N; X² is N;
X⁹ is C or N;
Y is CH or N;
each of X⁷ and X⁸ is independently CH or S;
each of R¹ and R² is independently H, D, a halogen, CN, an alkyl, an alkoxy, a cycloalkyl, an alkenyl, an alkynyl or a heterocycloalkyl; or, R¹, R² together with the carbon atom attached form a cycloalkyl or a heterocyclic group; the alkyl, the alkoxy, the cycloalkyl, the alkenyl, the alkynyl, the heterocycloalkyl, the cycloalkyl formed by R¹, R² and the carbon atom attached, or the heteroalkyl formed by R¹, R² and the carbon atom attached can further be substituted by a substituent selected from the group consisting of D, a halogen, CN, an alkyl, an alkoxy, a cycloalkyl, an alkenyl, an alkynyl, a heterocycloalkyl or an aryl;
R⁴ is an alkyl, an alkoxy, a cycloalkyl, an alkenyl, an alkynyl, a heterocycloalkyl, each of which are substituted by a substituent selected from the group consisting of an aryl, an aryl substituted by a halogen and/or CN, a heteroaryl or a heteroaryl substituted by CN; or an aryl or a heteroaryl wherein the aryl or the heteroaryl can further be substituted by a substituent selected from the group consisting of D, a halogen, CN, an alkyl, an alkoxy, a cycloalkyl, an alkenyl, an alkynyl, a heterocycloalkyl, an aryl, an aryl substituted by a halogen and/or CN, a heteroaryl or a heteroaryl substituted by CN;
each of R⁵ and R⁶ is independently H, D, OH, a halogen, CN, an alkyl, an alkoxy, a cycloalkyl, an alkenyl, an alkynyl or a heterocycloalkyl; or R⁵, R⁶ together with the carbon atom attached form a cycloalkyl or a heterocyclic group; the alkyl, the alkoxy, the cycloalkyl, the alkenyl, the alkynyl, the heterocycloalkyl, the cycloalkyl formed by R⁵, R⁶ together with the carbon atom attached or the heterocyclic group formed by R⁵, R⁶ together with the carbon atom attached can further be substituted by a substituent selected from the group consisting of D, a halogen, CN, an alkyl, an alkoxy, a cycloalkyl, an alkenyl, an alkynyl, a heterocycloalkyl or an aryl;
n is 0, 1 or 2.

2. The condensed ring derivative having a structure of formula II-1 or IV-1, the tautomer, the mesomer, the racemate, the enantiomer, the diastereoisomer, or the pharmaceutically acceptable salt thereof according to claim 1, wherein,
M is H or a pharmaceutically acceptable cation;
each of R¹ and R² is independently H, D or an alkyl; or R¹ and R², together with the carbon atom attached form a cycloalkyl;
R³ is H, a halogen, an alkyl or an aryl;
R⁴ is an aryl or a heteroaryl;
each of R⁵ and R⁶ is independently H, OH, a halogen or an alkyl.

3. The condensed ring derivative having a structure of formula II-1 or IV-1, the tautomer, the mesomer, the racemate, the enantiomer, the diastereoisomer, or the pharmaceutically acceptable salt thereof according to claim 1, wherein,
M is Na ion, K ion or Ca ion;
and/or, each of R¹ and R² is independently F, Cl, Br or I, C₁₋₄ alkyl, C₁₋₄ alkoxy, C₃₋₆ cycloalkyl, C₂₋₄ alkenyl, C₂₋₄ alkynyl, or a C₂₋₁₀ heterocycloalkyl having 1-2 heteroatom(s) selected from O, S or N, or R¹ and R² together with the carbon atom attached form a C₃₋₆ cycloalkyl, or, R¹ and R² together with the carbon atom attached form a C₂₋₅ heterocyclic group having 1-2 heteroatom(s) selected from O or S;
and/or, R³ is F, Cl, Br or I, C₁₋₄ alkyl, C₁₋₄ alkoxy, C₆₋₁₀ aryl, or, a C₂₋₅ heteroaryl having 1-3 heteroatom(s) selected from N, or, a C₂₋₁₀ heterocycloalkyl having 1-3 heteroatom(s) selected from N, O or S;
and/or, R⁴ is a C₆₋₁₀ aryl, or a C₂₋₁₀heteroaryl having 1-2 heteroatom(s) selected from O; and/or, each of R⁵ and R⁶ is independently F, Cl, Br or I, a C₁₋₄ alkyl, C₁₋₄ alkoxy, a C₃₋₆ cycloalkyl, a C₂₋₄ alkenyl, a C₂₋₄ alkynyl, or a C₂₋₁₀ heterocycloalkyl having 1-2 heteroatom(s) selected from O, S or N, or, R⁵ and R⁶ together with the carbon atom form a C₃₋₆ cycloalkyl, or, R⁵, R⁶ together with the carbon atom attached form a C₂₋₁₀ heterocycloalkyl having 1-2 heteroatom(s) selected from O or S.

4. The condensed ring derivative having a structure of formula II-1 or IV-1, the tautomer, the mesomer, the racemate, the enantiomer, the diastereoisomer, or the pharmaceutically acceptable salt thereof according to claim 1, wherein,
the cycloalkyl formed by R¹ and R² together with the carbon atom attached, or the heterocyclic group formed by R¹, R² together with the carbon atom attached, is substituted by a substituent selected from the group consisting of F, Cl, Br, I, a C₁₋₄ alkyl, a C₁₋₄ alkoxy, a C₃₋₆ cycloalkyl, a C₂₋₄ alkenyl, a C₂₋₄ alkynyl, a C₆₋₁₀ aryl, a C₂₋₁₀ heterocycloalkyl having 1-2 heteroatom(s) selected from O, S or N;
and/or, the cycloalkyl formed by R⁵, R⁶ together with the carbon atom attached or the heterocyclic group formed by R⁵, R⁶ together with the carbon atom attached is substituted by a substituent selected from the group consisting of F, Cl, Br, I, a C₁₋₄ alkyl, a C₁₋₄ alkoxy, a C₃₋₆ cycloalkyl, a C₂₋₄ alkenyl, a C₂₋₄ alkynyl, a C₆₋₁₀ aryl, a C₂₋₁₀ heterocycloalkyl having 1-2 heteroatom(s) selected from O, S or N;
and/or, the alkyl, the alkoxy, the aryl, the heteroaryl, the heterocycloalkyl or the amino defined in R³ is substituted by a substituent selected from the group consisting of F, Cl, Br, I, a C₁₋₄ alkyl, a C₆₋₁₀ aryl, 2,6-dichlorophenyl, 2,6-dichlorobenzyl and 2,6-dichlorobenzoyl;
and/or, the alkyl, the alkoxy, the cycloalkyl, the alkenyl, the alkynyl, the heterocycloalkyl defined in R⁴ is substituted by a substituent selected from the group consisting of 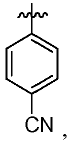 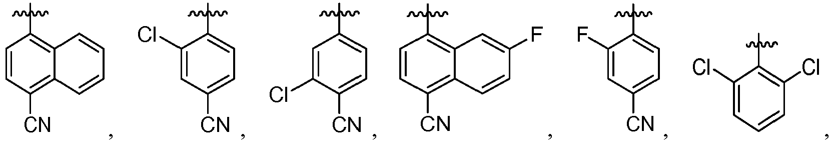 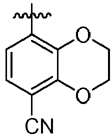 and a C₂₋₁₀ heteroaryl having 1-2 heteroatom(s) selected from O,
and/or the aryl or the heteroaryl defined in R⁴ is substituted by a substituent selected from the group consisting of F, Cl, Br, I, a C₁₋₄ alkyl, a C₁₋₄ alkoxy, a C₃₋₆ cycloalkyl, a C₂₋₄ alkenyl, a C₂₋₄ alkynyl, a C₆₋₁₀ aryl, a C₂₋₁₀ heterocycloalkyl having 1-2 heteroatom(s) selected from O, S 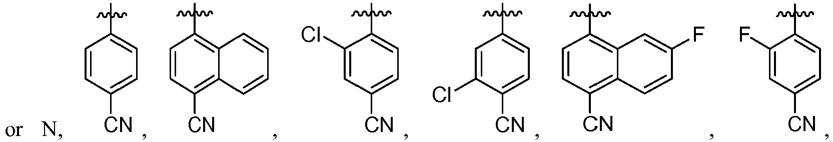 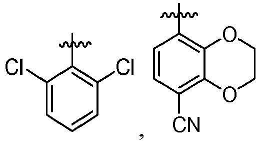 and a C₂₋₁₀ heteroaryl having 1-2 heteroatom(s) selected from O.

5. The condensed ring derivative having a structure of formula II-1 or IV-1, the tautomer, the mesomer, the racemate, the enantiomer, the diastereoisomer, or the pharmaceutically acceptable salt thereof according to claim 3, wherein,
each of R¹ and R² is independently methyl, ethyl, *n*-propyl, *iso*-propyl, *n*-butyl, *iso*-butyl or *tert*-butyl;
or, R¹ and R² together with the carbon atom attached form a cyclobutyl;
and/or, R³ is methyl, ethyl, *n*-propyl, *iso*-propyl, *n*-butyl, *iso-*butyl, *tert*-butyl, a phenyl, a pyridyl or 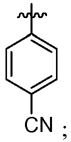 and/or, R⁴ is a phenyl, a naphthyl, 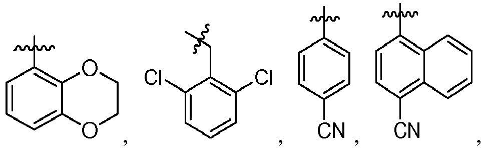 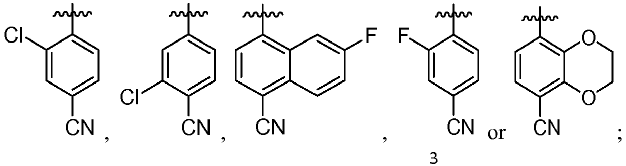 and/or, each of R⁵ and R⁶ is independently methyl, ethyl, *n*-propyl, *iso*-propyl, *n*-butyl, *iso-*butyl or *tert*-butyl.

6. The condensed ring derivative having a structure of formula II-1 or IV-1, the tautomer, the mesomer, the racemate, the enantiomer, the diastereoisomer, or the pharmaceutically acceptable salt thereof according to claim 1, wherein,
in the compound of general formula II-1,
each of R¹ and R² is independently H, methyl, ethyl, n-propyl, iso-propyl, n-butyl, iso-butyl or tert-butyl; or R¹, R² together with the carbon atom attached form a cyclobutyl;
M is H;
R⁴ is a phenyl, a naphthyl, 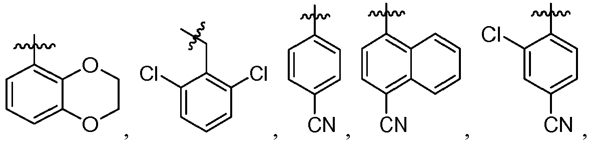 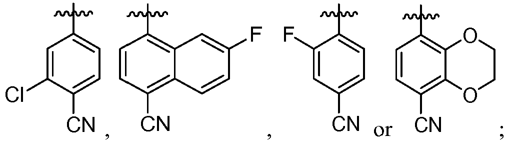 U is 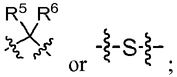 each of R⁵ and R⁶ is independently H, methyl, ethyl, n-propyl, iso-propyl, n-butyl, iso-butyl or tert-butyl;
n is 1;
in the compound of formula IV-1,
each of X⁷ and X⁸ is independently CH or S;
each of R¹ and R² is independently H, methyl, ethyl, n-propyl, iso-propyl, n-butyl, iso-butyl or tert-butyl;
R⁴ is a phenyl, a naphthyl, 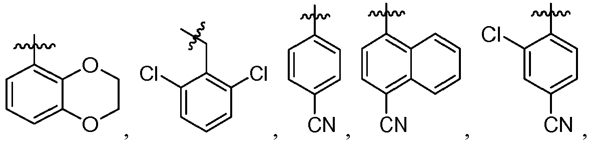 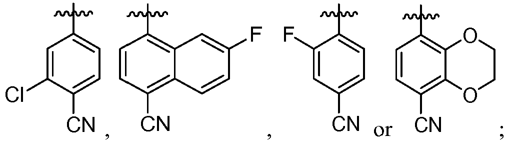 each of R⁵ and R⁶ is independently H, methyl, ethyl, n-propyl, iso-propyl, n-butyl, iso-butyl or tert-butyl;
n is 0 or 1.

7. The condensed ring derivative having a structure of formula II-1 or IV-1, the tautomer, the mesomer, the racemate, the enantiomer, the diastereoisomer, or the pharmaceutically acceptable salt thereof according to claim 6, wherein
in the compound of formula II-1, where U is 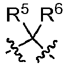 and R⁵ and R⁶ are H, R¹ and R² are not H at the same time;
in the compound of formula IV-1,
X⁷ is CH; X⁸ is S.

8. The condensed ring derivative having a structure of formula II-1 or IV-1, the tautomer, the mesomer, the racemate, the enantiomer, the diastereoisomer, or the pharmaceutically acceptable salt thereof according to claim 6, wherein
in the compound of formula II-1, where U is 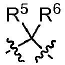 and R⁵ and R⁶ are H, R¹ and R² are not H at the same time;
in the compound of formula IV-1, where R¹ and/or R² is an alkyl, R⁵ and R⁶ are H; where R⁵ and/or R⁶ is an alkyl, R¹ and R² are H; where X⁷ is S, X⁸ is CH, R¹ and R² are alkyl, R⁵ and R⁶ are H, R⁴ is a phenyl.

9. The condensed ring derivative, the tautomer, the mesomer, the racemate, the enantiomer, the diastereoisomer, or the pharmaceutically acceptable salt thereof, according to at least one of the claims 1-8, which selected from the groups consisting of: 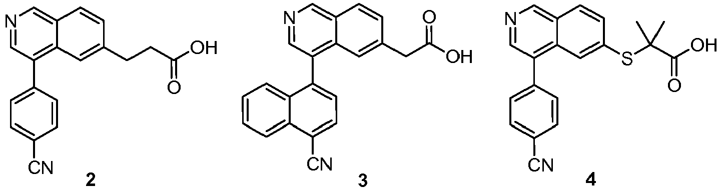 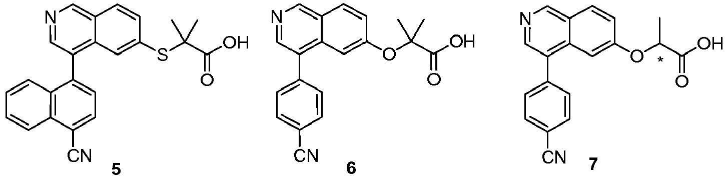 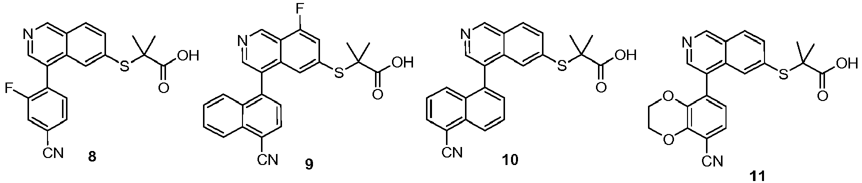 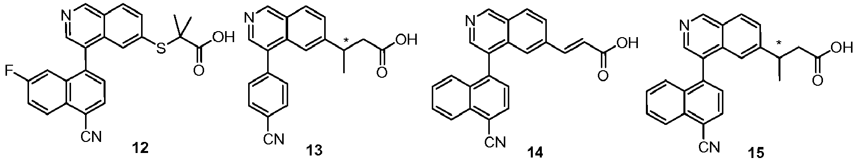 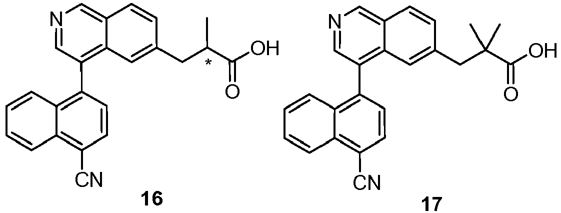 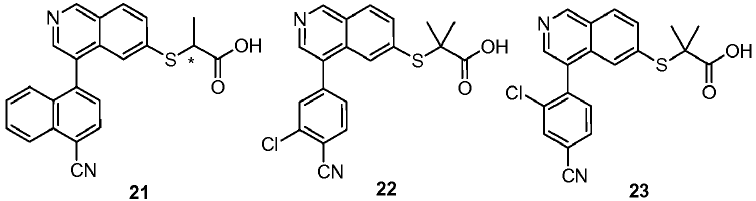 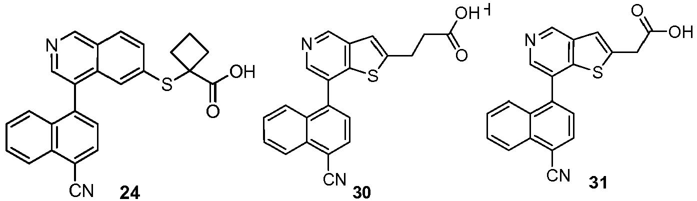 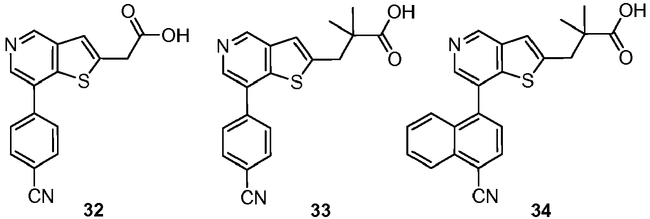 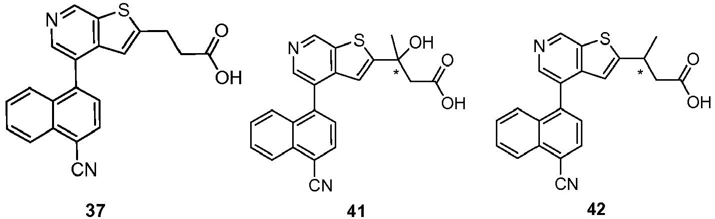 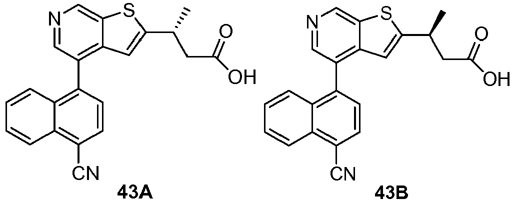 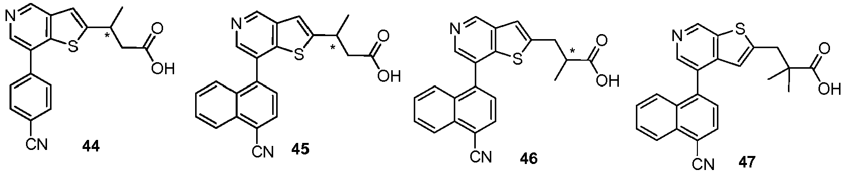 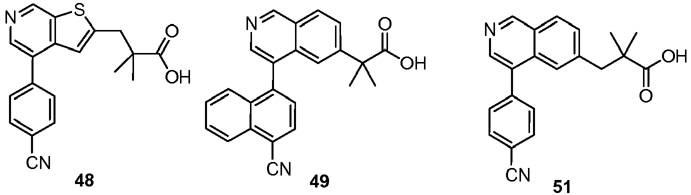 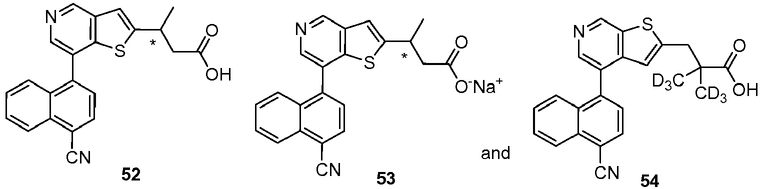 in the above compounds, the carbon atom marked with * refers to a chiral carbon atom or a non-chiral carbon atom, when it is a chiral carbon atom, it is of S-configuration or R-configuration, when it is a non-chiral carbon atom, it refers to racemate.

10. The compound of formula II-a-1 or IV-a-1: 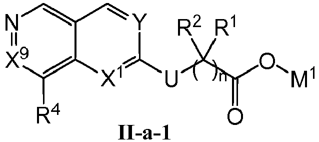 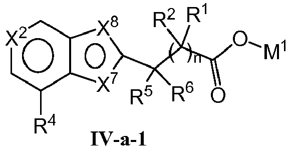 M¹ is an alkyl;
in the compound of formula II-a-1, X¹ is CH or N; Y is CH or N; the definitions of X⁹, R¹, R²,
R³, R⁴, U and n refers to those in any one of claims 1-9;
in the compound of formula IV-a-1, each of X⁷ and X⁸ is independently CH or S; the definitions of R¹, R², R⁴, R⁵, R⁶, X² and n refer to those in any one of claims 1-9.

11. The compound of formula II-a-1 or IV-a-1 according to claim 10, wherein, the compound is selected from the group consisting of 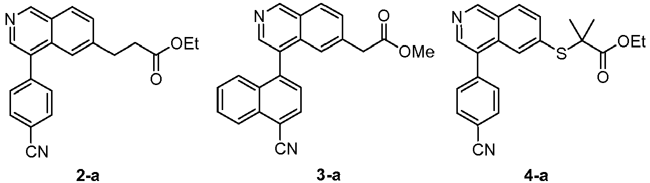 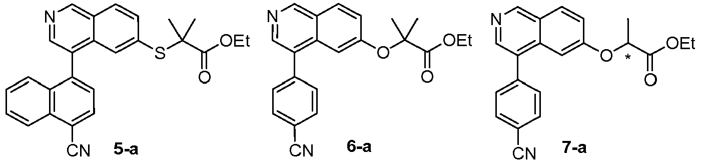 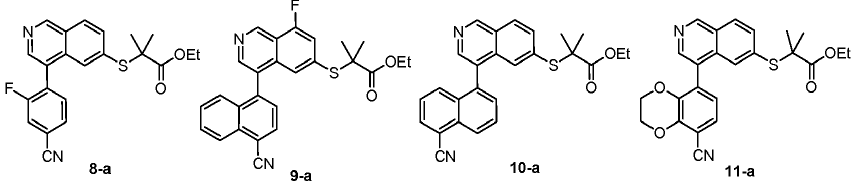 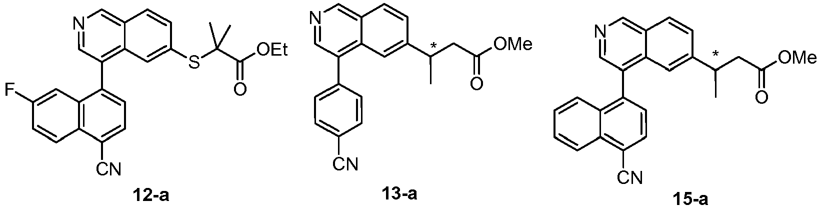 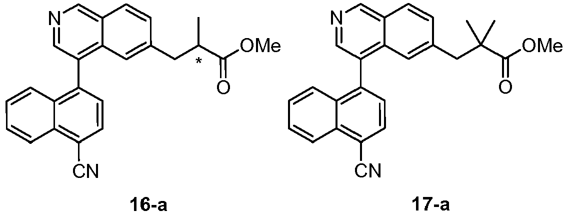 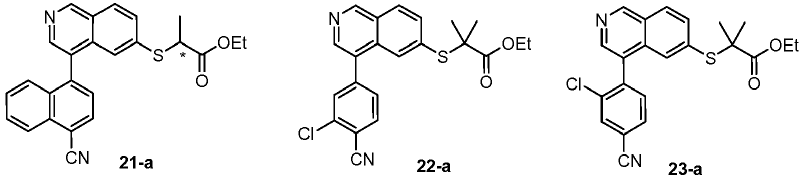 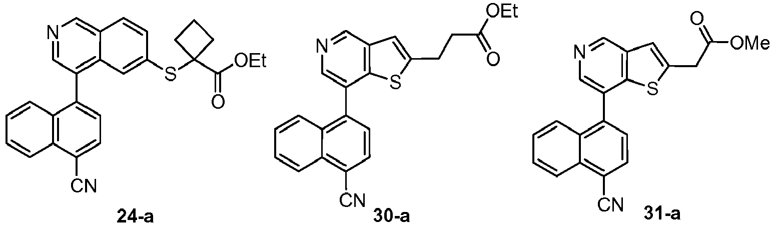 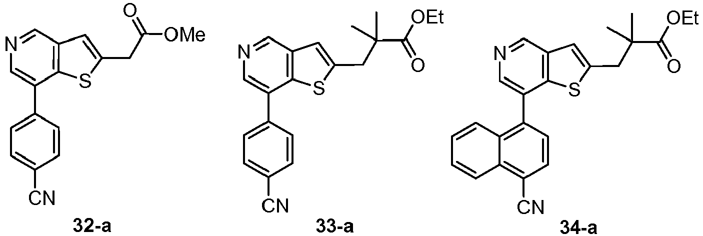 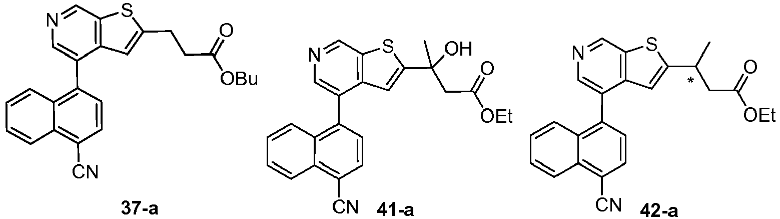 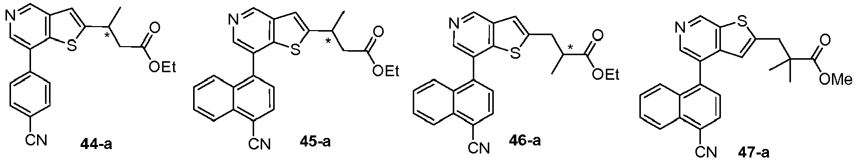 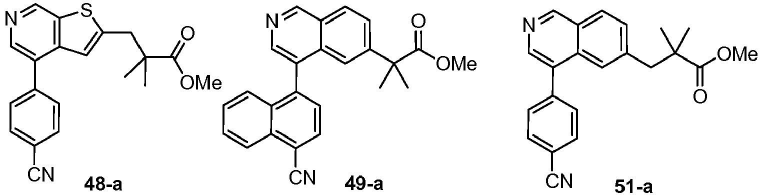 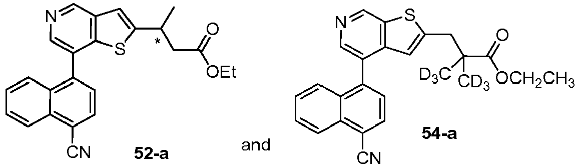 in the above compounds, the carbon atom marked with * is a chiral carbon atom or a non-chiral carbon atom, when it is a chiral carbon atom, it is of S-configuration or R-configuration, when it is a non-chiral carbon atom, it refers to racemate.

12. The condensed ring derivative having a structure of formula II-1 or IV-1, the tautomer, the mesomer, the racemate, the enantiomer, the diastereoisomer, or the pharmaceutically acceptable salt thereof according to at least one of claims 1-9 for use in preventing and/or treating hyperuricemia or the disease related to hyperuricemia.

13. The condensed ring derivative having a structure of formula II-1 or IV-1, the tautomer, the mesomer, the racemate, the enantiomer, the diastereoisomer, or the pharmaceutically acceptable salt thereof for use according to claim 12, wherein, the disease is related to hyperuricemia is selected from the group consisting of gout, hypertension, diabetes, hypertriglyceridemia, metabolic syndrome, coronary heart disease and kidney damage.

14. A pharmaceutical composition, which contains a pharmaceutically effective amount of the condensed ring derivative having a structure of formula II-1 or IV-1, the tautomer, the mesomer, the racemate, the enantiomer, the diastereoisomer, or the pharmaceutically acceptable salt thereof according to at least one of claims 1-9, and one or more than one pharmaceutically acceptable carrier and/or diluent.

15. The pharmaceutical composition according to claim 14, wherein, the composition further contains other uric acid-lowering drugs; the uric acid-lowering drugs is selected from the group consisting of uric acid transporter 1 inhibitor, xanthine oxidase inhibitor, xanthine oxidoreductase and xanthine dehydrogenase inhibitor.

16. The pharmaceutical composition according to claim 15, wherein, the composition further contains purine alcohol and/or Febuxostat.

## Patentansprüche

1. Kondensiertes Ringderivat mit einer Struktur der Formel II-1 oder IV-1, Tautomer, Mesomer, Racemat, Enantiomer, Diastereomer oder pharmazeutisch annehmbares Salz davon: 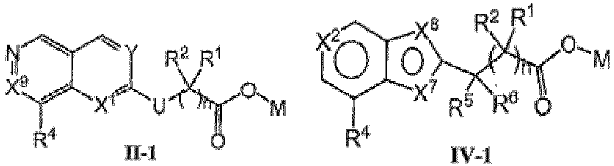 wobei
M = H, D oder ein pharmazeutisch annehmbares Kation ist;
U = 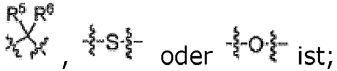
X¹ = CH oder N ist; X² = N ist;
X⁹ = C oder N ist;
Y = CH oder N ist;
X⁷ und X⁸ jeweils unabhängig CH oder S sind;
R¹ und R² jeweils unabhängig H, D, ein Halogen, CN, Alkyl, Alkoxy, Cycloalkyl, Alkenyl, Alkinyl oder Heterocycloalkyl sind; oder R¹, R² zusammen mit dem Kohlenstoffatom, an das sie gebunden sind, eine Cycloalkyl- oder heterocyclische Gruppe bilden; das Alkyl, Alkoxy, Cycloalkyl, Alkenyl, Alkinyl, Heterocycloalkyl, das durch R¹, R² zusammen mit dem Kohlenstoffatom, an das sie gebunden sind, gebildete Cycloalkyl oder das durch R¹, R² zusammen mit dem Kohlenstoffatom, an das sie gebunden sind, gebildete Heteroalkyl weiterhin mit einem Substituenten substituiert sein kann, der aus der Gruppe ausgewählt ist, die aus D, einem Halogen, CN, Alkyl, Alkoxy, Cycloalkyl, Alkenyl, Alkinyl, Heterocycloalkyl oder Aryl besteht;
R⁴ Folgendes ist: ein Alkyl, Alkoxy, Cycloalkyl, Alkenyl, Alkinyl, Heterocycloalkyl, die jeweils mit einem Substituenten substituiert sind, der aus der Gruppe ausgewählt ist, die aus einem Aryl, einem mit einem Halogen und/oder CN substituierten Aryl, einem Heteroaryl oder einem mit CN substituierten Heteroaryl besteht; oder ein Aryl oder Heteroaryl, wobei das Aryl oder Heteroaryl weiterhin mit einem Substituenten substituiert sein kann, der aus der Gruppe ausgewählt ist, die aus D, einem Halogen, CN, Alkyl, Alkoxy, Cycloalkyl, Alkenyl, Alkinyl, Heterocycloalkyl, Aryl, einem mit einem Halogen und/oder CN substituierten Aryl, einem Heteroaryl oder einem mit CN substituierten Heteroaryl besteht;
R⁵ und R⁶ jeweils unabhängig H, D, OH, ein Halogen, CN, Alkyl, Alkoxy, Cycloalkyl, Alkenyl, Alkinyl oder Heterocycloalkyl sind; oder R⁵, R⁶ zusammen mit dem Kohlenstoffatom, an das sie gebunden sind, eine Cycloalkyl- oder heterocyclische Gruppe bilden; das Alkyl, Alkoxy, Cycloalkyl, Alkenyl, Alkinyl, Heterocycloalkyl, das durch R⁵, R⁶ zusammen mit dem Kohlenstoffatom, an das sie gebunden sind, gebildete Cycloalkyl oder die durch R⁵, R⁶ zusammen mit dem Kohlenstoffatom, an das sie gebunden sind, gebildete heterocyclische Gruppe weiterhin mit einem Substituenten substituiert sein kann, der aus der Gruppe ausgewählt ist, die aus D, einem Halogen, CN, Alkyl, Alkoxy, Cycloalkyl, Alkenyl, Alkinyl, Heterocycloalkyl oder Aryl besteht;
n = 0, 1 oder 2 ist.

2. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 1, wobei
M = H oder ein pharmazeutisch annehmbares Kation ist;
R¹ und R² jeweils unabhängig H, D oder ein Alkyl sind oder R¹ und R² zusammen mit dem Kohlenstoffatom, an das sie gebunden sind, ein Cycloalkyl bilden;
R³ = H, ein Halogen, Alkyl oder Aryl ist;
R⁴ ein Aryl oder Heteroaryl ist;
R⁵ und R⁶ jeweils unabhängig H, OH, ein Halogen oder ein Alkyl sind.

3. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 1, wobei
M ein Na-Ion, K-Ion oder Ca-Ion ist;
und/oder R¹ und R² jeweils unabhängig F, Cl, Br oder I, C₁₋₄-Alkyl, C₁₋₄-Alkoxy, C₃₋₆-Cycloalkyl, C₂₋₄-Alkenyl, C₂₋₄-Alkinyl oder ein C₂₋₁₀-Heterocycloalkyl mit 1-2 Heteroatomen, die aus O, S oder N ausgewählt sind, sind oder R¹ und R² zusammen mit dem Kohlenstoffatom, an das sie gebunden sind, ein C₃₋₆-Cycloalkyl bilden oder R¹ und R² zusammen mit dem Kohlenstoffatom, an das sie gebunden sind, eine heterocyclische C₂₋₅-Gruppe mit 1-2 Heteroatomen, die aus O oder S ausgewählt sind, sind;
und/oder R³ = F, Cl, Br oder I, C₁₋₄-Alkyl, C₁₋₄-Alkoxy, C₆₋₁₀-Aryl oder C₂₋₅-Heteroaryl mit 1-3 Heteroatomen, die aus N ausgewählt sind, oder C₂₋₁₀-Heterocycloalkyl mit 1-3 Heteroatomen, die aus N, O oder S ausgewählt sind, ist;
und/oder R⁴ ein C₆₋₁₀-Aryl oder C₂₋₁₀-Heteroaryl mit 1-2 Heteroatomen, die aus O ausgewählt sind, ist;
und/oder R⁵ und R⁶ jeweils unabhängig F, Cl, Br oder I, C₁₋₄-Alkyl, C₁₋₄-Alkoxy, C₃₋₆-Cycloalkyl, C₂₋₄-Alkenyl, C₂₋₄-Alkinyl oder ein C₂₋₁₀-Heterocycloalkyl mit 1-2 Heteroatomen, die aus O, S oder N ausgewählt sind, sind oder R⁵ und R⁶ zusammen mit dem Kohlenstoffatom ein C₃₋₆-Cycloalkyl bilden oder R⁵, R⁶ zusammen mit dem Kohlenstoffatom, an das sie gebunden sind, ein C₂₋₁₀-Heterocycloalkyl mit 1-2 Heteroatomen, die aus O oder S ausgewählt sind, sind.

4. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 1, wobei
das durch R¹ und R² zusammen mit dem Kohlenstoffatom, an das sie gebunden sind, gebildete Cycloalkyl oder die durch R¹ und R² zusammen mit dem Kohlenstoffatom, an das sie gebunden sind, gebildete heterocyclische Gruppe mit einem Substituenten substituiert ist, der aus der Gruppe ausgewählt ist, die aus F, Cl, Br, I, C₁₋₄-Alkyl, C₁₋₄-Alkoxy, C₃₋₆-Cycloalkyl, C₂₋₄-Alkenyl, C₂₋₄-Alkinyl, C₆₋₁₀-Aryl, C₂₋₁₀-Heterocycloalkyl mit 1-2 Heteroatomen, die aus O, S oder N ausgewählt sind, besteht;
und/oder das durch R⁵, R⁶ zusammen mit dem Kohlenstoffatom, an das sie gebunden sind, gebildete Cycloalkyl oder die durch R⁵, R⁶ zusammen mit dem Kohlenstoffatom, an das sie gebunden sind, gebildete heterocyclische Gruppe mit einem Substituenten substituiert ist, der aus der Gruppe ausgewählt ist, die aus F, Cl, Br, I, C₁₋₄-Alkyl, C₁₋₄-Alkoxy, C₃₋₆-Cycloalkyl, C₂₋₄-Alkenyl, C₂₋₄-Alkinyl, C₆₋₁₀-Aryl, C₂₋₁₀-Heterocycloalkyl mit 1-2 Heteroatomen, die aus O, S oder N ausgewählt sind, besteht;
und/oder das bei R³ definierte Alkyl, Alkoxy, Aryl, Heteroaryl, Heterocycloalkyl oder Amino mit einem Substituenten substituiert ist, der aus der Gruppe ausgewählt ist, die aus F, Cl, Br, I, C₁₋₄-Alkyl, C₆₋₁₀-Aryl, 2,6-Dichlorphenyl, 2,6-Dichlorbenzyl und 2,6-Dichlorbenzoyl besteht;
und/oder das bei R⁴ definierte Alkyl, Alkoxy, Cycloalkyl, Alkenyl, Alkinyl, Heterocycloalkyl mit einem Substituenten substituiert ist, der aus der Gruppe ausgewählt ist, die aus 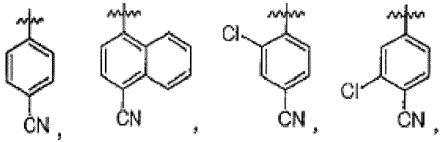 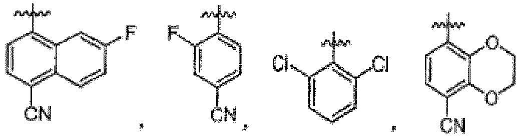 und einem C₂₋₁₀-Heteroaryl mit 1-2 Heteroatomen, die aus O ausgewählt sind, besteht, und/oder das bei R⁴ definierte Aryl oder Heteroaryl mit einem Substituenten substituiert ist, der aus der Gruppe ausgewählt ist, die aus F, Cl, Br, I, C₁₋₄-Alkyl, C₁₋₄-Alkoxy, C₃₋₆-Cycloalkyl, C₂₋₄-Alkenyl, C₂₋₄-Alkinyl, C₆₋₁₀-Aryl, C₂₋₁₀-Heterocycloalkyl mit 1-2 Heteroatomen, die aus O, S oder N ausgewählt sind, 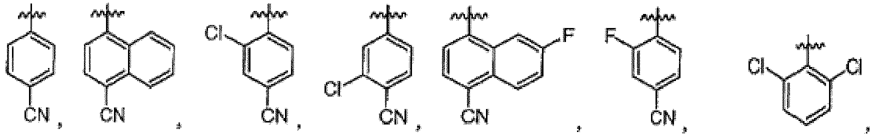 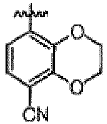 und einem C₂₋₁₀-Heteroaryl mit 1-2 Heteroatomen, die aus O ausgewählt sind, besteht.

5. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 3, wobei
R¹ und R² jeweils unabhängig Methyl, Ethyl, n-Propyl, Isopropyl, n-Butyl, Isobutyl oder tert-Butyl sind;
oder R¹ und R² zusammen mit dem Kohlenstoffatom, an das sie gebunden sind, ein Cyclobutyl bilden;
und/oder R³ Methyl, Ethyl, n-Propyl, Isopropyl, n-Butyl, Isobutyl, tert-Butyl, Phenyl, Pyridyl oder 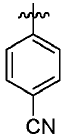 ist; und/oder R⁴ Phenyl, Naphthyl, 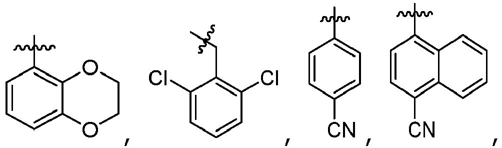 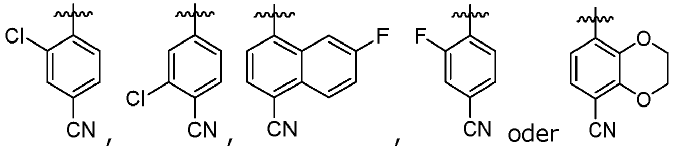 ist;
und/oder R⁵ und R⁶ jeweils unabhängig Methyl, Ethyl, n-Propyl, Isopropyl, n-Butyl, Isobutyl oder tert-Butyl sind.

6. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 3, wobei
in der Verbindung der allgemeinen Formel II-1:
R¹ und R² jeweils unabhängig H, Methyl, Ethyl, n-Propyl, Isopropyl, n-Butyl, Isobutyl oder tert-Butyl sind oder R¹ und R² zusammen mit dem Kohlenstoffatom, an das sie gebunden sind, ein Cyclobutyl bilden;
M = H ist;
R⁴ Phenyl, Naphthyl, 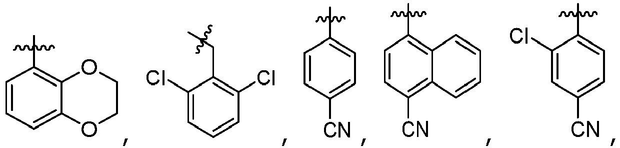 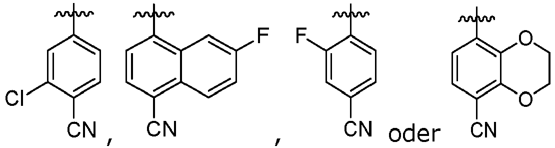 ist;
U = 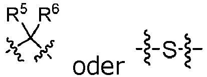 ist;
R⁵ und R⁶ jeweils unabhängig H, Methyl, Ethyl, n-Propyl, Isopropyl, n-Butyl, Isobutyl oder tert-Butyl sind;
n = 1 ist;
in der Verbindung der Formel IV-1:
X⁷ und X⁸ jeweils unabhängig CH oder S sind;
R¹ und R² jeweils unabhängig H, Methyl, Ethyl, n-Propyl, Isopropyl, n-Butyl, Isobutyl oder tert-Butyl sind;
R⁴ Phenyl, Naphthyl, 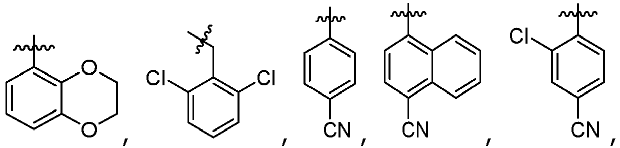 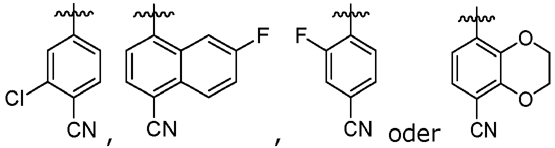 ist;
R⁵ und R⁶ jeweils unabhängig H, Methyl, Ethyl, n-Propyl, Isopropyl, n-Butyl, Isobutyl oder tert-Butyl sind;
n = 0 oder 1 ist.

7. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 6, wobei
in der Verbindung der Formel II-1, bei der U = 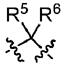 ist und R⁵ und R⁶ = H sind, R¹ und R² nicht gleichzeitig H sind;
in der Verbindung der Formel IV-1:
X⁷ = CH ist, X⁸ = S ist.

8. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß Anspruch 6, wobei
in der Verbindung der Formel II-1, bei der U = 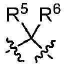 ist und R⁵ und R⁶ = H sind, R¹ und R² nicht gleichzeitig H sind;
in der Verbindung der Formel IV-1, bei der R¹ und/oder R² Alkyl sind, R⁵ und R⁶ = H sind; wenn R⁵ und/oder R⁶ Alkyl sind, R¹ und R² = H sind; bei der X⁷ = S ist, X⁸ = CH ist, R¹ und/oder R² Alkyl sind, R⁵ und R⁶ = H sind, R⁴ ein Phenyl ist.

9. Kondensiertes Ringderivat, Tautomer, Mesomer, Racemat, Enantiomer, Diastereomer oder pharmazeutisch annehmbares Salz davon gemäß wenigstens einem der Ansprüche 1 bis 8, das aus der Gruppe ausgewählt ist, die aus folgenden besteht: 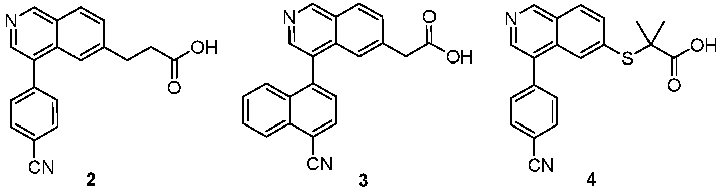 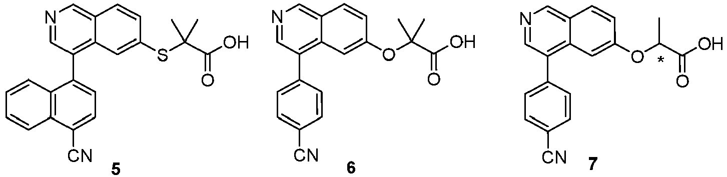 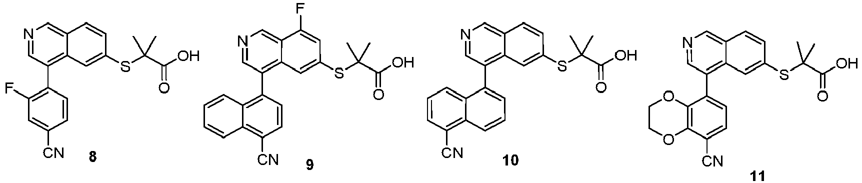 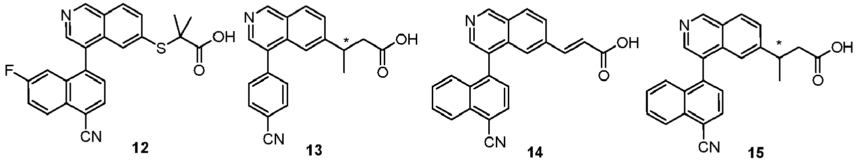 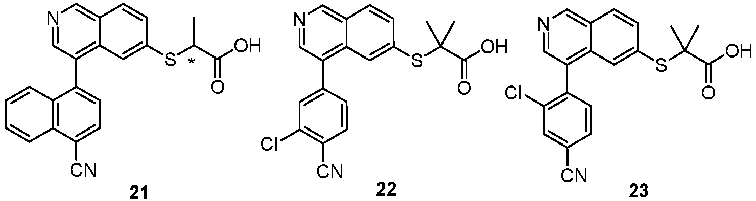 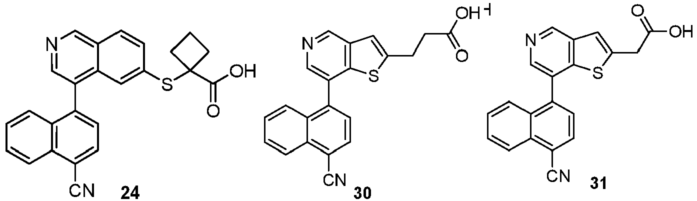 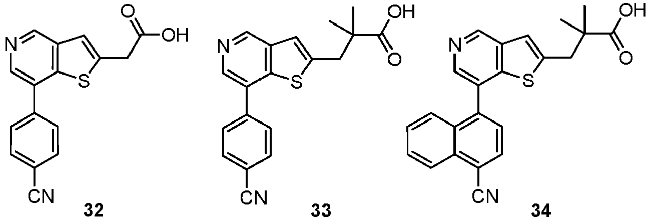 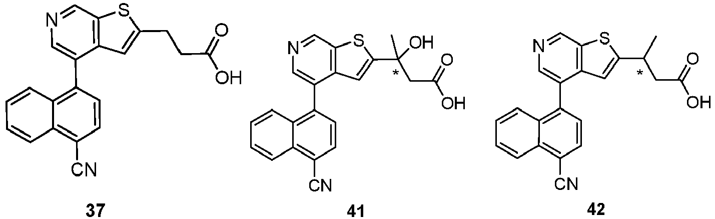 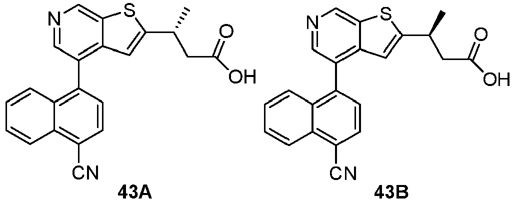 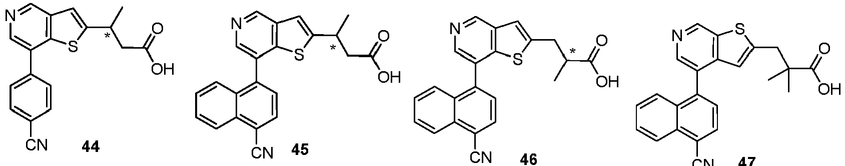 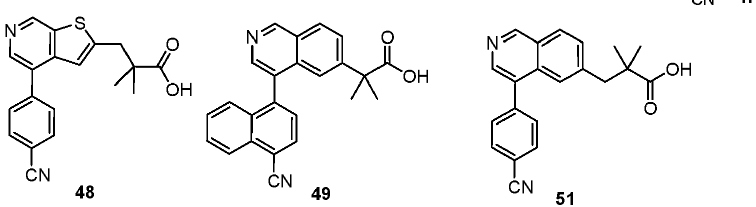 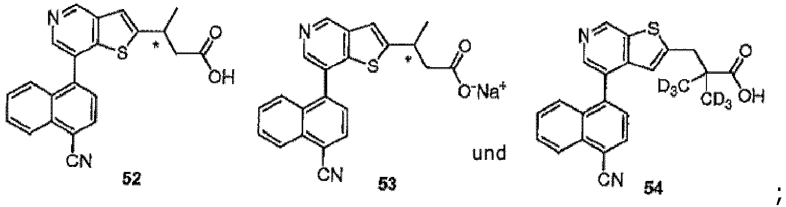 wobei sich in den obigen Verbindungen das mit * markierte Kohlenstoffatom auf ein chirales Kohlenstoffatom oder ein nichtchirales Kohlenstoffatom bezieht, das, wenn es ein chirales Kohlenstoffatom ist, in der S-Konfiguration oder R-Konfiguration vorliegt, und wenn es ein nichtchirales Kohlenstoffatom ist, sich auf ein Racemat bezieht.

10. Verbindung der Formel II-a-1 oder IV-a-1: 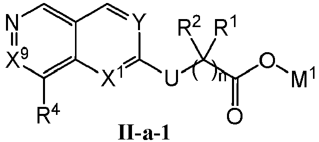 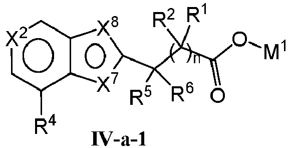 wobei M¹ ein Alkyl ist;
in der Verbindung der Formel II-a-1: X¹ = CH oder N ist, Y = CH oder N ist, sich die Definitionen von X⁹, R¹, R², R³, R⁴, U und n auf diejenigen in einem der Ansprüche 1 bis 9 beziehen;
in der Verbindung der Formel IV-a-1: X⁷ und X⁸ jeweils unabhängig CH oder S sind, sich die Definitionen von R¹, R², R⁴, R⁵, R⁶, X² und n auf diejenigen in einem der Ansprüche 1 bis 9 beziehen.

11. Verbindung der Formel II-a-1 oder IV-a-1 gemäß Anspruch 10, wobei die Verbindung aus der Gruppe ausgewählt ist, die aus folgenden besteht: 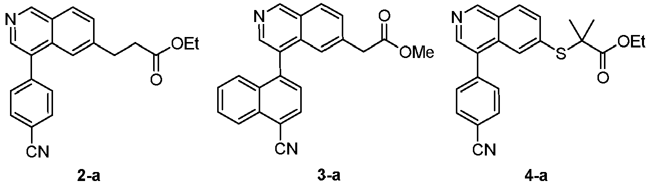 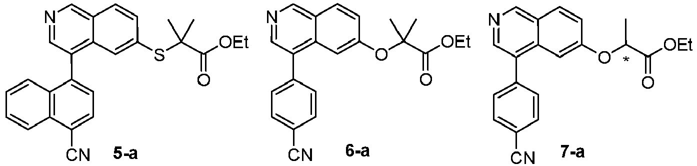 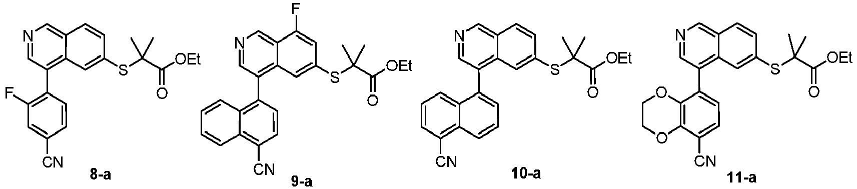 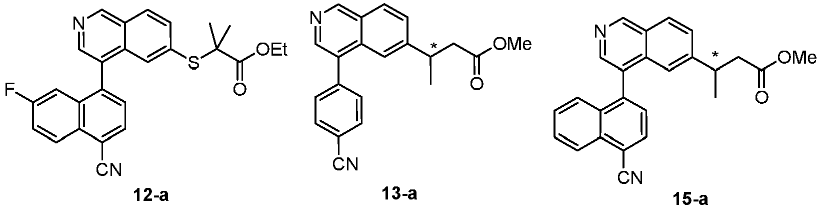 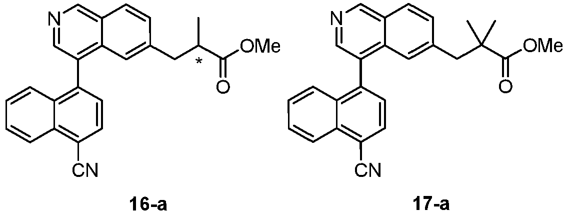 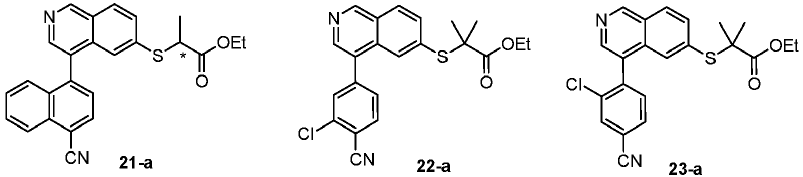 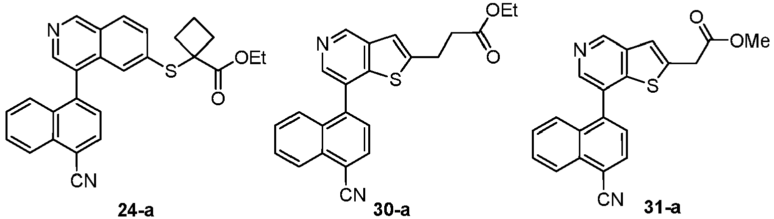 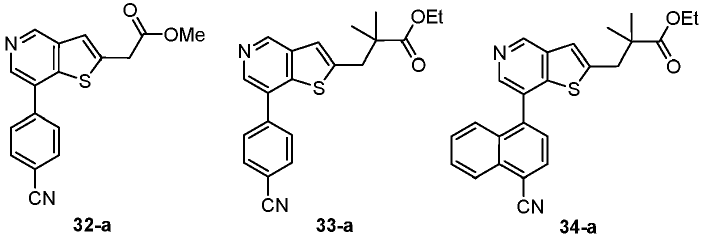 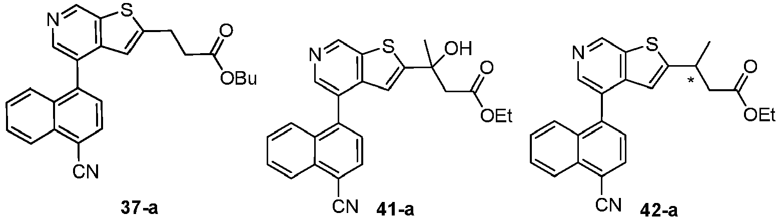 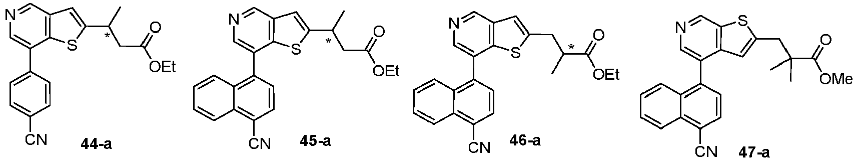 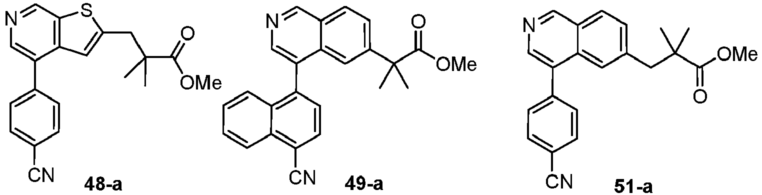 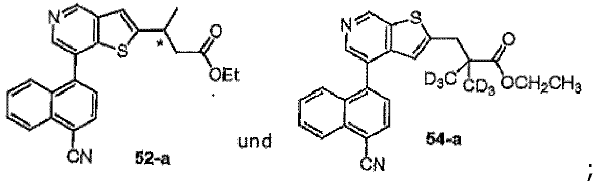 wobei sich in den obigen Verbindungen das mit * markierte Kohlenstoffatom auf ein chirales Kohlenstoffatom oder ein nichtchirales Kohlenstoffatom bezieht, das, wenn es ein chirales Kohlenstoffatom ist, in der S-Konfiguration oder R-Konfiguration vorliegt, und wenn es ein nichtchirales Kohlenstoffatom ist, sich auf ein Racemat bezieht.

12. Kondensiertes Ringderivat mit einer Struktur der Formel II-1 oder IV-1, Tautomer, Mesomer, Racemat, Enantiomer, Diastereomer oder pharmazeutisch annehmbares Salz davon gemäß wenigstens einem der Ansprüche 1 bis 9 zur Verwendung bei der Prävention und/oder Behandlung von Hyperurikämie oder einer mit Hyperurikämie zusammenhängenden Krankheit.

13. Kondensiertes Ringderivat mit einer Struktur der Formel II-1 oder IV-1, Tautomer, Mesomer, Racemat, Enantiomer, Diastereomer oder pharmazeutisch annehmbares Salz davon zur Verwendung gemäß Anspruch 12, wobei die mit Hyperurikämie zusammenhängende Krankheit aus der Gruppe ausgewählt ist, die aus Gicht, Bluthochdruck, Diabetes, Hypertriglyceridämie, metabolischem Syndrom, koronarer Herzkrankheit und Nierenschäden besteht.

14. Pharmazeutische Zusammensetzung, die eine pharmazeutisch wirksame Menge des kondensierten Ringderivats mit der Struktur der Formel II-1 oder IV-1, des Tautomers, Mesomers, Racemats, Enantiomers, Diastereomers oder seines pharmazeutisch annehmbaren Salzes gemäß einem der Ansprüche 1 bis 9 sowie einen oder mehr als einen pharmazeutisch annehmbaren Träger und/oder Verdünnungsmittel enthält.

15. Pharmazeutische Zusammensetzung gemäß Anspruch 14, wobei die Zusammensetzung weiterhin noch andere harnsäuresenkende Wirkstoffe enthält, wobei die harnsäuresenkenden Wirkstoffe aus der Gruppe ausgewählt sind, die aus Harnsäuretransporter-1-Inhibitor, Xanthin-Oxidase-Inhibitor, Xanthin-Oxidoreductase und Xanthin-Dehydrogenase-Inhibitor besteht.

16. Pharmazeutische Zusammensetzung gemäß Anspruch 15, wobei die Zusammensetzung weiterhin Purinalkohol und/oder Febuxostat enthält.

## Revendications

1. Dérivé à noyaux condensés ayant une structure répondant à la formule II-1 ou IV-1, tautomère, mésomère, racémate, énantiomère, diastéréomère ou sel pharmaceutiquement acceptable de celui-ci, 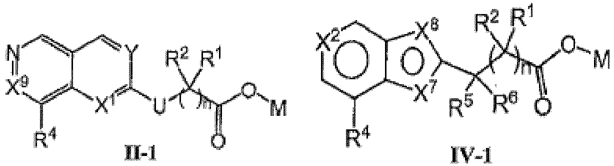 où
M est H, D ou un cation pharmaceutiquement acceptable,
U est 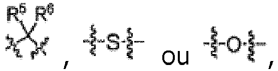
X¹ est CH ou N, X² est N,
X⁹ est C ou N,
Y est CH ou N,
X⁷ et X⁸ sont chacun indépendamment CH ou S,
R¹ et R² sont chacun indépendamment H, D, halogène, CN, alkyle, alcoxy, cycloalkyle, alcényle, alcynyle ou hétérocycloalkyle, ou R¹, R² ensemble, avec l'atome de carbone auxquel ils sont liés, représentent cycloalkyle ou un groupe hétérocyclique, ledit alkyle, alcoxy, cycloalkyle, alcényle, alcynyle, hétérocycloalkyle, ledit cycloalkyle formé par R¹, R² ensemble avec l'atome de carbone auxquel ils sont liés, ou ledit hétéroalkyle formé par R¹, R² ensemble avec l'atome de carbone auxquel ils sont liés peuvent en outre être substitués avec un substituant choisi dans le groupe consistant en D, un halogène, CN, alkyle, alcoxy, cycloalkyle, alcényle, alcynyle, hétérocycloalkyle ou aryle,
R⁴ est alkyle, alcoxy, cycloalkyle, alcényle, alcynyle, hétérocycloalkyle, chacun substitué avec un substituant choisi dans le groupe consistant en un aryle, un aryle substitué avec un halogène et/ou CN, hétéroaryle, ou hétéroaryle substitué avec un CN, ou un aryle ou hétéroaryle, dans lequel ledit aryle ou hétéroaryle peut en outre être substitué avec un substituant choisi dans le groupe consistant en D, halogène, CN, alkyle, alcoxy, cycloalkyle, alcényle, alcynyle, hétérocycloalkyle, aryle, un aryle substitué avec un halogène et/ou CN, hétéroaryle ou hétéroaryle substitué avec un CN,
R⁵ et R⁶ sont chacun indépendamment H, D, OH, halogène, CN, alkyle, alcoxy, cycloalkyle, alcényle, alcynyle ou hétérocycloalkyle, ou R⁵, R⁶ ensemble, avec l'atome de carbone auxquel ils sont liés, forment cycloalkyle ou un groupe hétérocyclique, ledit alkyle, alcoxy, cycloalkyle, alcényle, alcynyle, hétérocycloalkyle, ledit cycloalkyle formé par R⁵, R⁶ ensemble avec l'atome de carbone auxquel ils sont liés ou le groupe hétérocyclique formé par R⁵, R⁶ ensemble avec l'atome de carbone auxquel ils sont liés, peut en outre être substitué avec un substituant choisi dans le groupe consistant en D, halogène, CN, alkyle, alcoxy, cycloalkyle, alcényle, alcynyle, hétérocycloalkyle ou aryle,
n est 0, 1 ou 2.

2. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 1, dans lequel
M est H ou un cation pharmaceutiquement acceptable,
R¹ et R² sont chacun indépendamment H, D ou alkyle, ou R¹ et R² ensemble, avec l'atome de carbone auxquel ils sont liés, forment un cycloalkyle,
R³ est H, halogène, alkyle ou aryle,
R⁴ est un aryle ou hétéroaryle,
R⁵ et R⁶ sont chacun indépendamment H, OH, halogène ou alkyle.

3. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 1, dans lequel
M est un ion de Na, un ion de K, ou un ion de Ca,
et/ou R¹ et R² sont chacun indépendamment F, Cl, Br ou I, alkyle en C₁₋₄, alcoxy en C₁₋₄, cycloalkyle en C₃₋₆, alcényle en C₂₋₄, alcynyle en C₂₋₄, ou hétérocycloalkyle en C₂₋₁₀ ayant 1-2 hétéroatomes choisis parmi O, S ou N, ou R¹ et R² ensemble, avec l'atome de carbone auxquel ils sont liés, forment cycloalkyle en C₃₋₆, ou R¹ et R² ensemble, avec l'atome de carbone auxquel ils sont liés, forment un groupe hétérocyclique en C₂₋₅ ayant 1-2 hétéroatomes choisis parmi O ou S,
et/ou R³ est F, Cl, Br ou I, alkyle en C₁₋₄, alcoxy en C₁₋₄, aryle en C₆₋₁₀, ou hétéroaryle en C₂₋₅ ayant 1-3 hétéroatomes choisis parmi N, ou hétérocycloalkyle en C₂₋₁₀ ayant 1-3 hétéroatomes choisis parmi N, O ou S,
et/ou R⁴ est aryle en C₆₋₁₀ ou hétéroaryle en C₂₋₁₀ ayant 1-2 hétéroatomes choisis parmi O,
et/ou R⁵ et R⁶ sont chacun indépendamment F, Cl, Br ou I, alkyle en C₁₋₄, alcoxy en C₁₋₄, cycloalkyle en C₃₋₆, alcényle en C₂₋₄, alcynyle en C₂₋₄, ou hétérocycloalkyle en C₂₋₁₀ ayant 1-2 hétéroatomes choisis parmi O, S ou N, ou R⁵ et R⁶ ensemble, avec l'atome de carbone auxquel ils sont liés, forment un cycloalkyle en C₃₋₆, ou R⁵, R⁶ ensemble, avec l'atome de carbone auxquel ils sont liés, forment un hétérocycloalkyle en C₂₋₁₀ avec 1-2 hétéroatomes choisis parmi O ou S.

4. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 1, dans lequel
ledit cycloalkyle formé par R¹ et R² ensemble avec l'atome de carbone auxquel ils sont liés ou ledit groupe hétérocyclique formé par R¹ et R² ensemble avec l'atome de carbone auxquel ils sont liés est substitué avec un substituant choisi dans le groupe consistant en F, Cl, Br, I, alkyle en C₁₋₄, alcoxy en C₁₋₄, cycloalkyle en C₃₋₆, alcényle en C₂₋₄, alcynyle en C₂₋₄, aryle en C₆₋₁₀, hétérocycloalkyle en C₂₋₁₀ ayant 1-2 hétéroatomes choisis parmi O, S ou N,
et/ou ledit cycloalkyle formé par R⁵, R⁶ ensemble avec l'atome de carbone auxquel ils sont liés ou ledit groupe hétérocyclique formé par R⁵, R⁶ ensemble avec l'atome de carbone auxquel ils sont liés est substitué avec un substituant choisi dans le groupe consistant en F, Cl, Br, I, alkyle en C₁₋₄, alcoxy en C₁₋₄, cycloalkyle en C₃₋₆, alcényle en C₂₋₄, alcynyle en C₂₋₄, aryle en C₆₋₁₀, hétérocycloalkyle en C₂₋₁₀ ayant 1-2 hétéroatomes choisis parmi O, S ou N,
et/ou ledit alkyle, alcoxy, aryle, hétéroaryle, hétérocycloalkyle ou amino défini chez R³ est substitué avec un substituant choisi dans le groupe consistant en F, Cl, Br, I, alkyle en C₁₋₄, aryle en C₆₋₁₀, 2,6-dichlorophényle, 2,6-dichlorobenzyle, et 2,6-dichlorobenzoyle,
et/ou ledit alkyle, alcoxy, cycloalkyle, alcényle, alcynyle, hétérocycloalkyle défini chez R⁴ est substitué avec un substituant choisi dans le groupe consistant en 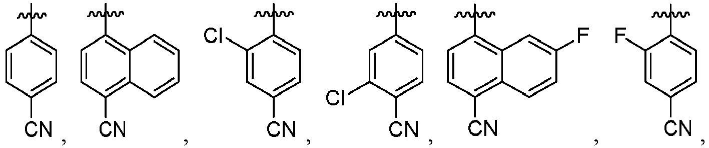 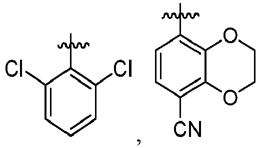 et un hétéroaryle en C₂₋₁₀ ayant 1-2 hétéroatomes choisis parmi O, et/ou ledit aryle ou hétéroaryle défini chez R⁴ est substitué avec un substituant choisi dans le groupe consistant en F, Cl, Br, I, alkyle en C₁₋₄, alcoxy en C₁₋₄, cycloalkyle en C₃₋₆, alcényle en C₂₋₄, alcynyle en C₂₋₄, aryle en C₆₋₁₀, hétérocycloalkyle en C₂₋₁₀ ayant 1-2 hétéroatomes choisis parmi O, S ou N, 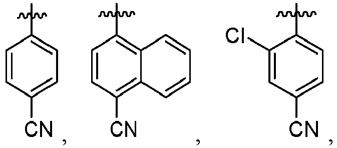 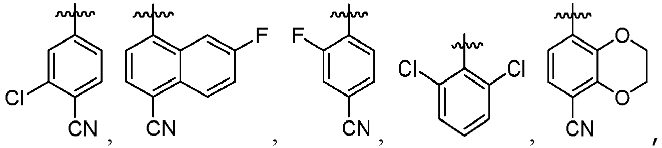 et hétéroaryle en C₂₋₁₀ ayant 1-2 hétéroatomes choisis parmi O.

5. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 3, dans lequel
R¹ et R² sont chacun indépendamment méthyle, éthyle, n-propyle, isopropyle, n-butyle, isobutyle, ou tert-butyle,
ou R¹ et R² ensemble, avec l'atome de carbone auxquel ils sont liés, forment un cyclobutyle,
et/ou R³ est méthyle, éthyle, n-propyle, isopropyle, n-butyle, isobutyle, tert-butyle, phényle, pyridyle, ou 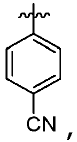 et/ou R⁴ est phényle, naphtyle, 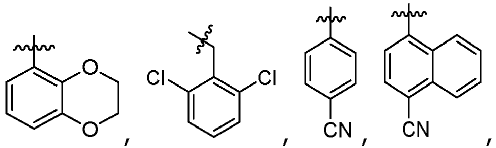 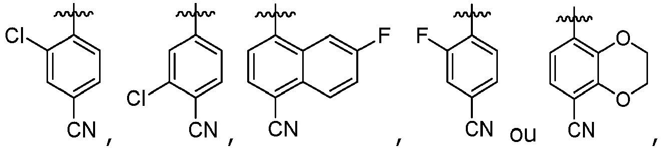 et/ou R⁵ et R⁶ sont chacun indépendamment méthyle, éthyle, n-propyle, isopropyle, n-butyle, isobutyle, ou tert-butyle.

6. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 3, dans lequel
dans le composé répondant à la formule générale II-1,
R¹ et R² sont chacun indépendamment H, méthyle, éthyle, n-propyle, isopropyle, n-butyle, isobutyle, ou tert-butyle, ou R¹ et R² ensemble, avec l'atome de carbone auxquel ils sont liés, forment un cyclobutyle,
M est H,
R⁴ est phényle, naphtyle, 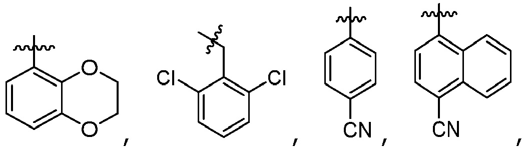 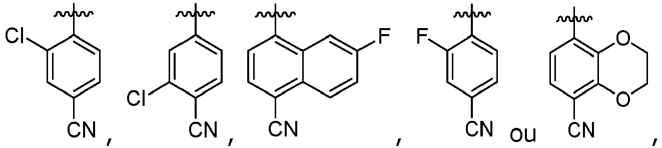 U est 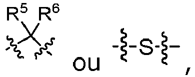 R⁵ et R⁶ sont chacun indépendamment H, méthyle, éthyle, n-propyle, isopropyle, n-butyle, isobutyle, ou tert-butyle,
n est 1,
dans le composé répondant à la formule IV-1,
X⁷ et X⁸ sont chacun indépendamment CH ou S,
R¹ et R² sont chacun indépendamment H, méthyle, éthyle, n-propyle, isopropyle, n-butyle, isobutyle, ou tert-butyle,
R⁴ est phényle, naphtyle, 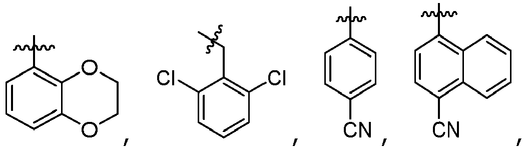 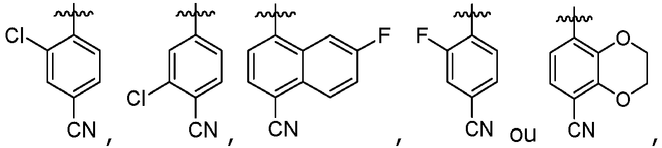 R⁵ et R⁶ sont chacun indépendamment H, méthyle, éthyle, n-propyle, isopropyle, n-butyle, isobutyle, ou tert-butyle,
n est 0 ou 1.

7. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 6, dans lequel
dans le composé répondant à la formule II-1, dans laquelle U est 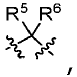 et
R⁵ et R⁶ sont H, R¹ et R² ne sont pas H en même temps,
dans le composé répondant à la formule IV-1,
X⁷ est CH, X⁸ est S.

8. Dérivé à noyaux condensés ayant une structure répondant à la formule II-1 ou IV-1, tautomère, mésomère, racémate, énantiomère, diastéréomère ou sel pharmaceutiquement acceptable de celui-ci selon la revendication 6, dans lequel
dans le composé répondant à la formule II-1, dans laquelle U est 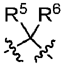 et
R⁵ et R⁶ sont H, R¹ et R² ne sont pas H en même temps;
dans le composé répondant à la formule IV-1, dans laquelle R¹ et/ou R² sont alkyle, R⁵ et R⁶ sont H, lorsque R⁵ et/ou R⁶ sont alkyle, R¹ et R² sont H, dans laquelle X⁷ est S, X⁸ est CH, R¹ et/ou R² sont alkyle, R⁵ et R⁶ sont H, R⁴ est un phényle.

9. Dérivé à noyaux condensés, tautomère, mésomère, racémate, énantiomère, diastéréomère ou sel pharmaceutiquement acceptable de celui-ci selon l'une au moins des revendications 1 à 8, choisi dans le groupe consistant en : 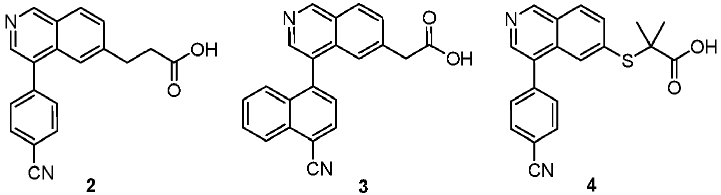 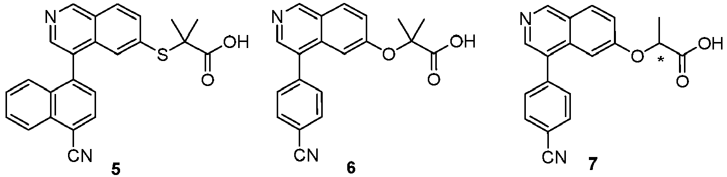 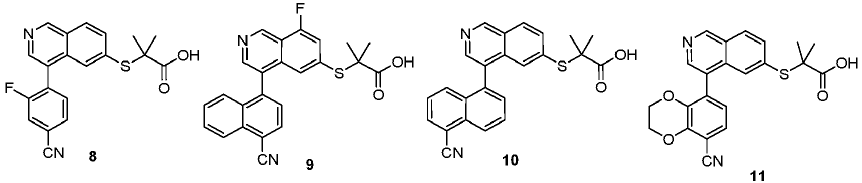 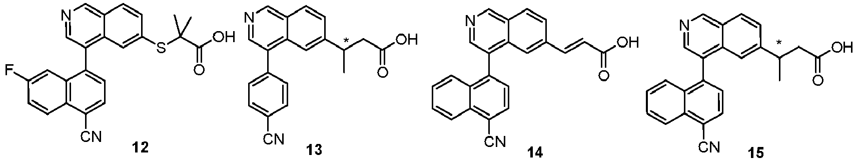 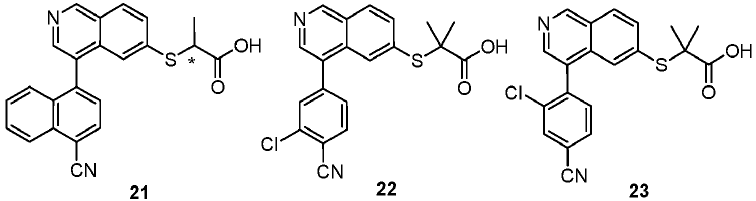 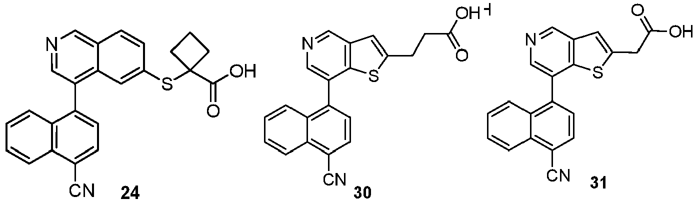 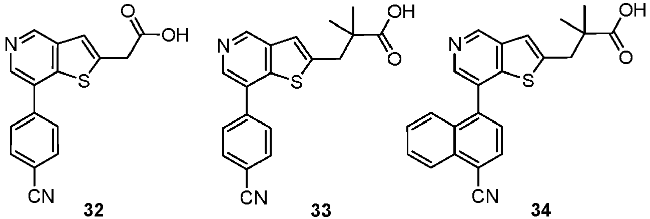 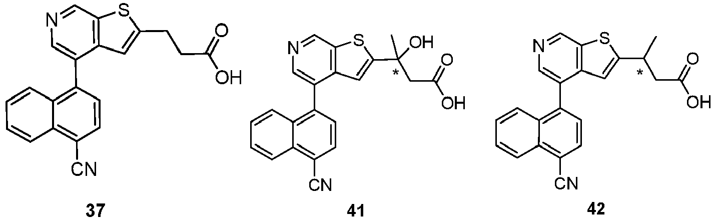 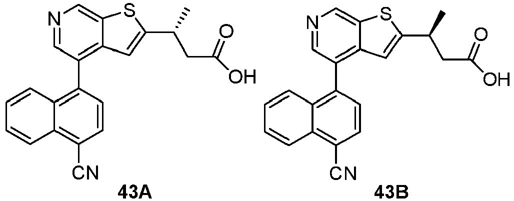 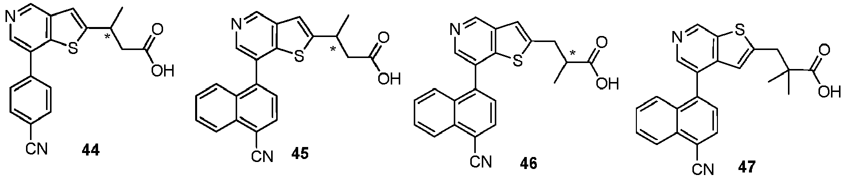 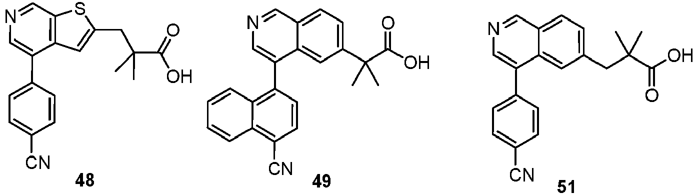 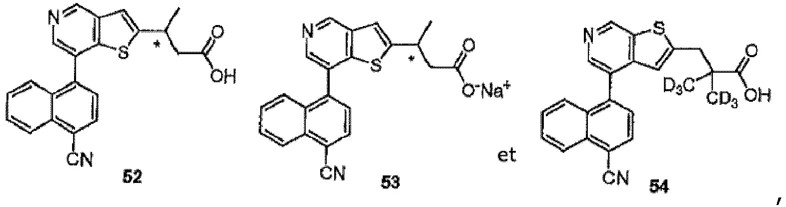 dans les composés ci-dessus, l'atome de carbone marqué avec * représente un atome de carbone chiral ou un atome de carbone non chiral, lorsqu'il est un atome de carbone chiral, est dans la configuration A ou dans la configuration R, et lorsqu'il est un atome de carbone non chiral, il représente un racémate.

10. Composé répondant à la formule II-a-1 ou IV-a-1 : 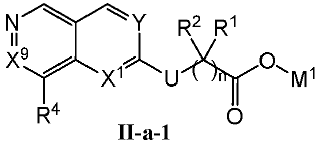 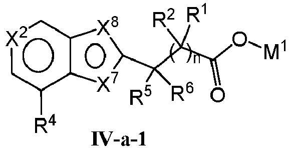 M¹ est un alkyle,
dans le composé répondant à la formule II-a-1, X¹ est CH ou N, Y est CH ou N, les définitions de X⁹, R¹, R², R³, R⁴, U et n se rapportent à celles dans l'une quelconque des revendications 1 à 9,
dans le composé répondant à la formule IV-a-1, X⁷ et X⁸ sont chacun indépendamment CH ou S, les définitions de R¹, R², R⁴, R⁵, R⁶, X² et n se rapportent à celles dans l'une quelconque des revendications 1 à 9.

11. Composé répondant à la formule II-a-1 ou IV-a-1 selon la revendication 10, dans lequel ledit composé est choisi dans le groupe consistant en 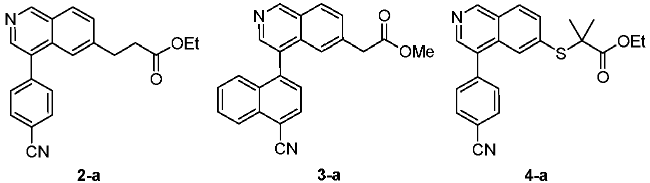 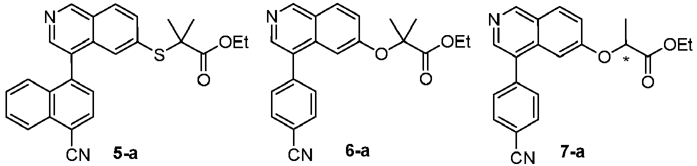 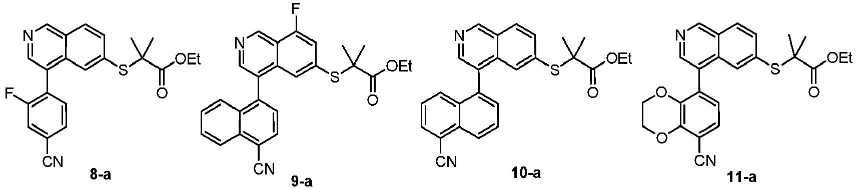 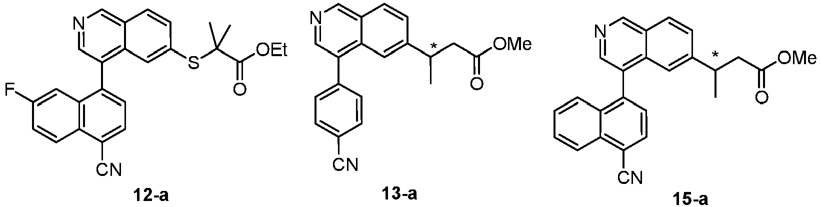 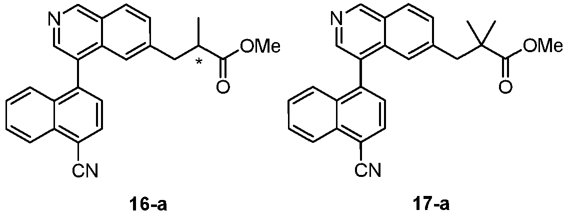 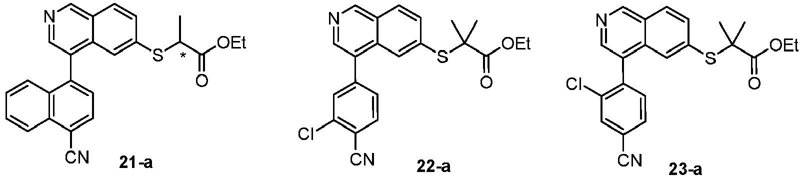 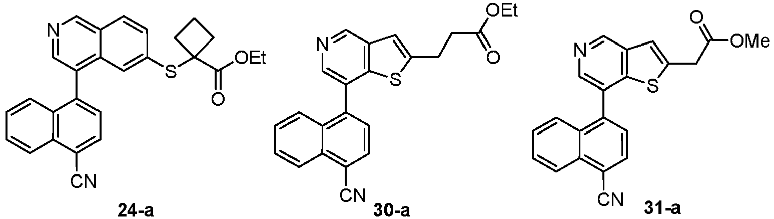 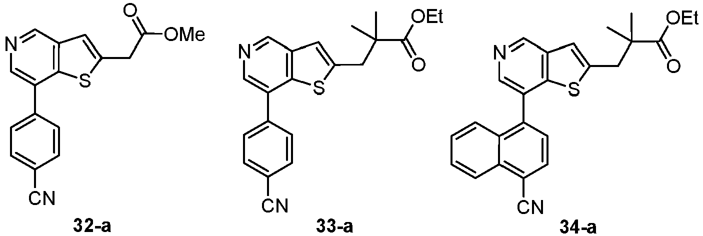 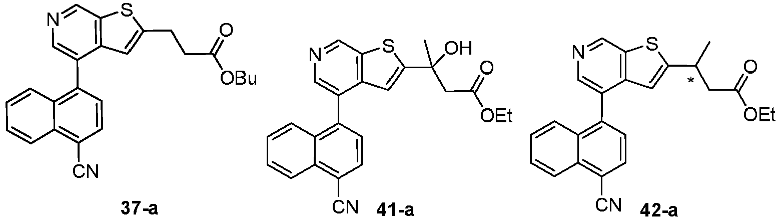 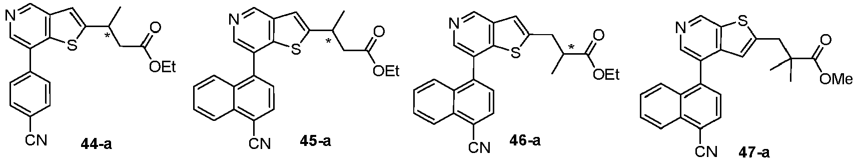 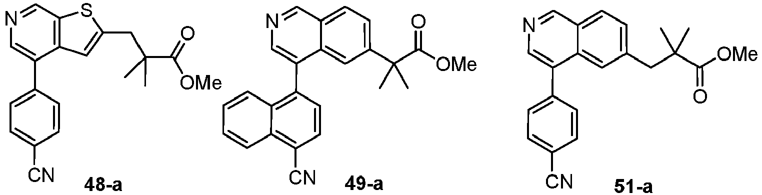 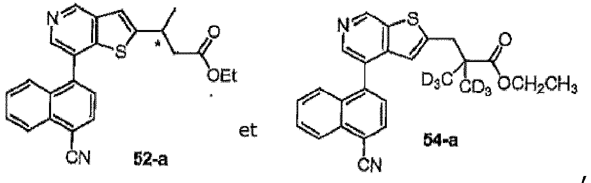 dans les composés ci-dessus, l'atome de carbone marqué avec * représente un atome de carbone chiral ou un atome de carbone non chiral, lorsqu'il est un atome de carbone chiral, est dans la configuration A ou dans la configuration R, et lorsqu'il est un atome de carbone non chiral, il représente un racémate.

12. Dérivé à noyaux condensés ayant une structure répondant à la formule II-1 ou IV-1, tautomère, mésomère, racémate, énantiomère, diastéréomère ou sel pharmaceutiquement acceptable de celui-ci selon l'une au moins des revendications 1 à 9 à utiliser dans la prévention et/ou le traitement de l'hyperuricémie ou d'une maladie associée à l'hyperuricémie.

13. Dérivé à noyaux condensés ayant une structure répondant à la formule II-1 ou IV-1, tautomère, mésomère, racémate, énantiomère, diastéréomère ou sel pharmaceutiquement acceptable de celui-ci à utiliser selon la revendication 12, dans lequel ladite maladie associée à l'hyperuricémie est choisie dans le groupe consistant en goutte, hypertension, diabète, hypertriglycéridémie, syndrome métabolique, maladie coronarienne, et atteinte rénale.

14. Composition pharmaceutique contenant une quantité pharmaceutiquement effective du dérivé à noyaux condensés ayant une structure répondant à la formule II-1 ou IV-1, du tautomère, mésomère, racémate, énantiomère, diastéréomère ou sel pharmaceutiquement acceptable de celui-ci selon l'une au moins des revendications 1 à 9, et un ou plusieurs véhicules et/ou diluants pharmaceutiquement acceptables.

15. Composition pharmaceutique selon la revendication 14, dans laquelle la composition contient en outre autres principes actifs diminuant le taux d'acide urique, lesdits principes actifs diminuant le taux d'acide urique étant choisis dans le groupe consistant en l'inhibiteur du transporteur 1 de l'acide urique, l'inhibiteur de l'oxydase de xanthine, l'oxydoréductase de xanthine, et l'inhibiteur de la déshydrogénase de xanthine.

16. Composition pharmaceutique selon la revendication 15, dans laquelle la composition contient en outre de l'alcool de purine et/ou du fébuxostat.
